# EUROPEAN PATENT APPLICATION

(11) **EP 4 458 984 A1**
(43) Date of publication of application: **06.11.2024**
(21) Application number: 22914805.1
(22) Date of filing: 27.12.2022
(51) Int. Cl.: C12Q 1/686, C12N 15/11

(54) **METHOD FOR DETECTING TARGET NUCLEIC ACID**

(30) Priority: 27.12.2021 CN 202111614026; 18.10.2022 CN 202211271772; 02.12.2022 CN 202211539752
(71) Applicant: Maccura Biotechnology Co., Ltd., Chengdu Sichuan 611731 (CN)
(72) Inventor: ZHAO, Yuhang, Chengdu, Sichuan 611731 (CN); HU, Waner, Chengdu, Sichuan 611731 (CN); WANG, Haixin, Chengdu, Sichuan 611731 (CN); HUANG, Qiuping, Chengdu, Sichuan 611731 (CN); GE, Zhiqi, Chengdu, Sichuan 611731 (CN)
(74) Representative: Laine IP Oy
(86) International application number: PCT/CN2022/142406
(87) International publication number: WO 2023/125552

(57) **Abstract**

Provided is a method for detecting a target nucleic acid, in particular a method for detecting a single or multiple target nucleic acid on digital PCR by using a universal probe. The detection method can achieve single-tube ultra-multiple detection, and has the advantages of a low fluorescence background, an adjustable melting temperature, good inclusiveness, low costs, simple and convenient operation, not being prone to causing pollution, high sensitivity, a wide application range and the like.

## Description

The present application claims the priority of the following patent application filed on December 27, 2021:
1. Chinese patent application No. CN 202111614026.1 entitled "primer-probe composition, detection method, and detection device".
   The present application also claims the priority of the following patent application filed on December 2, 2022:
2. Chinese patent application No. CN 202211539752.6 entitled "primer-probe composition and detection method".
   The present application also claims the priority of the following patent application filed on October 18, 2022:
3. Chinese patent application No. CN 202211271772.X entitled "multiple time-sharing nucleic acid detection device and detection method".

The above contents are incorporated herein by reference to the extent consistent with the present invention.

### Field of Technology

The present invention relates to the field of nucleic acid sequence detection, and in particular to a method for detecting a target nucleic acid. More specifically, the present invention relates to a method for detecting a single or multiple target nucleic acid on digital PCR by using a universal probe.

### Background

Polymerase chain reaction (PCR) is a molecular biological technique for enzymatic replication of DNA without using a living organism. PCR is commonly used in medical and biological research laboratories to undertake a variety of tasks, such as diagnosis of infectious diseases, gene cloning, phenotype identification of experimental animals, transcriptome research, detection of genetic diseases, identification of genetic fingerprints, and paternity testing. Because of unrivaled abilities of PCR to replicate and be precise, it is considered by molecular biologists to be a preferred method for nucleic acid detection. In the late 1990s, American ABI company launched Real Time Quantitative PCR (qPCR) technique and related products, which further developed PCR into a highly-sensitive, highly-specific and accurate quantitative nucleic acid sequence analysis technique.

Digital PCR (dPCR) is a technique for absolute quantification of nucleic acid molecules that utilizes the principle of limiting dilution to distribute one fluorescence quantitative PCR reaction system into thousands of individual nanoliter microreactors so that each microreactor contains no, or one or more copies of a target nucleic acid molecule (DNA target) to be simultaneously subj ected to single-molecule template PCR amplification. Different from the method of fluorescence quantitative PCR, which collects fluorescence at each amplification cycle, digital PCR collects a fluorescence signal of each reaction unit independently at the end of amplification, and finally works out an original number of copies or concentration of a target molecule through the principle of Poisson distribution and the ratio of positive/negative reaction units.

Compared to fluorescence quantitative PCR, digital PCR may perform accurate absolute quantitative detection without relying on a Ct value and a standard curve, and has the advantages of high sensitivity as well as high accuracy. Since digital PCR only determines two amplification states of "yes/no" when performing result interpretation, there is also no need to detect an intersection of a fluorescence signal and a set threshold line, and it is completely independent of identification of the Ct value, so that the impact of amplification efficiency on digital PCR reaction and result interpretation is greatly reduced, and the tolerance to a PCR reaction inhibitor is greatly improved. In addition, the process of distributing the reaction system in a digital PCR experiment may greatly reduce the concentration of a background sequence that competes with a target sequence locally, and thus digital PCR is particularly suitable for detection of a rare mutation in a complex background, and is currently mostly applied in liquid biopsies to achieve detection of rare mutation markers in peripheral blood of tumor patients.

In the process of multiple nucleic acid detection, a conventional sample detection device first amplifies a nucleic acid containing a target sequence to be detected and then performs signal acquisition at different temperatures. There are overlaps in signals obtained at different temperatures (i.e., different target sequences generating signals at the same time). A conventional calculation method is to separate the overlapping signals through image processing or other signal calculation methods, and then calculate a signal value generated by each target sequence respectively, which is complicated and prone to calculation errors.

### Summary

In order to solve the above problems, the present invention provides a method for detecting a target nucleic acid, which may detect one kind of target or two or more kinds of target nucleic acids. The method firstly amplifies a nucleic acid containing a target sequence to be detected and then performs signal acquisition. By setting an annealing temperature for amplification and an acquisition temperature for signal acquisition, a single signal can be achieved at different temperatures (at most one kind of target sequence generates a signal during a single signal acquisition). When there are two or more kinds of targets, the problems to be solved by the present invention further include how to detect and distinguish multiple different targets in single-tube reaction, and how to avoid a false positive signal due to a primer dimer in the presence of multiple sets of primers.

For this purpose, in a first aspect, the present invention provides a method for detecting a target nucleic acid, including the following steps:
S100, mixing a primer-probe composition, a sample to be detected and an amplification reagent to obtain a reaction system; the amplification reagent including a DNA polymerase and dNTPs;
S200, performing a first PCR amplification on the reaction system, with an annealing temperature for amplification of T1;
S300, performing any one of the following operations:
   operation 1, comprising:
      S310, performing a first signal acquisition at a first signal acquisition temperature t1;
      S320, performing a second PCR amplification, with an annealing temperature for amplification of T2;
   operation 2, comprising:
      S310', performing a first signal acquisition at a first signal acquisition temperature t1;
   operation 3, comprising:
      S310", performing a second PCR amplification, with an annealing temperature for amplification of T2;
      S320", performing a first signal acquisition at a first signal acquisition temperature t1;
      the above operations satisfying: T1 > T2, and T2 < t1;
      S400, performing a second signal acquisition at a second signal acquisition temperature t2, with t2 > t1, and t2 > T1;
      wherein there is one kind of target to be detected in the sample to be detected; and
      at most one of the first signal acquisition and the second signal acquisition contains a signal of the target.

In certain embodiments, one signal acquisition is performed after a first PCR amplification or first and second PCR amplifications, with a successively increasing signal acquisition temperature (t2 > t1). In certain embodiments, both the performing of two successive PCRs and only one PCR can generate a double-stranded product binding to a probe. When signal acquisition is performed at a relatively low temperature (t1), a detectable signal change is generated due to the formation of the double-stranded product. When signal acquisition is then performed at a higher temperature (t2), since t2 is higher than a melting temperature of the double-stranded product, the double-stranded product is in a single-stranded state. A signal acquired at t2 is different from a signal acquired at t1, that is, the signals from the two acquisitions are different, which can more accurately prove the presence of a target nucleic acid.

In certain embodiments, two PCRs and two signal acquisitions are performed, and the PCRs and the signal acquisitions are alternated: annealing temperatures for PCR amplifications successively decrease (T2 < T1), but signal acquisition temperatures successively increase (t2 > t1). That is, when a certain temperature is used as an annealing temperature of PCR for PCR cyclic amplification, a single-stranded pre-product formed by the target nucleic acid has an annealing temperature lower than that temperature, and thus cannot bind to the probe to form a double-stranded product, thereby failing to cause a detection signal change; and after this PCR amplification, i.e., performing signal acquisition at another temperature, a double-stranded product of a single-stranded pre-product formed by the target nucleic acid and the probe is in a double-stranded state, and a detectable target nucleic acid signal is generated. Singles from two acquisitions are different, which more accurately proves the presence of the target nucleic acid.

Therefore, by adopting the above PCR detection method, the presence or absence of the target nucleic acid can be more accurately proved: when the target nucleic acid is presented, signals from two acquisitions are different, and only one signal acquisition contains at most one kind of signal of the target nucleic acid; and if the target nucleic acid is not presented, both signal acquisitions contain no signals of any target nucleic acids.

T2 < T1 refers to a magnitude difference between the annealing temperature for the second PCR and the annealing temperature for the first PCR. The difference in annealing temperatures for the second PCR and the first PCR makes the number of classes of double-stranded products formed with a certain probe after the second PCR amplification increased by at most one, compared to the number of classes of double-stranded products formed with the probe after the first PCR amplification (no increase refers to no corresponding target nucleic acids in a product to be detected). In certain embodiments, the temperatures of T2 and T1 differ by 4°C or above, e.g., 4-40°C, e.g., 4-30°C, 4-20°C, 4-18°C, 4-15°C, 4-12°C, 4-10°C, 4-8°C, 4-6°C, 4-5°C, or the like.

t2 > t1 refers to a magnitude difference between the temperature for the second signal acquisition and the temperature for the first signal acquisition. The temperature difference between the second signal acquisition and the first signal acquisition makes the signal from the second signal acquisition different from the signal from the first signal acquisition. In certain embodiments, the temperatures of t2 and t1 differ by 6°C or above, e.g., 6-46°C, e.g., 6-40°C, 6-35°C, 6-30°C, 6-25°C, 6-20°C, 6-18°C, 6-15°C, 6-12°C, 6-10°C, 6-8°C, 6-7°C, or the like.

T2 < t1 refers to a magnitude difference between the annealing temperature for the second PCR and the temperature for the first signal acquisition. In certain embodiments, the temperatures of T2 and t1 differ by 5°C or above, e.g., 5-50°C, e.g., 5-40°C, 5-30°C, 5-20°C, 5-18°C, 5-15°C, 5-12°C, 5-10°C, 5-8°C, 5-7°C, 5-6°C, or the like.

t2 >T1 refers to a magnitude difference between the annealing temperature for the first PCR and the temperature for the second signal acquisition. In certain embodiments, the temperatures of t2 and T1 differ by 7°C or above, e.g., 7-50°C, e.g., 7-40°C, 7-30°C, 7-20°C, 7-18°C, 7-15°C, 7-12°C, 7-10°C, 7-8°C, or the like.

In certain implementations of the present invention, according to the above detection method, the annealing temperature for the first PCR amplification is T1, and the acquisition temperature for the first signal acquisition is t1; T1 ≤ t1.

In certain embodiments, at the first PCR amplification (with the annealing temperature of T1), one kind of single-stranded pre-products generated by a primer set and the probe specifically binds to the probe and extends by 0 base to form a double-stranded product, and the annealing temperature of the single-stranded pre-product is slightly lower than the melting temperature thereof (typically, an annealing temperature of an oligonucleotide is lower than a melting temperature (Tm) of the oligonucleotide). Signal acquisition is performed at t1 (T1 ≤ t1 < a melting temperature thereof), at which time, the single-stranded pre-product and the probe are in a double-stranded state, which makes the probe generate a detectable signal change. In certain embodiments, signal acquisition may also be performed at t1 (t1 ≤ T1), at which time, the single-stranded pre-product and the probe are also in a double-stranded state, which makes the probe generate a detectable signal change. A person skilled in the art may make a specific selection for the signal acquisition temperature t1 according to actual demands (e.g., during signal acquisition, the single-stranded pre-product and the probe are in a double-stranded state, and high signal strength is required for acquisition).

According to a preferred implementation of the present invention, the annealing temperature ranges from 40°C to 75°C, i.e., 40°C ≤ T2 < T1 ≤ 75°C.

According to a preferred implementation of the present invention, the signal acquisition temperature is 0-95°C, i.e., 0°C ≤ t1 < t2 ≤ 90°C. Typically, a person skilled in the art may determine the selection of the signal acquisition temperature according to actual situations of the designed primer-probe composition. For example, through the design of the primer-probe composition, a person skilled in the art may predict that a reaction product representing the target nucleic acid has a melting temperature of T. If it is desired to detect the signal of the target nucleic acid, the signal acquisition temperature ≤ T; and if it is not desired to detect the signal of the target nucleic acid, the signal acquisition temperature > T.

According to a preferred implementation of the present invention, reaction conditions for the first PCR amplification in step S200 include: initial denaturation at about 85°C to about 105°C for about 0 to about 15 min; denaturation at about 85°C to about 105°C for about 1 to about 60 s, and annealing and extending at about 40°C to about 75°C for about 3 to about 90 s, for 20 to 60 cycles; preferably, when the target in the sample to be detected is RNA, the amplification reagent further includes a reverse transcriptase, and the reaction conditions for performing the first PCR amplification on the reaction system include: reverse transcription at about 30 to about 65°C for about 2 to about 30 min; initial denaturation at about 85°C to about 105°C for about 0 to about 15 min; denaturation at about 85°C to about 105°C for about 1 to about 60 s, and annealing and extending at about 40°C to about 75°C for about 3 to about 90 s, for 20 to 60 cycles;
and/or, the second PCR amplification in step S300 includes the following reaction conditions: denaturation at about 85°C to about 105°C for about 1 to about 60 s, and annealing and extending at about 20°C to about 75°C for about 3 to about 90 s, for 1 to 20 cycles; alternatively, constant-temperature incubation at about 20°C to about 75°C for about 10 to about 1800 s.

According to a preferred implementation of the present invention, reaction conditions for the first PCR amplification in step S200 include: initial denaturation at about 90°C to about 96°C for about 5 to about 15 min; denaturation at about 90°C to about 95°C for about 10 to about 60 s, and annealing and extending at about 50°C to about 75°C for about 30 to about 90 s, for 35 to 50 cycles; further preferably, when the target in the sample to be detected is RNA, the amplification reagent further includes a reverse transcriptase, and the reaction conditions for performing the first PCR amplification on the reaction system include: reverse transcription at about 30 to about 65°C for about 2 to about 30 min; initial denaturation at about 90°C to about 96°C for about 5 to about 15 min; denaturation at about 90°C to about 95°C for about 10 to about 60 s, and annealing and extending at about 50°C to about 75°C for about 30 to about 90 s, for 35 to 50 cycles;
and/or, the second PCR amplification in step S300 includes the following reaction conditions: denaturation at about 85°C to about 105°C for about 2 to about 60 s, and annealing and extending at about 20°C to about 75°C for about 10 to about 90 s, for 1 to 20 cycles; alternatively, constant-temperature incubation at about 20°C to about 75°C for about 10 to about 1800 s.

According to a preferred implementation of the present invention, before performing the first PCR amplification on the reaction system, the method further includes: distributing the reaction system into 500 or more reaction units, with each reaction unit containing one target nucleic acid of the sample to be detected or containing no target nucleic acids of the sample to be detected; further preferably, the signal acquisition refers to an acquisition of a fluorescence signal by means of a camera; and the signal channel acquisition refers to an acquisition of the fluorescence signal by means of the camera under a fluorescence signal channel.

According to a preferred implementation of the present invention, the primer-probe composition includes: a probe (P) and a primer; the primer specifically binds to the target in the sample to be detected to generate a pre-product that contains one single-stranded pre-product capable of specifically binding to the probe, the single-stranded pre-product specifically binds to the probe and extends by >_ 0 base to form a double-stranded product, and the formation of the double-stranded product causes a probe signal change; further preferably, the single-stranded pre-product specifically binds to the probe and extends by 0-100 bases, more further preferably, by 1-100 bases, and an upper limit of the number of bases extended is the entire length of the probe; and/or, the primer has a concentration of about 30 nM to about 1,000 nM, and the probe (P) has a concentration of about 100 nM to about 1,200 nM. In the present invention, the concentration unit nM refers to nmol/L.

Specifically, extending by 0 base refers to no extending after the single-stranded pre-product specifically binds (hybridizes) to the probe (P). A person skilled in the art may, according to actual demands, achieve the above effect of "specifically binding but not extending" by designing a primer sequence in the primer set and/or a sequence of the probe. For example, it is designed in such a way that the resulting first single-stranded pre-product binds to a 5' end of the probe, or it is designed in such a way that a 3' end of the resulting first single-stranded pre-product contains a region that is not complementarily paired with the probe.

According to a preferred implementation of the present invention, the probe (P), as one freely-designed sequence that is not paired with or binding to any target nucleic acids, includes a probe signal detection region (H), and the probe is modified with detection labels; the primer includes a forward primer (F) and/or a reverse primer (R);
the forward primer (F) contains a target sequence binding region that is specifically paired with and binding to a target sequence;
the reverse primer (R) contains a primer signal detection region (h) and a target sequence binding region, and the primer signal detection region (h) is located at a 5' end of the target sequence binding region, or the reverse primer (R) sequentially contains an extension block region (M), the primer signal detection region (h) and the target sequence binding region from a 5' end to a 3' end;
where the target sequence binding region of the reverse primer (R) is a sequence that is specifically paired with and binding to the target sequence; the primer signal detection region (h) of the reverse primer (R) is one sequence that is not paired with or binding to any target sequences, and a complementary sequence (h') of the primer signal detection region (h) is specifically paired with and binding to the probe signal detection region (H) of the probe (P); the extension block region (M) of the reverse primer (R) is a freely-designed sequence that is not the same as or complementary with a sequence of any part of the probe (P) or any target sequences;
further preferably, there is a gap of 0-20 bases between the primer signal detection region (h) and the target sequence binding region in the reverse primer (R). The number of the gap bases may be 0, 1, 2, 3, 4, 5, 6, 7, 8, 9, 10, 11, 12, 13, 14, 15, 16, 17, 18, 19, 20, or a range thereof. In certain embodiments, there is a gap of 0-20 bases between the extension block region (M) and the primer signal detection region (h) of the reverse primer (R). The number of the gap bases may be 0, 1, 2, 3, 4, 5, 6, 7, 8, 9, 10, 11, 12, 13, 14, 15, 16, 17, 18, 19, 20, or a range thereof.

In the present invention, "specifically paired with or binding to" or "specifically binding" refers to complementary pairing and binding of bases of two single-stranded nucleic acid molecules with complementary sequences under certain conditions (a suitable temperature and ionic strength, etc.). It is well known to a person skilled in the art that the two single-stranded nucleic acid molecules do not have to be fully complementarily paired in order to bind specifically, and that the two single-stranded nucleic acid molecules can also bind specifically in the presence of a few mismatched bases.

According to a preferred implementation of the present invention, the forward primer (F) further includes a probe anchoring region (A), and the forward primer (F) sequentially includes the probe anchoring region (A) and a target sequence binding region from a 5' end to a 3' end; where the probe anchoring region (A) is one sequence that is not paired with or binding to any target sequences; further preferably, there is a gap of 0-20 bases between the probe anchoring region (A) and the target sequence binding region; where the number of the gap bases may be 0, 1, 2, 3, 4, 5, 6, 7, 8, 9, 10, 11, 12, 13, 14, 15, 16, 17, 18, 19, 20, or a range thereof;
the probe (P) further includes a primer anchoring region (A'), the primer anchoring region (A') is a sequence that is complementary with the probe anchoring region (A) of the forward primer (F); further preferably, there is a gap of 0-20 bases between the primer anchoring region (A') and the probe signal detection region (H) in the probe (P).

According to a preferred implementation of the present invention, the forward primer and the reverse primer are structurally interchangeable. For example, the primer structure of "the reverse primer (R) containing a primer signal detection region (h) and a target sequence binding region, and the forward primer (F) containing a target sequence binding region" may be interchanged into "the forward primer (F) containing a primer signal detection region (h) and a target sequence binding region, and the reverse primer (R) containing a target sequence binding region"; alternatively, the primer structure of "the reverse primer (R) containing a primer signal detection region (h) and a target sequence binding region, and the forward primer (F) containing a probe anchoring region (A) and a target sequence binding region" may be interchanged into "the forward primer (F) containing a primer signal detection region (h) and a target sequence binding region, and the reverse primer (R) containing a probe anchoring region (A) and a target sequence binding region".

It is well known to a person skilled in the art that to avoid a primer dimer, the target sequence binding region in the forward primer (F) and the target sequence binding region in the reverse primer (R) are different and cannot specifically bind.

According to a preferred implementation of the present invention, at least one kind of the forward primers (F) in the primers contains a first forward primer and a second forward primer, and the first forward primer sequentially contains a forward amplification product capturing region (B) and a target sequence binding region from a 5' end to a 3' end; and the second forward primer contains a forward amplification product capturing region (B) that is not paired with or binding to a target sequence or any fragments thereof; further preferably, there is a gap of 0-20 bases between the forward amplification product capturing region (B) and the target sequence binding region in the first forward primer.

According to a preferred implementation of the present invention, the second forward primer further includes a probe anchoring region (A), and further preferably, there is a gap of 0-20 bases between the probe anchoring region (A) and the forward amplification product capturing region (B) in the second forward primer.

According to a preferred implementation of the present invention, at least one kind of the reverse primers (R) in the primers contains a first reverse primer and a second reverse primer, and the first reverse primer sequentially contains a reverse amplification product capturing region (C) and a target sequence binding region from a 5' end to a 3' end; and the second reverse primer sequentially contains a primer signal detection region (h) and a reverse amplification product capturing region (C) from a 5' end to a 3' end, and the reverse amplification product capturing region (C) is not paired with a target sequence or any fragments thereof;
further preferably, there is a gap of 0-20 bases between the reverse amplification product capturing region (C) and the target sequence binding region in the first reverse primer; and/or, there is a gap of 0-20 bases between the primer signal detection region (h) and the reverse amplification product capturing region (C) in the second reverse primer. The number of the gap bases may be 0, 1, 2, 3, 4, 5, 6, 7, 8, 9, 10, 11, 12, 13, 14, 15, 16, 17, 18, 19, 20, or a range thereof.

In some implementations of the present invention, the above method for detecting the target nucleic acid is a digital PCR detection method. In some implementations of the present invention, before performing the first PCR amplification on the reaction system, the above method for detecting the target nucleic acid further includes: distributing the reaction system into 500 or more reaction units, with each reaction unit containing one target nucleic acid of the sample to be detected or containing no target nucleic acids of the sample to be detected; preferably, the signal acquisition refers to an acquisition of a fluorescence signal by means of a camera; and the signal channel acquisition refers to an acquisition of the fluorescence signal by means of the camera under a fluorescence signal channel. In the above method for detecting the target nucleic acid, the primer-probe composition may be a conventional primer-probe composition in the art, e.g., a primer-probe composition containing a molecular beacon, and may also be a primer-probe composition for nucleic acid sequence detection provided in a third aspect below.

For this purpose, in a second aspect, the present invention provides a method for detecting two or more kinds of target nucleic acids, comprising the following steps:
mixing a primer-probe composition, a sample to be detected and an amplification reagent to obtain a reaction system; the primer-probe composition including at least one kind of probe (P), and at least two kinds of primers for different targets; the amplification reagent including a DNA polymerase and dNTPs;
performing n PCR amplifications and n signal acquisitions on the reaction system; one signal acquisition being performed after each PCR amplification, and each signal acquisition including at least one signal channel acquisition; each signal channel acquisition containing at most one kind of target signal;
an annealing temperature for n<th> PCR amplification being Tn, an acquisition temperature for n<th> signal acquisition being tn, Tn < tn; Tn < T(n-1), and Tn < t(n-1); tn > t(n-1), and tn > T(n-1);
n being an integer ≥ 2, and n ≤ a number of classes of targets to be detected in the sample to be detected;.

In certain embodiments, one signal acquisition is performed after each PCR amplification, annealing temperatures for PCR amplifications successively decrease (Tn < T(n-1)), but signal acquisition temperatures successively increase (tn > t(n-1)). That is, when a certain temperature is used as an annealing temperature of PCR for PCR cyclic amplification, all single-stranded pre-products with an annealing temperature higher than that temperature can bind to the probe to form double-stranded products, thereby causing a detection signal change, and single-stranded pre-products with an annealing temperature lower than that temperature is still in a single-stranded state; and after this PCR amplification, i.e., performing signal acquisition at another temperature, the double-stranded products of only one kind of class of single-stranded pre-products and the probe are in a double-stranded state, and a detectable target nucleic acid signal is generated. (The reasons are that, as for other classes of single-stranded pre-products: part of the single-stranded pre-products cannot be annealed with the probe at the stage of PCR cylic amplification to form double-stranded products due to a lower annealing temperature; and the other part of the single-stranded pre-products can be annealed with the probe at the stage of PCR cylic amplification to form double-stranded products because of a high annealing temperature, but since such double-stranded products have a melting temperature lower than a signal acquisition temperature, the resulting double-stranded products are also in a single-stranded state at the signal acquisition temperature) Therefore, by adopting the above PCR detection method, there can be at most one kind of signal of the target nucleic acid among signals from each signal channel acquisition, and if the target nucleic acid is not presented, there is no signals of the target nucleic acid.

Tn < T(n-1) refers to a magnitude difference between the annealing temperature for the n<th> PCR and the annealing temperature for the (n-1)<th> PCR. The difference in annealing temperatures for the n<th> PCR and the (n-1)<th> PCR makes the number of classes of double-stranded products formed with a certain probe after the n<th> PCR amplification increased by at most one, compared to the number of classes of double-stranded products formed with the probe after the (n-1)<th> PCR amplification (no increase refers to no corresponding target nucleic acids in a product to be detected). In certain embodiments, the temperatures of Tn and T(n-1) differ by 4°C or above, e.g., 4-40°C, e.g., 4-30°C, 4-20°C, 4-18°C, 4-15°C, 4-12°C, 4-10°C, 4-8°C, 4-6°C, 4-5°C, or the like.

tn > t(n-1) refers to a magnitude difference between the temperature for the n<th> signal acquisition and the temperature for the (n-1)<th> signal acquisition. The temperature difference between the n<th> signal acquisition and the (n-1)<th> signal acquisition makes a double-stranded product at the n<th> signal acquisition different from a double-stranded product at the (n-1)<th> signal acquisition. For example, the double-stranded product at the (n-1)<th> signal acquisition is A, and the double-stranded product at the n<th> signal acquisition is B (at the n<th> signal acquisition, since the melting temperature of the double-stranded product A is lower than the temperature for the n<th> signal acquisition, at this time, the double-stranded product A is in a single-stranded state). In certain embodiments, the temperatures of tn and t(n-1) differ by 6°C or above, e.g., 6-46°C, e.g., 6-40°C, 6-35°C, 6-30°C, 6-25°C, 6-20°C, 6-18°C, 6-15°C, 6-12°C, 6-10°C, 6-8°C, 6-7°C, or the like.

Tn < tn refers to a magnitude difference between the annealing temperature for the n<th> PCR and the temperature for the n<th> signal acquisition. The difference in temperatures for the n<th> PCR and the n<th> signal acquisition makes possible to obtain a specific double-stranded product after the n<th> PCR amplification, and a signal of the double-stranded product may be obtained at the n<th> signal acquisition. In order to obtain the signal of the double-stranded product, at the n<th> signal acquisition (under the condition of the signal acquisition temperature tn ), the double-stranded product is not in a single-stranded state. In certain embodiments, Tn and tn differ by 1°C or above, e.g., 1-50°C, 1-40°C, 1-30°C, 1-25°C, 2-20°C, 2-15°C, 2-12°C, 2-10°C, 2-7°C, 2-6°C, 2-5°C, 3-5°C, 4-5°C, or the like. In certain embodiments, the signal acquisition temperature tn used is less than or equal to the melting temperature of the double-stranded product.

Tn < t(n-1) refers to a magnitude difference between the annealing temperature for the n<th> PCR and the temperature for the (n-1)<th> signal acquisition. The difference in temperatures for the n<th> PCR and the (n-1)<th> signal acquisition makes the double-stranded product at the (n-1)<th> signal acquisition still presented at the n<th> PCR, and in the meantime, another kind of double-stranded product different from the double-stranded product is further generated. In certain embodiments, the temperatures of Tn and t(n-1) differ by 5°C or above, e.g., 5-50°C, e.g., 5-40°C, 5-30°C, 5-20°C, 5-18°C, 5-15°C, 5-12°C, 5-10°C, 5-8°C, 5-7°C, 5-6°C, or the like.

tn >T(n-1) refers to a magnitude difference between the annealing temperature for the (n-1)<th> PCR and the temperature for the n<th> signal acquisition. In certain embodiments, the temperatures of tn and T(n-1) differ by 7°C or above, e.g., 7-50°C, e.g., 7-40°C, 7-30°C, 7-20°C, 7-18°C, 7-15°C, 7-12°C, 7-10°C, 7-8°C, or the like.

In the present invention, the number of classes of probes is determined according to the number of classes of detection labels thereof. Different kinds of probes are modified with different detection labels (i.e., in certain embodiments, detection groups), which may perform signal acquisition under different signal channels (or fluorescence signals). For example, the primer-probe composition of the present invention contains two kinds of probes, one kind of the probes is modified with detection groups of VIC and BHQ1, and a double-stranded product formed by specifically binding to the probe undergoes signal acquisition in a VIC channel; and the other kind of probe is modified with detection groups of FAM and BHQ1, and a double-stranded product formed by specifically binding to the probe undergoes signal acquisition in a FAM channel.

In certain embodiments, one signal acquisition is performed after each PCR amplification, each signal acquisition includes m signal channel acquisitions, where m is an integer ≥ 1, and m refers to the number of classes of detection groups of the probes in the primer-probe composition. As previously mentioned, after each PCR amplification, the number of classes of double-stranded products formed with the same kind of probe is increased by at most one, compared to the previous PCR amplification. If the number of classes of the probes in the primer-probe composition is m, after each PCR amplification, the number of classes of double-stranded products formed with the m kinds of probes is increased by at most m, compared to the previous PCR amplification (the number of classes of double-stranded product formed with each kind of probe is increased by at most one). Through m different signal channel acquisitions, target nucleic acid signals corresponding to the increased m kinds of double-stranded products may be distinguished without crossing each other, thereby achieving multiple detection.

According to a preferred implementation of the present invention, the at least one signal acquisition includes m signal channel acquisitions, m refers to the number of classes of the detection labels of the probe in the primer-probe composition, and a product of n and m ≥ the number of classes of targets to be detected in the sample to be detected. In order to facilitate programming of a detection instrument, in certain embodiments, if there are two kinds of probes in the primer-probe composition, each signal acquisition refers to perform two signal channel acquisitions under two signal channels. In certain embodiments, there are five kinds of targets in the sample to be detected. The primer-probe composition for the five kinds of targets contains two kinds of probes, with the first probe detecting three kinds of targets and the second probe detecting two kinds of targets. Thus, only three signal acquisitions are required for the reaction product, and each signal acquisition refers to perform two signal channel acquisitions under two signal channels. As such, there is a situation in which three signal acquisitions are performed under a certain signal channel, but there are only two kinds of corresponding targets, thus a signal from a certain signal channel acquisition has no targets, or signals from two adjacent signal channel acquisitions are the same. In these embodiments, the number of signal acquisitions is set in terms of the detection channel that detects the maximum number of targets, which achieves that the number of signal acquisitions < the number of classes of target nucleic acids, and reduces the number of signal acquisitions. Although it does not fully meet the situation that other signal channels have the minimum number of signal acquisitions, it is easier to set up an instrument program, and a person skilled in the art may choose according to actual demands. In certain embodiments, a person skilled in the art, according to the design of the primer-probe composition, chooses not to perform signal acquisition in channels without an expected target, thereby reducing the number of signal acquisitions and simplifying operation steps.

In certain implementations of the present invention, according to the above detection method, the annealing temperature for the first PCR amplification is T1, and the acquisition temperature for the first signal acquisition is 11; T1 ≤ t1.

In certain embodiments, at the first PCR amplification (with the annealing temperature of T1), one kind of single-stranded pre-products generated by a primer set and the probe specifically binds to the probe and extends by 0 base to form a double-stranded product, and the annealing temperature of the single-stranded pre-product is slightly lower than the melting temperature thereof (typically, an annealing temperature of an oligonucleotide is lower than a melting temperature (Tm) of the oligonucleotide). Signal acquisition is performed at t1 (T1 ≤ t1 < a melting temperature thereof), at which time, the single-stranded pre-product and the probe are in a double-stranded state, which makes the probe generate a detectable signal change. In certain embodiments, signal acquisition may also be performed at t1 (t1 ≤ T1), at which time, the single-stranded pre-product and the probe are also in a double-stranded state, which makes the probe generate a detectable signal change. A person skilled in the art may make a specific selection for the signal acquisition temperature t1 according to actual demands (e.g., during signal acquisition, the single-stranded pre-product and the probe are in a double-stranded state, and high signal strength is required for acquisition).

In certain embodiments, the annealing temperature is: 40°C ≤ Tn < T(n-1) ≤ 75°C (n being an integer ≥ 2).

In certain embodiments, the acquisition temperature is: 0°C ≤ t(n-1) < tn ≤ 90°C (n being an integer ≥ 2).

According to a preferred implementation of the present invention, reaction conditions for performing a first PCR amplification on the reaction system include: initial denaturation at about 85°C to about 105°C for about 0 to about 15 min; denaturation at about 85°C to about 105°C for about 1 to about 60 s, and annealing and extending at about 40°C to about 75°C for about 3 to about 90 s, for 20 to 60 cycles; preferably, when a target in the sample to be detected is RNA, the amplification reagent further includes a reverse transcriptase, and the reaction conditions for performing the first PCR amplification on the reaction system include: reverse transcription at about 30 to about 65°C for about 2 to about 30 min; initial denaturation at about 85°C to about 105°C for about 0 to about 15 min; denaturation at about 85°C to about 105°C for about 1 to about 60 s, and annealing and extending at about 40°C to about 75°C for about 3 to about 90 s, for 20 to 60 cycles.

According to a preferred implementation of the present invention, reaction conditions for performing a first PCR amplification on the reaction system include: initial denaturation at about 90°C to about 96°C for about 5 to about 15 min; denaturation at about 90°C to about 95°C for about 10 to about 60 s, annealing and extending at about 50°C to about 75°C for about 30 to about 90 s, for 35 to 50 cycles; preferably, when a target in the sample to be detected is RNA, the amplification reagent further includes a reverse transcriptase, and the reaction conditions for performing the first PCR amplification on the reaction system include: reverse transcription at about 30 to about 65°C for about 2 to about 30 min; initial denaturation at about 90°C to about 96°C for about 5 to about 15 min; denaturation at about 90°C to about 95°C for about 10 to about 60 s, and annealing and extending at about 50°C to about 75°C for about 30 to about 90 s, for 35 to 50 cycles.

According to a preferred implementation of the present invention, reaction conditions for performing the first PCR amplification on the reaction system include: initial denaturation at about 85°C to about 105°C for about 0 to about 15 min; denaturation at about 85°C to about 105°C for about 1 to about 60 s, and annealing and extending at about 40°C to about 75°C for about 3 to about 90 s, for 20 to 60 cycles; denaturation at about 85°C to about 105°C for about 1 to about 60 s, and annealing and extending at about 20°C to about 75°C for about 3 to about 90 s, for 1 to 20 cycles; alternatively, the reaction conditions for performing the first PCR amplification on the reaction system include: initial denaturation at about 85°C to about 105°C for about 0 to about 15 min; denaturation at about 85°C to about 105°C for about 1 to about 60 s, and annealing and extending at about 40°C to about 75°C for about 3 to about 90 s, for 20 to 60 cycles; and constant-temperature incubation at about 20°C to about 75°C for about 10 to about 1800 s;
preferably, when the target in the sample to be detected is RNA, the amplification reagent further includes a reverse transcriptase, and the reaction conditions for performing the first PCR amplification on the reaction system include: reverse transcription at about 30 to about 65°C for about 2 to about 30 min; initial denaturation at about 85°C to about 105°C for about 0 to about 15 min; denaturation at about 85°C to about 105°C for about 1 to about 60 s, and annealing and extending at about 40°C to about 75°C for about 3 to about 90 s, for 20 to 60 cycles; denaturation at about 85°C to about 105°C for about 1 to about 60 s, annealing and extending at about 20°C to about 75°C for about 3 to about 90 s, for 1 to 20 cycles; alternatively, the reaction conditions for performing the first PCR amplification on the reaction system include: reverse transcription at about 30 to about 65°C for about 2 to about 30 min; initial denaturation at about 85°C to about 105°C for about 0 to about 15 min; denaturation at about 85°C to about 105°C for about 1 to about 60 s, and annealing and extending at about 40°C to about 75°C for about 3 to about 90 s, for 20 to 60 cycles; and constant-temperature incubation at about 20°C to about 75°C for about 10 to about 1800 s.

According to a preferred implementation of the present invention, reaction conditions for performing the first PCR amplification on the reaction system include: initial denaturation at about 90°C to about 96°C for about 5 to about 15 min; denaturation at about 90°C to about 95°C for about 10 to about 60 s, and annealing and extending at about 50°C to about 75°C for about 30 to about 90 s, for 35 to 50 cycles; denaturation at about 90°C to about 95°C for about 10 to about 60 s, and annealing and extending at about 20°C to about 75°C for about 30 to about 90 s, for 1 to 20 cycles; alternatively, the reaction conditions for performing the first PCR amplification on the reaction system include: initial denaturation at about 90°C to about 96°C for about 5 to about 15 min; denaturation at about 90°C to about 95°C for about 10 to about 60 s, and annealing and extending at about 50°C to about 75°C for about 30 to about 90 s, for 35 to 50 cycles; and constant-temperature incubation at about 20°C to about 75°C for about 10 to about 1000 s;
preferably, when the target in the sample to be detected is RNA, the amplification reagent further includes a reverse transcriptase, and the reaction conditions for performing the first PCR amplification on the reaction system include: reverse transcription at about 30 to about 65°C for about 2 to about 30 min; initial denaturation at about 90°C to about 96°C for about 5 to about 15 min; denaturation at about 90°C to about 95°C for about 10 to about 60 s, and annealing and extending at about 50°C to about 75°C for about 30 to about 90 s, for 35 to 50 cycles; denaturation at about 90°C to about 95°C for about 10 to about 60 s, and annealing and extending at about 20°C to about 75°C for about 30 to about 90 s, for 1 to 20 cycles; alternatively, the reaction conditions for performing the first PCR amplification on the reaction system include: reverse transcription at about 30 to about 65°C for about 2 to about 30 min; initial denaturation at about 90°C to about 96°C for about 5 to about 15 min; denaturation at about 90°C to about 95°C for about 10 to about 60 s, and annealing and extending at about 50°C to about 75°C for about 30 to about 90 s, for 35 to 50 cycles; and constant-temperature incubation at about 20°C to about 75°C for about 10 to about 1000 s.

According to a preferred implementation of the present invention, other PCR amplifications after the first PCR amplification include the following reaction conditions: denaturation at about 85°C to about 105°C for about 1 to about 60 s, and annealing and extending at about 20°C to about 75°C for about 3 to about 90 s, for 1 to 20 cycles; alternatively, constant-temperature incubation at about 20°C to about 75°C for about 10 to about 1800 s.

According to a preferred implementation of the present invention, other PCR amplifications after the first PCR amplification include the following reaction conditions: denaturation at about 90°C to about 95°C for about 10 to about 60 s, and annealing and extending at about 20°C to about 75°C for about 30 to about 90 s, for 1 to 20 cycles; alternatively, constant-temperature incubation at about 20°C to about 75°C for about 10 to about 1000 s.

According to a preferred implementation of the present invention, at least one kind of the primers specifically binds to the target to generate a pre-product that contains one single-stranded pre-product capable of specifically binding to the probe (P), the single-stranded pre-product specifically binds to the probe (P) and extends to form a double-stranded product, and the formation of the double-stranded product causes a probe signal change; preferably, the single-stranded pre-product specifically binds to the probe and extends by 0-100 bases, more preferably, by 1-100 bases, and an upper limit of the number of bases extended is the entire length of the probe; and/or, the primer has a concentration of about 30 nM to about 1,000 nM, and the probe (P) has a concentration of about 100 nM to about 1,200 nM. If multiple targets share one kind of probe, a greater number of probes will be used, e.g., 1,300 nM, 1,400 nM, 1,500 nM, 1,600 nM, 1,700 nM, 1,800 nM, 1,900 nM, 2,000 nM, or more.

Specifically, extending by 0 base refers to no extending after the single-stranded pre-product specifically binds (hybridizes) to the probe (P). A person skilled in the art may, according to actual demands, achieve the above effect of "specifically binding but not extending" by designing a primer sequence in the primer set and/or a sequence of the probe. For example, it is designed in such a way that the resulting first single-stranded pre-product binds to a 5' end of the probe, or it is designed in such a way that a 3' end of the resulting first single-stranded pre-product contains a region that is not complementarily paired with the probe.

According to a preferred implementation of the present invention, the at least two kinds of primers for the different targets include any one of the following:
(1) at least two kinds of forward primers (F) and at least one kind of reverse primer (R);
(2) at least one kind of forward primer (F) and at least two kinds of reverse primers (R);
(3) at least two kinds of forward primers (F) and at least two kinds of reverse primers (R);
   the different forward primers (F) each independently contain a target sequence binding region that is specifically paired with and binding to different target sequences;
   and/or, the probe (P), as one freely-designed sequence that is not paired with or binding to any target sequences, includes a probe signal detection region (H), and the probe is modified with detection labels;
   and/or, the reverse primer (R) contains a primer signal detection region (h) and a target sequence binding region, and the primer signal detection region (h) is located at a 5' end of the target sequence binding region; where target sequence binding regions of the different reverse primers (R) are sequences that are specifically paired with and binding to different target sequences; each primer signal detection regions (h) of the different reverse primers (R) is one sequence that is not paired with or binding to any target sequences but partially or wholly identical to the probe signal detection region (H) of the probe (P); preferably, sequences of the primer signal detection regions (h) of the different reverse primers (R) are different from each other.

In the present invention, each of the primer signal detection regions (h) of the different reverse primers (R) being one sequence that is partially or wholly identical to the probe signal detection region (H) of the probe (P) means that a complementary sequence of the primer signal detection region (h) of the reverse primer (R) is specifically paired with and binding to the probe signal detection region (H) of the probe (P).

According to a preferred implementation of the present invention, when the primer includes at least two kinds of the primers, since the forward primer and the reverse primer are structurally interchangeable, there is no limitation on the ratio of the forward primer to the reverse primer. In certain embodiments, the content of the primers containing no primer signal detection regions h is greater than that of other primers containing primer signal detection regions h, preferably, the content of the primers containing no primer signal detection regions h is 1.2-20 times that of the other primers containing primer signal detection regions h, more preferably, the content of the primers containing no primer signal detection regions h is 2, 2.5, 3, 4, 5, 6, 7, 8, 9 or 10 times that of the other primers containing primer signal detection regions h.

According to a preferred embodiments of the present invention, at least one kind of the reverse primers (R) in the primers sequentially contains an extension block region (M), a primer signal detection region (h) and a target sequence binding region from a 5' end to a 3' end; the extension block region (M) is a freely-designed sequence that is not same as or complementary with a sequence of any part of the probe (P) or any target sequences; if there are other kinds of reverse primers, 5' ends of the other kinds of reverse primers (R) contain no extension block regions (M).

In certain embodiments, if there are other kinds of reverse primers, the other kinds of reverse primers (R) include primer signal detection regions (h) and target sequence binding regions, and the primer signal detection regions (h) are located at 3' ends of the target sequence binding regions; and in certain embodiments, if there are other kinds of reverse primers, the other kinds of reverse primers (R) sequentially contain primer signal detection regions (h) and target sequence binding regions from 5' ends to 3' ends.

According to a preferred implementation of the present invention, at least one kind of the forward primers (F) in the primers further includes a probe anchoring region (A), and the probe anchoring region (A) in the forward primer (F) is located at a 5' end of the target sequence binding region; where the probe anchoring region (A) is one sequence that is not paired with or binding to any target sequences; sequences of target sequence binding regions of different forward primers (F) are different; further preferably, there is a gap of 0-20 bases between the probe anchoring region (A) and the target sequence binding region in the forward primer (F); where the number of the gap bases may be 0, 1, 2, 3, 4, 5, 6, 7, 8, 9, 10, 11, 12, 13, 14, 15, 16, 17, 18, 19, 20, or a range thereof;
the probe (P), as one sequence that is not paired with or binding to any target sequences, includes a primer anchoring region (A') and a probe signal detection region (H); the primer anchoring region (A') is a sequence that is complementary with the probe anchoring region (A) of the forward primer (F); further preferably, there is a gap of 0-20 bases between the primer anchoring region (A') and the probe signal detection region (H) in the probe (P). The number of the gap bases may be 0, 1, 2, 3, 4, 5, 6, 7, 8, 9, 10, 11, 12, 13, 14, 15, 16, 17, 18, 19, 20, or a range thereof.

According to a preferred implementation of the present invention, at least one kind of the forward primers (F) in the primers sequentially contains a probe anchoring region (A) and a target sequence binding region from a 5' end to a 3' end; where the probe anchoring region (A) is one sequence that is not paired with or binding to any target sequences; sequences of target sequence binding regions of different forward primers (F) are different; preferably, there is a gap of 0-20 bases between the probe anchoring region (A) and the target sequence binding region in the forward primer; where the number of the gap bases may be 0, 1, 2, 3, 4, 5, 6, 7, 8, 9, 10, 11, 12, 13, 14, 15, 16, 17, 18, 19, 20, or a range thereof;
the probe (P), as one sequence that is not paired with or binding to any target sequences, includes a primer anchoring region (A') and probe signal detection region (H); the primer anchoring region (A') is a sequence that is reversely complementary with the probe anchoring region (A) of the forward primer (F); preferably, there is a gap of 0-20 bases between the primer anchoring region (A') and the probe signal detection region (H) in the probe (P). The number of the gap bases may be 0, 1, 2, 3, 4, 5, 6, 7, 8, 9, 10, 11, 12, 13, 14, 15, 16, 17, 18, 19, 20, or a range thereof.

According to a preferred implementation of the present invention, at least one kind of the forward primers (F) in the primers contains a first forward primer and a second forward primer, and the first forward primer sequentially contains a forward amplification product capturing region (B) and a target sequence binding region from a 5' end to a 3' end; and the second forward primer contains a forward amplification product capturing region (B) that is not paired with or binding to a target sequence or any fragments thereof, further preferably, there is a gap of 0-20 bases between the forward amplification product capturing region (B) and the target sequence binding region in the first forward primer. The number of the gap bases may be 0, 1, 2, 3, 4, 5, 6, 7, 8, 9, 10, 11, 12, 13, 14, 15, 16, 17, 18, 19, 20, or a range thereof.

According to a preferred implementation of the present invention, the second forward primer further includes a probe anchoring region (A), preferably, there is a gap of 0-20 bases between the probe anchoring region (A) and the forward amplification product capturing region (B) in the second forward primer.

According to a preferred implementation of the present invention, at least one kind of the reverse primers (R) in the primers contains a first reverse primer and a second reverse primer, and the first reverse primer sequentially contains a reverse amplification product capturing region (C) and a target sequence binding region from a 5' end to a 3' end; and the second reverse primer sequentially contains a primer signal detection region (h) and a reverse amplification product capturing region (C) from a 5' end to a 3' end, and the reverse amplification product capturing region (C) is not paired with a target sequence or any fragments thereof;
preferably, there is a gap of 0-20 bases between the reverse amplification product capturing region (C) and the target sequence binding region in the first reverse primer; and/or, there is a gap of 0-20 bases between the primer signal detection region (h) and the reverse amplification product capturing region (C) in the second reverse primer. The number of the gap bases may be 0, 1, 2, 3, 4, 5, 6, 7, 8, 9, 10, 11, 12, 13, 14, 15, 16, 17, 18, 19, 20, or a range thereof.

In some implementations of the present invention, the above method for detecting two or more kinds of target nucleic acids is a digital PCR detection method. In some implementations of the present invention, before performing the first PCR amplification on the reaction system, the above method for detecting two or more kinds of target nucleic acids further includes: distributing the reaction system into 500 or more reaction units, with each reaction unit containing one target nucleic acid of the sample to be detected or containing no target nucleic acids of the sample to be detected; preferably, the signal acquisition refers to perform an acquisition of a fluorescence signal by means of a camera; and the signal channel acquisition refers to perform an acquisition of the fluorescence signal by means of the camera under a fluorescence signal channel.

In the above method for detecting the target nucleic acid, the primer-probe composition may be a conventional primer-probe composition in the art, e.g., a primer-probe composition containing a molecular beacon, and may also be a primer-probe composition for nucleic acid sequence detection provided in a third aspect below.

For this purpose, in a third aspect, the present invention provides a primer-probe composition for nucleic acid sequence detection, including a first probe and a first primer mixture;
the first primer mixture includes at least two kinds of primer sets, and the different kinds of primer sets specifically bind to different kinds of target nucleic acids respectively;
the primer sets in the first primer mixture specifically bind to the corresponding target nucleic acids to generate a pre-product, the pre-product contains a single-stranded pre-product specifically binding to the first probe, and the single-stranded pre-product specifically binds to the first probe and extends by >_ 0 base to form a double-stranded product, and the formation of the double-stranded product causes a detectable signal change;
the different kinds of primer sets in the first primer mixture and the corresponding target nucleic acids generate different single-stranded pre-products; and when an annealing temperature of a first single-stranded pre-product and the first probe is higher than an annealing temperature of a second single-stranded pre-product and the first probe, a melting temperature of a double-stranded product generated by the first single-stranded pre-product and the first probe is lower than a melting temperature of a double-stranded product generated by the second single-stranded pre-product and the first probe.

The first probe refers to one class of probe that is modified with the same detection labels and may have the same or different base sequences. That is, the number of classes of probes is determined according to the number of classes of detection labels thereof. In certain embodiments, a signal generated from one kind of probe may undergo signal acquisition in one detection channel (or a fluorescence channel); and signals generated from two kinds of probes may undergo signal acquisition in two different detection channels. In certain embodiments, the same detection labels do not mean that detection groups for modification are exactly the same. For example, the detection groups include fluorophores and quenchers. Signals generated from probes may also undergo signal acquisition in one detection channel (or fluorescence channel) as long as the fluorophores are the same.
"at least two kinds of primer sets" refers to at least two classes of primer sets. Each primer set is specific to a different target nucleic acid, that is, each primer set can specifically bind to a corresponding target nucleic acid. In certain embodiments, one kind of primer set can specifically bind to one class of non-typed target nucleic acids all, and thus this class of non-typed target nucleic acids may also be considered as one kind of target nucleic acid.

In certain embodiments, a primer set belongs to a two-primer case, i.e., including a pair of upstream and downstream primers specific to the same target nucleic acid. In certain embodiments, a primer set belongs to a single-primer case, i.e., only containing one kind of primer specific to a certain target nucleic acid. The primer can specifically bind to the corresponding target nucleic acid but cannot specifically bind to other kinds of target nucleic acids.

In certain embodiments, different kinds of primer sets may share the same primer. For example, the same kind of primer is included in both primer sets. The same kind of primer refers to sequences of primers being exactly the same.

Typically, an annealing temperature of an oligonucleotide is about 5°C lower than a melting temperature (Tm) of the oligonucleotide, although the annealing temperature of the oligonucleotide may be determined to be about 4.9, 4.8, 4.7, 4.6, 4.5, 4.4, 4.3, 4.2, 4.1, 3.9, 3.8, 3.7, 3.6, 3.5, 3.4, 3.3, 3.2, 3.1, 3.0°C, or more, or any range therebetween (including end values) lower than Tm of the oligonucleotide. The annealing temperature and melting temperature of the oligonucleotide are related to factors such as the length and composition of the oligonucleotide. A person skilled in the art may adjust the annealing temperature and melting temperature of the oligonucleotide according to actual needs. For example, a higher melting temperature may be obtained by increasing the GC content of the oligonucleotide, making the length of the oligonucleotide longer.

In certain embodiments, the lower the annealing temperature required for a single-stranded pre-product generated by a certain kind of primer set and a target nucleic acid thereof to specifically bind to the probe, the higher the melting temperature of a double-stranded product formed by the single-stranded pre-product specifically binding to the probe and extending by >_ 0 base. For example, by adjusting the lengths and/or sequences of the primer sets, the primer sets and the target nucleic acids specifically bind to obtain different single-stranded pre-products. Since the different single-stranded pre-products bind to the same probe at different locations, the annealing temperature of the first single-stranded pre-product and the probe (here referred to as "a first annealing temperature") is higher than the annealing temperature of the second single-stranded pre-product and the probe (here referred to as "a second annealing temperature"), that is, the first annealing temperature is higher than the second annealing temperature; and since the different single-stranded pre-products bind to the same probe at different locations, the lengths by which the different single-stranded pre-products bind to the probe and extend may be adjusted, so that a melting temperature of a double-stranded product generated by the first single-stranded pre-product and the probe (here referred to as "a first melting temperature") is lower than a melting temperature of a double-stranded product generated by the second single-stranded pre-product and the probe (here referred to as "a second melting temperature"), that is, the first melting temperature is lower than the second melting temperature.

In the present invention, the expressions of "higher than" and "lower than" are used for describing the difference between two temperatures, and the presence of the difference enables different kinds of single-stranded pre-products to be in different states of single strands or double strands when performing signal acquisition at a certain temperature. For example, at a certain signal temperature, the first single-stranded pre-product is in a single-stranded state, and the second single-stranded pre-product binds to the probe to be in a double-stranded state. In certain embodiments, "higher than" is used for describing a difference of 4°C or above, e.g., 4-40°C, e.g., 4-30°C, 4-20°C, 4-18°C, 4-15°C, 4-12°C, 4-10°C, 4-8°C, 4-6°C, 4-5°C, or the like, between two annealing temperatures. In certain embodiments, "lower than" is used for describing a difference of 6°C or above, e.g., 6-46°C, e.g., 6-40°C, 6-35°C, 6-30°C, 6-25°C, 6-20°C, 6-18°C, 6-15°C, 6-12°C, 6-10°C, 6-8°C, 6-7°C, or the like, between two melting temperatures.

With the above primer-probe composition, by setting high-low differences between annealing temperatures as well as between melting temperatures, it may be achieved that when signal acquisition is performed at a certain temperature, at most one kind of single-stranded pre-product is in a double-stranded state with the probe, and therefore, at most one kind of signal of a target nucleic acid is generated ("at most" means that if the target nucleic acid is not presented in the sample to be detected, no signals will be generated; and if it is presented, only the signal of the kind of target nucleic acid is presented, and signals of other target nucleic acids will not be presented at the same time). Therefore, signals acquired at the same temperature do not overlap.

In certain embodiments, there are one kind of probe (a first probe) and two kinds of primer sets in the primer-probe composition; the two kinds of primer sets are a first primer set and a second primer set respectively; the first primer set and the second primer set specifically bind to the a first target nucleic acid and a second target nucleic acid respectively to generate a first single-stranded pre-product and a second single-stranded pre-product respectively, an annealing temperature of the first single-stranded pre-product and the probe is a first annealing temperature, and an annealing temperature of the second single-stranded pre-product and the probe is a second annealing temperature; and a double-stranded product generated by the first single-stranded pre-product and the probe is a first double-stranded product, a melting temperature of which is a first melting temperature, and a double-stranded product generated by the second single-stranded pre-product and the probe is a second double-stranded product, a melting temperature of which is a second melting temperature. After a PCR cycle is performed at an annealing temperature of T1 (the first annealing temperature ≥ T1 > the second annealing temperature), only the first double-stranded product is generated; then a first signal acquisition is performed at a temperature of t1 (t1 < the first melting temperature), at which time, only the first double-stranded product is in a double-stranded state, and a resulting signal is a signal of the first target nucleic acid; after a PCR cycle is then performed at an annealing temperature of T2 (the second annealing temperature ≥ T2), the second double-stranded product is generated, and the reaction system also contains the first double-stranded product; and then a second signal acquisition is performed at a temperature of t2 (the first melting temperature < t2 < the second melting temperature), at which time, only the second double-stranded product is in a double-stranded state, a resulting signal is a signal of the second target nucleic acid (since t2 > the first melting temperature, the first double-stranded product is in a melting state, no signals are generated).

Similarly, when three or more kinds of primer sets are contained, and when a certain temperature is used as an annealing temperature of PCR for PCR cyclic amplification, all single-stranded pre-products with an annealing temperature higher than that temperature can bind to the probe to form double-stranded products, and single-stranded pre-products with an annealing temperature lower than that temperature are still in a single-stranded state; and when signal acquisition is performed at a certain temperature, the double-stranded products of only one class of single-stranded pre-products and the probe are in a double-stranded state, and a detectable target nucleic acid signal is generated. As for other classes of single-stranded pre-products, part of the single-stranded pre-products cannot be annealed with the probe at the stage of PCR cylic amplification to form double-stranded products due to a low annealing temperature; and the other part of the single-stranded pre-products can be annealed with the probe at the stage of PCR cylic amplification to form double-stranded products because of a high annealing temperature, but since such double-stranded products have a melting temperature lower than a signal acquisition temperature, the resulting double-stranded products are also in a single-stranded state at the signal acquisition temperature).

In the present invention, the expressions of "first", "second", "third", "fourth", "fifth" and the like are only for the purpose of description so as to distinguish defined objects, instead of defining the order or primary and secondary in any way.

In certain embodiments, the above primer-probe composition for target nucleic acid detection includes a first primer set specific to a first target nucleic acid, the first primer set specifically binds to the first target nucleic acid to generate a first pre-product that contains a first single-stranded pre-product specifically binding to the probe, the first single-stranded pre-product specifically binds to the probe and extends by 0 base to form a first double-stranded product, and formation of the first double-stranded product causes a detectable signal change; an annealing temperature of the first single-stranded pre-product and the probe is higher than annealing temperatures of other single-stranded pre-products and the same probe; and a melting temperature of the first double-stranded product generated by the first single-stranded pre-product and the probe is lower than melting temperatures of double-stranded products generated by other single-stranded pre-products and the same probe.

In the embodiments, the first single-stranded pre-product generated by the primer set specific to a certain target nucleic acid (here described as "the first primer set) does not extend after specifically binding to the probe. A person skilled in the art may, according to actual demands, achieve the above effect of "specifically binding but not extending" by designing a primer sequence in the primer set and/or a sequence of the probe. For example, it is designed in such a way that the resulting first single-stranded pre-product binds to a 5' end of the probe, or it is designed in such a way that a 3' end of the resulting first single-stranded pre-product contains a region that is not complementarily paired with the probe.

The above "first" is only for the purposes of description and distinguishing so as to distinguish defined objects, instead of defining the order or primary and secondary in any way, and are not used for defining the number of classes of target nucleic acids, primer sets, pre-products, single-stranded pre-products, or double-stranded products. Here, it is only for the purpose of describing the situation that in the above primer-probe composition, there is one kind of primer set that generates a single-stranded pre-product that does not extend after specifically binding to the probe, and the single-stranded pre-product has the maximum annealing temperature with the probe (higher than the annealing temperatures of any other kinds of classes of single-stranded pre-products and the same probe), but the resulting double-stranded product has the lowest melting temperature (lower than the melting temperatures of any other classes of double-stranded products).

In some implementations of the present invention, the above primer-probe composition for target nucleic acid detection further includes a second probe and a second primer mixture; the second probe and the first probe have different base sequences and are modified with different detection labels; and the second primer mixture includes at least one kind of primer set, and different kinds of primer sets specifically bind to different kinds of target nucleic acids respectively.

In the implementations, the primer-probe composition includes another kind of probe (the second probe). The number of classes of probes is determined according to the number of classes of detection labels thereof. Different kinds of probes need to perform signal acquisitions under different detection channels due to different detection labels. In certain embodiments, different detection labels do not mean that detection groups for modification are completely different. For example, the detection groups include fluorophores and quenchers. Signals generated from two kinds of probes may also undergo signal acquisition in two detection channels (or fluorescence channels) as long as the fluorophores are different (the quenchers may be the same or different). In the embodiments, the increase in the number of classes of probes may achieve performing signal detection of different target nucleic acids under different channels, and thus may achieve more multiple detection of the target nucleic acids.

In the embodiments, the presence of "first probe" and "second probe" is not for the purpose of definition, nor is it used for defining that there are only two kinds of probes in the embodiments. Here, it is only used for distinguishing that "first probe" and "second probe" belong to different classes of probes, and the two kinds of probes are modified with different detection labels and have different base sequences. In certain embodiments, in order to detect more classes of target nucleic acids, the primer-probe composition for target nucleic acid detection further includes a third probe and a third primer mixture, a fourth probe and a fourth primer mixture, a fifth probe and a fifth primer mixture, a sixth probe and a sixth primer mixture, or more probes and primer mixtures. Different probes are modified with different detection labels and have different base sequences; and primer sets in different primer mixtures are also different. In other words, in order to achieve more multiple detection of the target nucleic acids, a person skilled in the art may design more kinds of primer sets corresponding to the target nucleic acids and more kinds of probes corresponding to the primer sets as needed.

In some implementations of the present invention, according to the primer-probe composition for target nucleic acid detection, the primer sets in the second primer mixture specifically bind to the corresponding target nucleic acids to generate a pre-product, the pre-product contains a single-stranded pre-product specifically binding to the second probe, and the single-stranded pre-product specifically binds to the second probe and extends by >_ 0 base to form a double-stranded product, and the formation of the double-stranded product causes a detectable signal change.

Preferably, the different primer sets in the second primer mixture and the corresponding target nucleic acids generate different single-stranded pre-products; and when an annealing temperature of a third single-stranded pre-product and the second probe is higher than an annealing temperature of a fourth single-stranded pre-product and the second probe, a melting temperature of a double-stranded product generated by the third single-stranded pre-product and the second probe is lower than a melting temperature of a double-stranded product generated by the fourth single-stranded pre-product and the second probe.

With the setting of high-low relationships between the annealing temperatures as well as between the melting temperatures, it may be achieved that a signal acquired at a certain detection temperature under a certain detection channel is a signal of at most one kind of target nucleic acid (the "at most" means that if the target nucleic acid is not presented in the sample to be detected, no signals will be generated; and if it is presented, only the signal of this kind of target nucleic acid is presented, and signals of other target nucleic acids will not be presented at the same time).

In the present invention, "third", "fourth" and the like are only for the purpose of description so as to distinguish defined objects, instead of defining the order or primary and secondary in any way. "Third" in the embodiments are only used for being distinguished from "fourth". That is, "third single-stranded pre-product" and "fourth single-stranded pre-product" belong to different classes, and target nucleic acids corresponding thereto belong to different classes.

In the present invention, "third", "fourth" and the like are only for the purpose of description so as to distinguish defined objects, instead of defining the number of classes of single-stranded pre-products. For example, the presence of "fourth single-stranded pre-product" in the embodiments is not used for defining that there are only four kinds of target nucleic acids in the embodiments. In certain embodiments, four or more kinds of target nucleic acids are further included, and four or more single-stranded pre-products are generated. In certain embodiments, the second primer mixture includes three or more kinds of primer sets. Annealing temperatures and melting temperatures of single-stranded pre-products generated by the primer sets and corresponding target nucleic acids are similar to those of the third single-stranded pre-product and the fourth single-stranded pre-product. That is, the lower the annealing temperature required for a single-stranded pre-product generated by a certain kind of primer set and a target nucleic acid thereof to specifically bind to the probe, the higher the melting temperature of a double-stranded product formed by the single-stranded pre-product specifically binding to the probe and extending by >_ 0 base. In order to achieve the following effects: when a certain temperature is used as an annealing temperature of PCR for PCR cyclic amplification, all single-stranded pre-products with an annealing temperature higher than that temperature can bind to the second probe to form double-stranded products, and single-stranded pre-products with an annealing temperature lower than that temperature are still in a single-stranded state; and when signal acquisition is performed at a certain temperature, the double-stranded products of only one class of single-stranded pre-products and the second probe are in a double-stranded state, and a detectable target nucleic acid signal is generated. As for other classes of single-stranded pre-products, part of the single-stranded pre-products cannot be annealed with the second probe at the stage of PCR cylic amplification to form double-stranded products due to a low annealing temperature; and the other part of the single-stranded pre-products can be annealed with the second probe at the stage of PCR cylic amplification to form double-stranded products because of a high annealing temperature, but since such double-stranded products have a melting temperature lower than a signal acquisition temperature, the resulting double-stranded products are also in a single-stranded state at the signal acquisition temperature).

In some implementations of the present invention, according to the above primer-probe composition for target nucleic acid detection, a melting temperature of a double-stranded product generated by the first single-stranded pre-product and the first probe is equal to or approximate to a melting temperature of a double-stranded product generated by the third single-stranded pre-product and the second probe; preferably, an annealing temperature of the first single-stranded pre-product and the first probe is equal to or approximate to an annealing temperature of the third single-stranded pre-product and the second probe.

In the implementations, the primer-probe composition includes two or more kinds of probes. The number of classes of probes is determined according to the number of classes of detection labels thereof. Different kinds of probes need to perform signal acquisition under different detection channels due to different detection labels.

In the present invention, the expressions of "equal to" and "approximate to" are used for describing that there is no difference between the two temperatures, or that the difference is small. Moreover, because the difference is small, or there is no difference, different classes of single-stranded pre-products achieve the same state of single strands or double strands when undergoing signal acquisition at a certain temperature. For example, at a certain temperature, both the first single-stranded pre-product and the third single-stranded pre-product are in a single-stranded state, or both the first single-stranded pre-product and the third single-stranded pre-product are in a double-stranded state. It is worth noting that although all the single-stranded pre-products may maintain a double-stranded state when undergoing signal acquisition at a certain temperature, the single-stranded pre-products bind different classes of probes in the double-stranded state. In certain embodiments, "equal to" and "approximate to" are used for describing a difference of within 4°C, e.g., 0-4°C, preferably, 0-2°C, more preferably, 0-1°C, between two annealing temperatures/ two melting temperatures.

In the implementations, "first", "second", "third" and the like are only for the purpose of description so as to distinguish defined objects, instead of defining the order or primary and secondary in any way, and are not used for defining the number of classes of single-stranded pre-products or probes. For example, the presence of "third single-stranded pre-product" in the embodiments is not used for defining that there are only three kinds of target nucleic acids and three kinds of single-stranded pre-products in the embodiments. "Third" in the embodiments are only for the purpose of being distinguished from "first". That is, "first single-stranded pre-product" and "third single-stranded pre-product" belong to different classes, and target nucleic acids corresponding thereto belong to different classes. For another example, the presence of "first probe" and "second probe" in the embodiments is not used for defining that there are only two kinds of probes in the embodiments. Here, it is only used for distinguishing that "first probe" and "second probe" belong to different classes of probes, and the two kinds of probes are modified with different detection labels and have different base sequences. The embodiments also include cases of three, or three or more kinds of probes. In order to achieve more multiple detection of the target nucleic acids, a person skilled in the art may design more kinds of probes as needed.

With the setting of the above same or similar melting temperatures, it may be achieved that when signal acquisition is performed at a certain temperature, two or more kinds of single-stranded pre-products may be in a double-stranded state with the probe. However, since at that temperature, different single-stranded pre-products in a double-stranded state bind to different kinds of probes, signal acquisition may be performed at the same temperature but under different detecting channels, so as to identify target nucleic acids corresponding to the different single-stranded pre-products. As such, more multiple detection may be achieved by increasing detection channels (fluorescence channels). In addition, the number of changes in signal acquisition temperature may further be reduced, that is, at a certain signal acquisition temperature, different targets may be detected only by converting a detection channel; the number of PCR cycles may also be reduced, that is, after a certain PCR cycle, compared to a previous PCR amplification, the number of classes of double-stranded products formed by each kind of probe is increased by at most one (if there are two kinds of probes, two kinds of double-stranded products are increased).

However, it is worth noting that when signal acquisition is performed at a certain temperature, two or more kinds of single-stranded pre-products may be in a double-stranded state with the probe not only through the setting of the above same or similar melting temperatures (different single-stranded pre-products in the double-stranded state bind to different kinds of probes). When the melting temperatures are not the same as or similar (e.g., the two melting temperatures differ by 6°C or above), by adjusting a signal acquisition temperature to be lower than melting temperatures of different double-stranded products generated by different single-stranded pre-products and diferent kinds of probes, the above effect may also be achieved. For example, a melting temperature of a double-stranded product generated by the first single-stranded pre-product and the first probe is Tm1, which is not equal to or approximate to a melting temperature Tm2 of a double-stranded product generated by the third single-stranded pre-product and the second probe, and by adjusting a signal acquisition temperature to be lower than Tm1 and Tm2, the two kinds of double-stranded products are still in a double-stranded state at the signal acquisition temperature.

In some implementations of the present invention, according to the above primer-probe composition for target nucleic acid detection, the first probe or the second probe, as one sequence that does not specifically bind to any target nucleic acids, includes a probe signal detection region (H), and sequences of probe signal detection regions (H) of different probes are different from each other;
each primer set includes a first primer and a second primer, and the first primer contains target sequence binding regions 1; the second primer contains primer signal detection regions (h) and target sequence binding regions 2, and the primer signal detection regions (h) are located at 5' ends of the target sequence binding regions 2; each primer signal detection region (h) is one sequence that does not specifically bind to any target nucleic acids and partially or wholly identical to the probe signal detection region (H) of the corresponding probe; and sequences of primer signal detection regions (h) of the second primers in different primer sets are different from each other.

In certain embodiments, the target sequence binding regions 1 and the target sequence binding regions 2 specifically bind to different locations of the target nucleic acids respectively.

In certain embodiments, each primer signal detection region (h) and the probe signal detection region (H) of the probe have partially or completely identical sequences, that is, reverse complementary sequences (h') of the primer signal detection regions (h) can specifically bind to part or all of the sequences of the probe signal detection regions (H) of the probes.

In certain embodiments, the above first primers and second primers specifically bind to the target nucleic acids to generate a pre-product, and the pre-product contains one single-stranded pre-product with the reverse complementary sequences (h') of the primer signal detection regions (h). The reverse complementary sequences (h') of the single-stranded pre-product specifically bind to the probe signal detection regions (H) of the probes and extend by >_ 0 base to form a double-stranded product, and the formation of the double-stranded product causes a detectable signal change.

In certain embodiments, the sequences of the primer signal detection regions (h) of the second primers in the different primer sets are different from each other. That is, the sequences of the primer signal detection regions (h) of the second primers in the different primer sets have different bases and/or lengths, so that reverse complementary sequences (h') of different single-stranded pre-products are different. The different single-stranded pre-products specifically bind to the probes and extend by 0 or more bases to form different double-stranded products, and the melting temperatures of the different double-stranded products can be separated from each other; alternatively, the different single-stranded pre-products specifically bind to different kinds of probes respectively and extend by >_ 0 base to form different double-stranded products, the melting temperatures of the different double-stranded products cannot be separated from each other, however, since the different double-stranded products have different kinds of probes, the different double-stranded products can be distinguished through different detection channels. That is, the different kinds of target nucleic acids can be distinguished through the melting temperatures and/or the detection channels to achieve multiple detection.

In certain embodiments, there is a gap of 0-20 bases between the primer signal detection regions (h) of the second primers and the target sequence binding regions 2. The number of the gap bases may be 0, 1, 2, 3, 4, 5, 6, 7, 8, 9, 10, 11, 12, 13, 14, 15, 16, 17, 18, 19, 20, or a range thereof.

In some implementations of the present invention, according to the above primer-probe composition for target nucleic acid detection, the first probe or the second probe further includes a primer anchoring region (A'); the first primer of the at least one kind of primer set in the first primer mixture or the second primer mixture further includes a probe anchoring region (A), and the probe anchoring region (A) is located at a 5' end of the target sequence binding region 1; and the probe anchoring region (A) does not specifically bind to any target nucleic acids but specifically binds to the primer anchoring region (A').

The first primers and the second primers in the above primer sets specifically bind to the target nucleic acids to generate a pre-product, and the pre-product contains one single-stranded pre-product with the probe anchoring regions (A) and the reverse complementary sequences (h') of the primer signal detection regions (h). The probe anchoring regions (A) and the reverse complementary sequences (h') of the single-stranded pre-product specifically bind to the primer anchoring regions (A') and the probe signal detection regions (H) of the probes respectively and extend by >_ 0 base to form a double-stranded product, and the formation of the double-stranded product causes a detectable signal change.

By adjusting the probe anchoring regions (A) of the first primers in the different primer sets and/or sequence compositions and/or lengths of the primer signal detection regions (h) of the second primers in the different primer sets, the different primer sets and the target nucleic acids thereof generate different single-stranded pre-products. The different single-stranded pre-products specifically bind to the probes to generate different annealing temperatures, and specifically bind and extend by >_0 base to form double-stranded products with different melting temperatures, so that the lower the annealing temperature required for a single-stranded pre-product generated by a certain kind of primer set and a target nucleic acid thereof to specifically bind to a probe, the higher the melting temperature of a double-stranded product formed by the single-stranded pre-product specifically binding to the probe and extending by >_0 base.

In certain embodiments, the target sequence binding regions 1 of the first primers in the different primer sets have different sequences; the probe anchoring regions (A) of the first primers in the different primer sets may be the same or different; preferably, there is a gap of 0-20 gaps between the probe anchoring regions (A) of the first primers and the target sequence binding regions 1. The number of the gap bases may be 0, 1, 2, 3, 4, 5, 6, 7, 8, 9, 10, 11, 12, 13, 14, 15, 16, 17, 18, 19, 20, or a range thereof.

In some implementations of the present invention, according to the above primer-probe composition for target nucleic acid detection, in the first primer mixture or the second primer mixture, the second primer of at most one kind of primer set further includes an extension block region (M), the extension block region (M) is located at a 5' end of the primer signal detection region (h), and neither the extension block region (M) nor a complementary sequence thereof specifically binds to any probes or any target nucleic acids.

The above each first primer and each second primer containing the extension block region (M) specifically bind to target nucleic acids respectively to generate a pre-product, and the pre-product contains one single-stranded pre-product with the reverse complementary sequence (h') of the primer signal detection region (h). The reverse complementary sequence (h') of the single-stranded pre-product specifically binds to the probe and extends by 0 base to form a double-stranded product (due to the presence of the extension block region, the single-stranded pre-product specifically binds to the probe and does not extend), thereby causing the probe to generate a detectable signal change.

In some embodiments of the present invention, a single-stranded pre-product generated after a primer specifically binds to a target nucleic acid does not extend after specifically binding to a probe (i.e., extending by 0 base). A person skilled in the art may, according to actual demands, achieve the above effect of "specifically binding but not extending" by designing a primer sequence in the primer set and/or a sequence of the probe. For example, it is designed in such a way that the resulting first single-stranded pre-product binds to a 5' end of the probe, or it is designed in such a way that a 3' end of the resulting first single-stranded pre-product contains a region that is not complementarily paired with the probe.

"First primer mixture", "second primer mixture" and the like are only for the purpose of description so as to distinguish defined objects. "First primer mixture" and "second primer mixture" each represent one set of primer mixtures that specifically binds to probes. The primer mixtures include a plurality of primer sets, and reverse complementary sequences (h') of primer signal detection regions (h) contained in second primers in the primer sets each may specifically bind to the same probe. However, the primer sets in the "first primer mixture" and the "second primer mixture" specifically bind to different kinds of probes.

In certain embodiments, in one set of primer mixtures that specifically binds to the same kind of probe, the second primer of at most one kind of primer set contains an extension block region (M). A single-stranded pre-product generated after the primer set specifically binds to a target nucleic acid requires a maximum annealing temperature for specifically binding to the probe, and specifically binds to the probe and does not extend to generate a double-stranded product with a lowest melting temperature.

In the present invention, "first primer mixture" and "second primer mixture" are only for the purpose of description so as to distinguish defined objects, instead of defining the order or primary and secondary in any way. For example, in the above primer-probe composition, the first primers and the second primers of the primer sets are structurally interchangeable. For example, the first primers contain primer signal detection regions (h) and target sequence binding regions, and the second primers contain target sequence binding regions; and for another example, the first primers contain primer signal detection regions (h) and target sequence binding regions, and the second primers contain probe anchoring regions (A) and target sequence binding regions. In certain embodiments, "first primer" is also known as "forward primer", and "second primer" is also known as "reverse primer".

In the present invention, the probe is one freely-designed sequence that is not paired with or binding to any target nucleic acids, and the probe is modified with detection labels. Preferably, the detection labels include a first detection group and a second detection group, and the first detection group and the second detection group generate a change in signal through a change in distance; preferably, the first detection group and the second detection group are spaced by 3-250 angstroms; preferably, by 3-201 angstroms; more preferably, by 3-140 angstroms; and/or, the first detection group is a reporter dye, and the second detection group is a quencher or other modification groups that can generate a signal change with the first detection group through fluorescence resonance energy transfer.

In the present invention, locations of the first detection group and the second detection group on the probe make the reverse complementary sequence (h') of the single-stranded pre-product specifically bind to the probe (P) and extend by >_ 0 base to form a double-stranded product. The probe may generate a detectable signal change as long as the formation of the double-stranded product can cause the locations of the first detection group and the second detection group to change. The locations of the first detection group and the second detection group are interchangeable.

In certain embodiments, in the absence of target nucleic acids to be detected, no single-stranded pre-products specifically bind to the probes, the probes are in a single-stranded state or have other secondary structures, at which time, there is a relatively short distance between the first detection group and the second detection group, and the efficiency of fluorescence resonance energy transfer is relatively high; and if the first primers contain probe anchoring regions (A), in the absence of the target nucleic acids to be detected, even if primer anchoring regions (A') of the probes are complementary with the probe anchoring regions (A) in the first primers, other parts of the probes are in a single-stranded state or have other secondary structures, at which time, there is still a relatively short distance between the first detection group and the second detection group, and the efficiency of fluorescence resonance energy transfer is relatively high. However, in the presence of the target nucleic acids to be detected, the first primers and the second primers specifically bind to target sequences respectively and extend to generate a double-stranded amplification product, with one single-stranded amplification product specifically binding to the probes to form a double-stranded product. At this time, the distance between the first detection group and the second detection group becomes longer, and the efficiency of fluorescence resonance energy transfer is reduced, so that a fluorescence signal changes and can be detected by an instrument.

For this purpose, in a fourth aspect, the present invention provides a PCR detection kit, including the primer-probe composition for target nucleic acid detection in the third aspect. It is known to a person skilled in the art that in order to achieve PCR detection, the kit further includes an amplification reagent for amplification, such as a DNA polymerase and dNTPs. When a nucleic acid template is RNA, the amplification reagent further includes an reverse transcriptase. The amplification reagent further includes some reagents that promote PCR reaction, such as KCl, MgCl₂, Tris-HCl, dithiothreitol (DTT) and (NH₄)₂SO₄. In certain preferred embodiments, the PCR detection kit provided by the present invention is a digital PCR detection kit, including the primer-probe composition for target nucleic acid detection in the third aspect.

For this purpose, in a fifth aspect, the present invention provides a multiple time-sharing nucleic acid detection device, including:
a reaction liquid containing part, configured to contain a plurality of micro liquids containing a sample and a reagent;
a temperature adjustment part, configured to adjust a temperature of the micro liquids in the reaction liquid containing part;
a signal detection part, configured to detect a signal generated by the micro liquids in the reaction liquid containing part;
a control part,
the control part controlling the temperature adjustment part to adjust a temperature of the reaction liquid containing part in a first temperature adjustment mode in such a way that among the plurality of micro liquids, only a first part of micro liquids generates a valid signal; and controlling the signal detection part to perform signal detection on the reaction liquid containing part to obtain a first signal;
the control part controlling the temperature adjustment part to adjust the temperature of the reaction liquid containing part in a second temperature adjustment mode in such a way that among the plurality of micro liquids contained in the same reaction liquid containing part, only a second part of micro liquids different from the first part of micro liquids generates a valid signal; and controlling the signal detection part to perform signal detection on the reaction liquid containing part to obtain a second signal.

The micro liquids are mixtures containing a sample and a reagent.

The above first temperature adjustment mode is different from the second temperature adjustment mode. In certain embodiments, the difference refers to a difference in lowest temperatures for the two temperature adjustment modes. In certain embodiments, the lowest temperature for the first temperature adjustment mode is higher than the lowest temperature for the second temperature adjustment mode.

In one embodiment, the control part controls the temperature adjustment part to adjust the temperature of the micro liquids in the reaction liquid containing part into a state of temperature t2, and controls the signal detection part to perform signal detection on the reaction liquid containing part to obtain the first signal,
the control part controls the temperature adjustment part to adjust the temperature of the micro liquids in the reaction liquid containing part into a state of temperature t3, and controls the signal detection part to perform signal detection on the reaction liquid containing part to obtain the second signal,
wherein, t2 < t3.

In certain embodiments, the temperatures of t2 and t3 differ by 6°C or above, e.g., 6-46°C, e.g., 6-40°C, 6-35°C, 6-30°C, 6-25°C, 6-20°C, 6-18°C, 6-15°C, 6-12°C, 6-10°C, 6-8°C, 6-7°C, or the like.

In one embodiment, the reagent includes a first reagent and a second reagent;
when the temperature of the micro liquids in the reaction liquid containing part is t2, the first reagent in the first part of micro liquids is combined with the sample to generate a signal,
when the temperature of the micro liquids in the reaction liquid containing part is t3, the second reagent in the second part of micro liquids is combined with the sample to generate a signal.

In one other embodiment, the first temperature adjustment mode includes an incubation stage and a signal acquisition stage,
at the incubation stage, the temperature adjustment part periodically changes the temperature of the micro liquids in the reaction liquid containing part, so that the temperature of the reaction liquid containing part is repeatedly reduced from a high temperature to a low temperature T2, or the temperature adjustment part maintains the reaction liquid containing part at a constant temperature T2,
at the signal acquisition stage, the signal detection part performs signal detection on the reaction liquid containing part to obtain the first signal.

In one embodiment, the second temperature adjustment mode includes an incubation stage and a signal acquisition stage,
at the incubation stage, the temperature adjustment part periodically changes the temperature of the micro liquids in the reaction liquid containing part, so that the temperature of the reaction liquid containing part is repeatedly reduced from a high temperature to a low temperature T3, or the temperature adjustment part maintains the reaction liquid containing part at a constant temperature T3,
at the signal acquisition stage, the signal detection part performs signal detection on the reaction liquid containing part to obtain the second signal,
wherein, T2 > T3, and t2 > T3.

In certain embodiments, the temperatures of T2 and T3 differ by 4°C or above, e.g., 4-40°C, e.g., 4-30°C, 4-20°C, 4-18°C, 4-15°C, 4-12°C, 4-10°C, 4-8°C, 4-6°C, 4-5°C, or the like.

In certain embodiments, the temperatures of t2 and T3 differ by 5°C or above, e.g., 5-50°C, e.g., 5-40°C, 5-30°C, 5-20°C, 5-18°C, 5-15°C, 5-12°C, 5-10°C, 5-8°C, 5-7°C, 5-6°C, or the like.

In one embodiment, when the temperature of the micro liquids in the reaction liquid containing part is T2, the first reagent in the first part of micro liquids is combined with the sample to generate a signal;
when the temperature of the micro liquids in the reaction liquid containing part is T3, the first reagent in the first part of micro liquids is combined with the sample to generate a signal, and the second reagent in the second part of micro liquids is combined with the sample to generate a signal.

In one embodiment, before the control part controls the temperature adjustment part to adjust the temperature of the micro liquids in the reaction liquid containing part in the first temperature adjustment mode and the second temperature adjustment mode:
the control part controls the temperature adjustment part to periodically change the temperature of the micro liquids in the reaction liquid containing part so as to repeatedly reduce the temperature of the reaction liquid containing part from a high temperature to a low temperature T1, so that among the plurality of micro liquids, only a fourth part of micro liquids generate a valid signal, and the fourth part of micro liquids is different from both the first part of micro liquids and the second part of micro liquids, wherein, T1 > T2.

In certain embodiments, the temperatures of T1 and T2 differ by 4°C or above, e.g., 4-40°C, e.g., 4-30°C, 4-20°C, 4-18°C, 4-15°C, 4-12°C, 4-10°C, 4-8°C, 4-6°C, 4-5°C, or the like.

In one embodiment, the reagent further includes a fourth reagent;
when the temperature of the micro liquids in the reaction liquid containing part is T1, the fourth reagent in the fourth part of micro liquids is combined with the sample to generate a signal;
when the temperature of the micro liquids in the reaction liquid containing part is T2, the fourth reagent in the fourth part of micro liquids is combined with the sample to generate a signal, and the first reagent in the first part of micro liquids is combined with the sample to generate a signal;
when the temperature of the micro liquids in the reaction liquid containing part is T3, the fourth reagent in the fourth part of micro liquids is combined with the sample to generate a signal, the first reagent in the first part of micro liquids is combined with the sample to generate a signal, and the second reagent in the second part of micro liquids is combined with the sample to generate a signal.

In one embodiment, the signal detection part is controlled to perform signal detection on the reaction liquid containing part to obtain a fourth signal.

In one embodiment, in the process of the signal detection part performing signal detection on the reaction liquid containing part to obtain the fourth signal, the control part controls the temperature adjustment part to adjust the temperature of the micro liquids in the reaction liquid containing part to t1, or to be maintained at T1,
in the state of the temperature of the micro liquids in the reaction liquid containing part being T1 or t1, the fourth reagent in the fourth part of micro liquids maintains combined with the sample and continues to generate a signal.

In one embodiment, in an initial state, at most one unit of sample is distributed to the micro liquids.

In one embodiment, the signal is a fluorescence signal, and the signal detection part is a camera.

In one embodiment, the control part controls the signal detection part to perform signal detection on the reaction liquid containing part at a position of the reaction liquid containing part that is flatly paved with micro liquids.

In one embodiment, the control part controls the temperature adjustment part to perform temperature adjustment on the reaction liquid containing part at a position of the reaction liquid containing part that is flatly paved with micro liquids.

For this purpose, in a sixth aspect, the present invention provides a detection method for detecting a plurality of micro liquids containing a sample and a reagent, including the steps:
adjusting a temperature of a plurality of micro liquids in a first temperature adjustment mode in such a way that among the plurality of micro liquids, only a first part of micro liquids generates a valid signal; performing signal detection on the plurality of micro liquids to obtain a first signal;
adjusting the temperature of the micro liquids in a second temperature adjustment mode in such a way that among the plurality of micro liquids, only a second part of micro liquids generates a valid signal, the second part of micro liquids being different from the first part of micro liquids; performing signal detection on the plurality of micro liquids to obtain a second signal.

In one embodiment, the temperature of the plurality of micro liquids is adjusted into a state of t2, and signal detection is performed on the plurality of micro liquids to obtain the first signal,
the temperature of the plurality of micro liquids is adjusted into a state of t3, and signal detection is performed on the plurality of micro liquids to obtain the second signal,
wherein, t2 < t3.

In certain embodiments, the temperatures of t2 and t3 differ by 6°C or above, e.g., 6-46°C, e.g., 6-40°C, 6-35°C, 6-30°C, 6-25°C, 6-20°C, 6-18°C, 6-15°C, 6-12°C, 6-10°C, 6-8°C, 6-7°C, or the like.

In one embodiment, the reagent includes a first reagent and a second reagent;
when the temperature of the micro liquids in the reaction liquid containing part is t2, the first reagent in the first part of micro liquids is combined with the sample to generate a signal,
when the temperature of the micro liquids in the reaction liquid containing part is t3, the second reagent in the second part of micro liquids is combined with the sample to generate a signal.

In one embodiment, the first temperature adjustment mode includes an incubation stage and a signal acquisition stage,
at the incubation stage, the temperature of the plurality of micro liquids is adjusted to repeatedly reduce the temperature of the plurality of micro liquids from a high temperature to a low temperature T2, or the temperature of the plurality of micro liquids is adjusted to maintain the plurality of micro liquids at a constant temperature T2,
at the signal acquisition stage, signal detection is performed on the plurality of micro liquids to obtain the first signal.

In one embodiment, the second temperature adjustment mode includes an incubation stage and a signal acquisition stage,
at the incubation stage, the temperature of the plurality of micro liquids is adjusted to repeatedly reduce the temperature of the plurality of micro liquids from a high temperature to a low temperature T3, or the temperature of the plurality of micro liquids is adjusted to maintain the plurality of micro liquids at a constant temperature T3,
at the signal acquisition stage, signal detection is performed on the plurality of micro liquids to obtain the second signal.
wherein, T2 > T3, and t2 > T3.

In certain embodiments, the temperatures of T2 and T3 differ by 4°C or above, e.g., 4-40°C, e.g., 4-30°C, 4-20°C, 4-18°C, 4-15°C, 4-12°C, 4-10°C, 4-8°C, 4-6°C, 4-5°C, or the like.

In certain embodiments, the temperatures of t2 and T3 differ by 5°C or above, e.g., 5-50°C, e.g., 5-40°C, 5-30°C, 5-20°C, 5-18°C, 5-15°C, 5-12°C, 5-10°C, 5-8°C, 5-7°C, 5-6°C, or the like.

In one embodiment, when the temperature of the plurality of micro liquids is T2, the first reagent in the first part of micro liquids is combined with the sample to generate a signal;
when the temperature of the plurality of micro liquids is T3, the first reagent in the first part of micro liquids is combined with the sample to generate a signal, and the second reagent in the second part of micro liquids is combined with the sample to generate a signal.

In one embodiment, before adjusting the temperature of the plurality of micro liquids in the first temperature adjustment mode and the second temperature adjustment mode:
periodically changing the temperature of the plurality of micro liquids to repeatedly reduce the temperature of the plurality of micro liquids from a high temperature to a low temperature T1, so that among the plurality of micro liquids, only a fourth part of micro liquids generate a valid signal, the fourth part of micro liquids being different from both the first part of micro liquids and the second part of micro liquids, wherein, T1 > T2.

In certain embodiments, the temperatures of T1 and T2 differ by 4°C or above, e.g., 4-40°C, e.g., 4-30°C, 4-20°C, 4-18°C, 4-15°C, 4-12°C, 4-10°C, 4-8°C, 4-6°C, 4-5°C, or the like.

In one embodiment, the reagent further includes a fourth reagent;
when the temperature of the plurality of micro liquids is T1, the fourth reagent in the fourth part of micro liquids is combined with the sample to generate a signal;
when the temperature of the plurality of micro liquids is T2, the fourth reagent in the fourth part of micro liquids is combined with the sample to generate a signal, and the first reagent in the first part of micro liquids is combined with the sample to generate a signal.
when the temperature of the plurality of micro liquids is T3, the fourth reagent in the fourth part of micro liquids is combined with the sample to generate a signal, the first reagent in the first part of micro liquids is combined with the sample to generate a signal, and the second reagent in the second part of micro liquids is combined with the sample to generate a signal.

In one embodiment, signal detection is performed on the plurality of micro liquids to obtain the fourth signal.

In one embodiment, in the process of performing signal detection on the plurality of micro liquids to obtain the fourth signal, the temperature of the plurality of micro liquids is adjusted to t1 or maintains at T1,
in the state of the temperature of the plurality of micro liquids being T1 or t1, the fourth reagent in the fourth part of micro liquids maintains combined with the sample and continues to generate a signal.

In one embodiment, in an initial state, at most one unit of sample is distributed to the micro liquids.

In one embodiment, the signal is a fluorescence signal, and signal detection is performed on the plurality of micro liquids through a camera.

Compared with the prior art, the detection method and primer-probe composition according to the present invention have the following advantages.
(1) non-crossing detection signals: according to the detection method and primer-probe composition provided by the present invention, there is at most one kind of detection signal of a target nucleic acid in each signal acquisition for each fluorescence channel, and the detection result is more intuitive and accurate, thereby avoiding overlapping of two or more kinds of fluorescence signals of the target nucleic acid and a resulting detection error;
(2) multiple detection: the detection method and primer-probe composition provided by the present invention can simultaneously perform analysis in single-tube reaction in two dimensions of a fluorescence channel and a melting temperature. That is, by using the same fluorescence channel, different targets (target nucleic acids) can be detected through melting temperature characteristics; or by using different fluorescence channels, the detection of the classes of the targets which are a product of the number of the fluorescence channels and the melting temperature characteristics can be achieved;
(3) low fluorescence background: only by using one kind of probe for each fluorescence channel, distinguishing can be performed through different melting temperatures of different single-stranded pre-products and the probe, which greatly reduces a fluorescence background in digital PCR reaction and improves reaction sensitivity; and meanwhile, the number of fluorescence probes in the reagent is reduced, which greatly reduces the reagent cost and is conducive to large-scale popularization and application;
(4) adjustable melting temperature: the method provided by the present invention uses different melting temperatures of a secondary amplification product (the target nucleic acid is amplified with the primer set to generate a single-stranded pre-product, which is a "primary amplification product"; and the single-stranded pre-product specifically binds to the probe to form a double-stranded product, which is a "Secondary amplification product") for distinguishing, and therefore, by adjusting the length or sequence composition of the primer signal detection region (h) or adjusting the location at which the primer signal detection region (h) is reversely complementary with the full signal detection region (H) of the probe (P), the melting temperature of the double-stranded product formed with the probe (P) is increased or reduced, thereby adjusting different signal acquisition temperatures;
(5) good inclusiveness: according to the primer probe design method of the present invention, there are two parts (the first primer and the second primer) complementarily paired with the target sequence (target nucleic acid) (the probe thereof is not complementarily paired with the target nucleic acid), and compared with a Taqman hydrolysis probe method which requires three parts (the first primer, the second primer and the Taqman probe) to be complementarily paired with the target sequence, the primer probe design method of the present invention has better inclusiveness and less difficulty in designing when a number of hypervariable regions, such as viral or bacterial genomes, are presented in the target sequence;
(6) less sample consumption: the primer-probe composition and detection method provided by the present invention consumes few samples and thus is particularly suitable for detecting rare samples, which can greatly increases the concentration of the samples added into detection reaction, thereby improving the sensitivity of detection, and at the same time, single-tube reaction provides the possibility of subsequent extraction-free one-tube detection;
(7) high sensitivity: the method according to the present invention has very low requirements for the length of the target sequence, and when the type of the target is a short-fragment nucleic acid, such as a free nucleic acid, the shorter target sequence has higher sensitivity in detection;
(8) a wide range of application: the method according to the present invention is applicable to nucleic acid detection of multiple sample types, including a serum sample, a plasma sample, a whole blood sample, a sputum sample, a swab sample, a lavage fluid sample, a fresh tissue sample, a formalin-fixed and paraffin-embedded tissue (FFPE), etc.

Compared with the prior art, the multiple time-sharing nucleic acid detection device and detection method of the present invention have the advantages that different nucleic acids to be detected generating valid signals at different times can be achieved, that is, the signals from the different nucleic acids to be detected are isolated to avoid the station of signal overlapping and a resulting detection error, and to achieve time-sharing and high-precision detection of multiple the nucleic acid.

### Brief Description of the Drawings

The drawings are used to provide a further understanding of the present invention and constitute a part of the description to explain the present invention together with the specific implementations below, but do not constitute a limitation on the present invention. In the drawings,

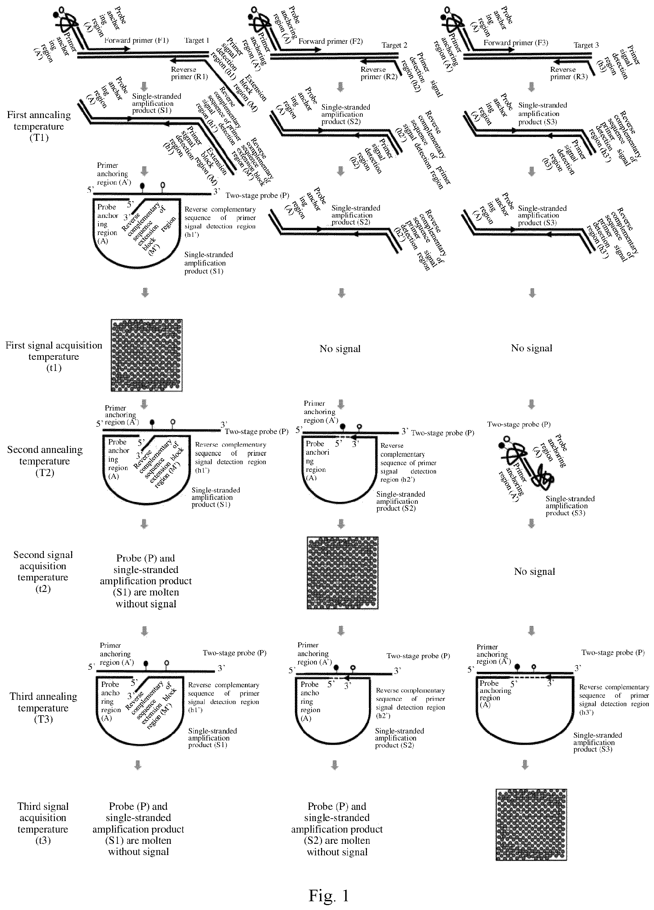

Fig. 1 is a design principle diagram of one kind of primer probe in the present invention (for three kinds of targets);
   when a target sequence is not presented in the sample or amplification has not yet begun, only a primer anchoring region (A') of a probe (P) is reversely complementary with a probe anchoring region (A) of a forward primer (F 1/2/3), other parts of the probe do not interact with any sequences, and at this time, a distance between a first detection group and a second detection group for modifying the probe (P) is d1; during PCR amplification, annealing is performed at a relatively high first annealing temperature (T1), a forward primer (F1) and a reverse primer (R1) specifically bind to a target sequence 1 respectively and extend to generate a double-stranded pre-product, with one single-stranded pre-product (S1) sequentially having a probe anchoring region (A1), a target sequence, a reverse complementary sequence (h1') of a primer signal detection region, and a reverse complementary sequence (M') of an extension block region from a 5' end to a 3' end; a forward primer (F2) and a reverse primer (R2) specifically bind to a target sequence 2 respectively and extend to generate a double-stranded pre-product, with one single-stranded pre-product (S2) sequentially having a probe anchoring region (A2), a target sequence, and a reverse complementary sequence (h2') of a primer signal detection region from a 5' end to a 3' end; optionally, if a forward primer (F3) and a reverse primer (R3) are presented, the forward primer (F3) and the reverse primer (R3) specifically bind to a target sequence 3 respectively and extend to generate a double-stranded pre-product, with one single-stranded pre-product (S3) sequentially having a probe anchoring region (A3), a target sequence, and a reverse complementary sequence (h3') of a primer signal detection region from a 5' end to a 3' end. After PCR amplification is completed, a first signal acquisition is performed at a relatively low first signal acquisition temperature (t1). At this time, the probe signal detection region (H) of the probe (P) is reversely and complementarily paired with the reverse complementary sequence (h1') of the primer signal detection region of the single-stranded pre-product (S1), a primer anchoring region (A1') of the probe (P) is reversely and complementarily paired with the probe anchoring region (A1) at the 5' end of the single-stranded pre-product (S1), to obtain a double-stranded product (D1) formed with the probe (P), while the reverse complementary sequence (M') of the extension block region at the 3' end of the single-stranded pre-product (S1) is not complementary with any parts of the probe (P) or the target sequence; at this time, all or part of sequences of the probe (P) changes from a single-stranded state to a double-stranded state, the distance between the first detection group and the second detection group changes, thus generating a signal that may be detected by an instrument; while at this time, both the reverse complementary sequence (h2') of the primer signal detection region at the 3' end of the single-stranded pre-product (S2) and the reverse complementary sequence (h3') of the primer signal detection region at the 3' end of the single-stranded pre-product (S3) have annealing temperatures that are lower than the first annealing temperature (T1) and the first signal acquisition temperature (t1), and thus are not reversely complementary with the probe signal detection region (H) of the probe (P) and do not generate extending; after the completion of the first signal acquisition at the first signal acquisition temperature (t1), a secondary amplification cycle of the single-stranded pre-product (S2) is performed with a cycle condition of high-temperature melting, and annealing is performed at a relatively low second annealing temperature (T2) (T2 < T1, and T2 < t1), at which time, the probe signal detection region (H) of the probe (P) is complementarily paired with the reverse complementary sequence (h2') of the primer signal detection region at the 3' end of the single-stranded pre-product (S2), the primer anchoring region (A2') of the probe is reversely and complementarily paired with the probe anchoring region (A2) at the 5' end of the single-stranded pre-product (S2), the 3' end of the single-stranded pre-product (S2) may continues extending to the 5' end of the probe (P), to obtain part or all of reverse complementary sequences of the probe (P), i.e., completing secondary amplification with the single-stranded pre-product (S2) as a primer and the probe (P) as a template, and an amplified double-stranded product (D2) formed with the probe (P) is obtained, at which time, all or part of sequences of the probe (P) changes from the single-stranded state to the double-stranded state, and the distance between the first detection group and the second detection group changes, thus generating a signal; after the completion of cyclic amplification at the second annealing temperature (T2), the temperature is increased to a relatively high second signal acquisition temperature (t2) for a second signal acquisition (t2 > t1, and t2 > T1), and at this time, since the second signal acquisition temperature (t2) is higher than a melting temperature of the hybrid double-stranded product (D1), the hybrid double-stranded product (D1) formed by the single-stranded pre-product (S1) and the probe (P) cannot form a double-stranded structure; the reverse complementary sequence (h3') of the primer signal detection region at the 3' end of the single-stranded pre-product (S3) has an annealing temperature that is lower than the second annealing temperature (T2), and thus is not reversely complementary with the probe signal detection region (H) of the probe (P) and do not generate extending; while an amplified double-stranded product (D2) formed by the single-stranded pre-product (S2) and the probe (P) forms a double-stranded state, the distance between the first detection group and the second detection group becomes longer, and the efficiency of fluorescence resonance energy transfer is relatively low, so that a fluorescence signal changes and may be detected by the instrument; after the completion of the second signal acquisition at the second signal acquisition temperature (t2), secondary amplification cycle of the single-stranded product (S3) is performed, with cycle condition of high-temperature melting, and annealing is performed at a lower third annealing temperature (T3) (T3 < T2, and T3 < t2), at which time, the probe signal detection region (H) of the probe (P) is complementarily paired with the reverse complementary sequence (h3') of the primer signal detection region at the 3' end of the single-stranded pre-product (S3), the primer anchoring region (A3') of the probe is reversely and complementarily paired with the probe anchoring region (A3) at the 5' end of the single-stranded pre-product (S3), the 3' end of the single-stranded pre-product (S3) may continue extending to the 5' end of the probe (P), to obtain part or all of reverse complementary sequences of the probe (P), i.e., completing secondary amplification with the single-stranded pre-product (S3) as a primer and the probe (P) as a template, and an amplified double-stranded product (D3) formed with the probe (P) is obtained, at which time, all or part of sequences of the probe (P) changes from the single-stranded state to the double-stranded state, the distance between the first detection group and the second detection group changes, thus generating a signal; after the completion of cyclic amplification at the third annealing temperature (T3), the temperature is increased to a higher third signal acquisition temperature (t3) for a second signal acquisition (t3 > t2, and t3 > T2), and at this time, since the third signal acquisition temperature (t3) is higher than the melting temperature of the hybrid double-stranded product (D1), the hybrid double-stranded product (D1) formed by the single-stranded pre-product (S1) and the probe (P) cannot form a double-stranded structure; since the third signal acquisition temperature (t3) is higher than the melting temperature of the amplified double-stranded product (D2), the amplified double-stranded product (D2) formed by the single-stranded pre-product (S2) and the probe (P) cannot form a double-stranded structure; while the amplified double-stranded product (D3) formed by the single-stranded pre-product (S3) and the probe (P) forms a double-stranded structure, the distance between the first detection group and the second detection group becomes longer, and the efficiency of fluorescence resonance energy transfer is relatively low, so that the fluorescence signal changes and can be detected by the instrument.
Fig. 2A is a diagram of signals of digital PCR detection reaction of Example 1 at a first signal acquisition temperature (60°C); and Fig. 2B is a diagram of signals of digital PCR detection reaction of Example 1 at a second signal acquisition temperature (78°C).
Fig. 3A is a diagram of signals of digital PCR detection reaction of Example 2 at the first signal acquisition temperature (60°C); and Fig. 3B is a diagram of signals of digital PCR detection reaction of Example 2 at the second signal acquisition temperature (78°C).
Fig. 4A is a diagram of signals of digital PCR detection reaction of Example 3 at the first signal acquisition temperature (60°C); and Fig. 4B is a diagram of signals of digital PCR detection reaction of Example 3 at the second signal acquisition temperature (78°C).
Fig. 5A is a diagram of signals of digital PCR detection reaction of Example 4 at a first signal acquisition temperature (56°C); and Fig. 5B is a diagram of signals of digital PCR detection reaction of Example 4 at a second signal acquisition temperature (72°C).
Fig. 6A is a diagram of signals of digital PCR detection reaction of Example 5 at a first signal acquisition temperature (55°C); Fig. 6B is a diagram of signals of digital PCR detection reaction of Example 5 at a second signal acquisition temperature (65°C); and Fig. 3C is a diagram of signals of digital PCR detection reaction of Example 5 at a third signal acquisition temperature (73°C).
Fig. 7A is a diagram of signals of digital PCR detection reaction of Example 6 at a first signal acquisition temperature (72°C), and positive droplets different from the background can be seen from the figures; and Fig. 7B is a diagram of signals of digital PCR detection reaction of Example 6 at a second signal acquisition temperature (82°C), and it can be seen from the figures that all droplets are negative.

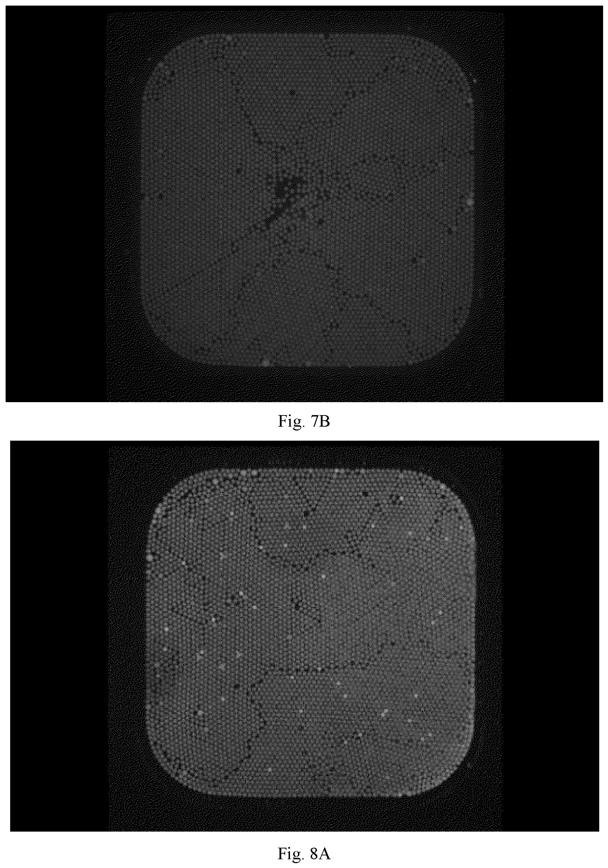

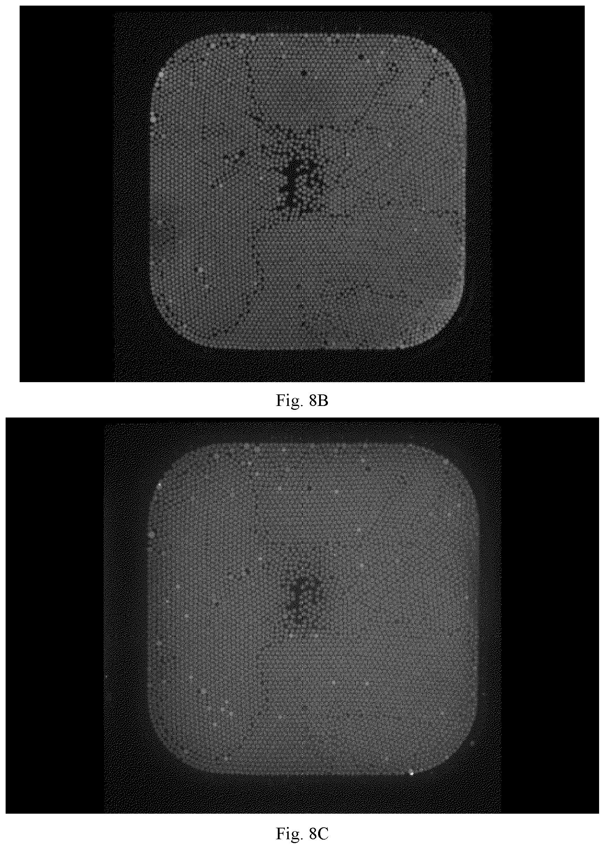

Fig. 8Ais a diagram of signals acquired at the first signal acquisition temperature (60°C) under a VIC fluorescence channel in Example 7, and positive droplets different from the background can be seen; Fig. 8B is a diagram of signals acquired at a second signal acquisition temperature (68°C) under the VIC fluorescence channel in Example 7, positive droplets different from the background can be seen, and positions of the positive droplets presented at that temperature and under that channel are different from those of the positive droplets presented at the first signal acquisition temperature (60°C) and under the VIC channel; and Fig. 8C is a diagram of signals acquired at the second signal acquisition temperature (68°C) under a FAM fluorescence channel in Example 7, positive droplets different from the background can be seen, and the positions of the positive droplets presented at that temperature and under that channel are different from those of the positive droplets presented at the first signal acquisition temperature (60°C) and under the VIC channel and the positive droplets presented at the second signal acquisition temperature (68°C) and under the VIC channel.
Fig. 9A is a diagram of signals of Example 8 at the first signal acquisition temperature (60°C). Fig. 9B is a diagram of signals of Example 8 at the second signal acquisition temperature (78°C).
Fig. 10A is a diagram of signals of Example 9 at the first signal acquisition temperature (60°C). Fig. 10B is a diagram of signals of Example 9 at the second signal acquisition temperature (78°C).
Fig. 11A is a diagram of signals of Example 10 at the first signal acquisition temperature (60°C). Fig. 11B is a diagram of signals of Example 10 at the second signal acquisition temperature (78°C).
Fig. 12A is a diagram of signals of Example 11 at the first signal acquisition temperature (60°C). Fig. 12B is a diagram of signals of Example 11 at the second signal acquisition temperature (78°C).
Fig. 13A is a diagram of signals of Example 1 at a first signal acquisition temperature (61°C). Fig. 13B is a diagram of signals of Example 12 at the second signal acquisition temperature (68°C). Fig. 13C is a diagram of signals of Example 12 at a third signal acquisition temperature (76°C).
Fig. 14A is a schematic diagram of a multiple time-sharing nucleic acid detection device disclosed by an embodiment of the present application; Fig. 14B is a schematic diagram of a multiple time-sharing nucleic acid detection device disclosed by another embodiment of the present application; and Fig. 14C is a structural diagram of a well plate composed of a plurality of reaction liquid containing parts disclosed by yet another embodiment of the present application.

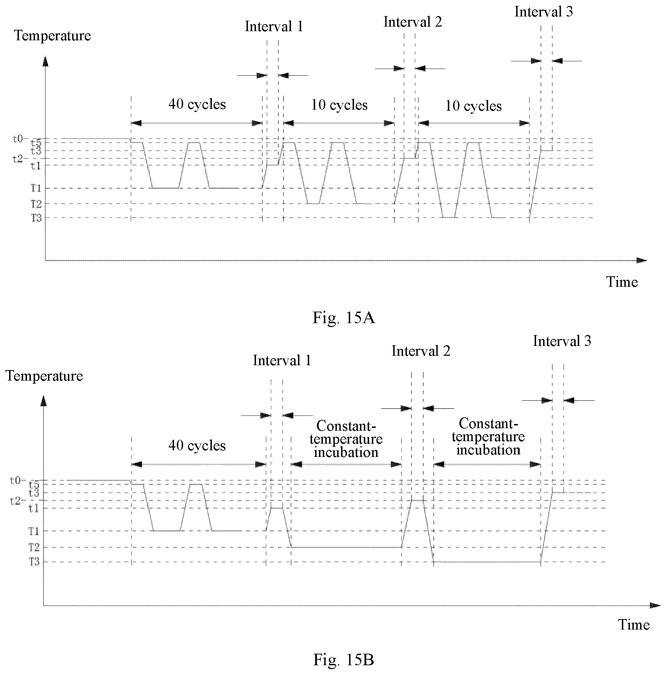

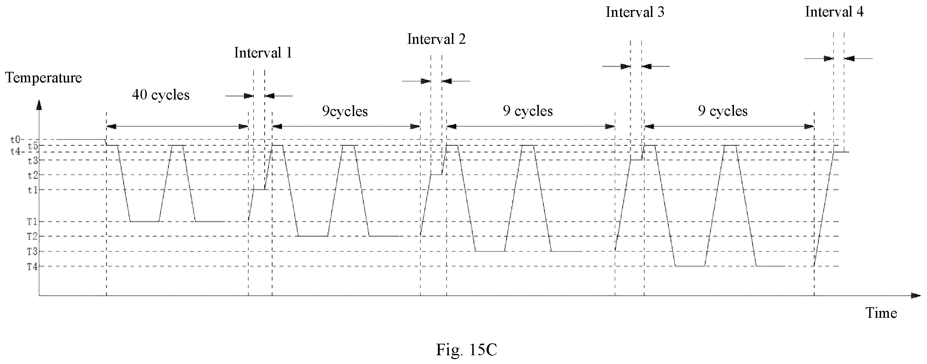

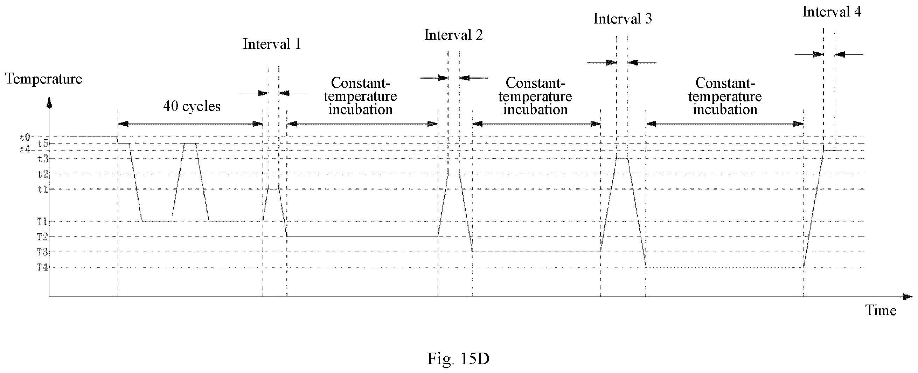

Fig. 15A is a temperature variation curve chart of a controller controlling a temperature adjustment part to adjust a temperature of a reaction liquid containing part disclosed by an embodiment of the present application; Fig. 15B is a temperature variation curve chart of the controller controlling the temperature adjustment part to adjust the temperature of the reaction liquid containing part disclosed by another embodiment of the present application; Fig. 15C is a temperature variation curve chart of the controller controlling the temperature adjustment part to adjust the temperature of the reaction liquid containing part disclosed by yet another embodiment of the present application; and Fig. 15D is a temperature variation curve chart of the controller controlling the temperature adjustment part to adjust the temperature of the reaction liquid containing part disclosed by still another embodiment of the present application.

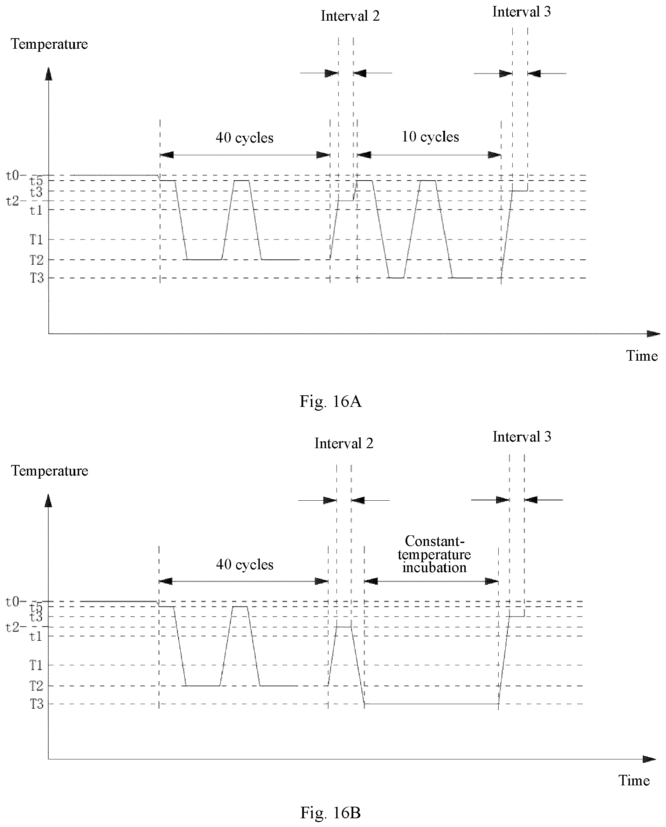

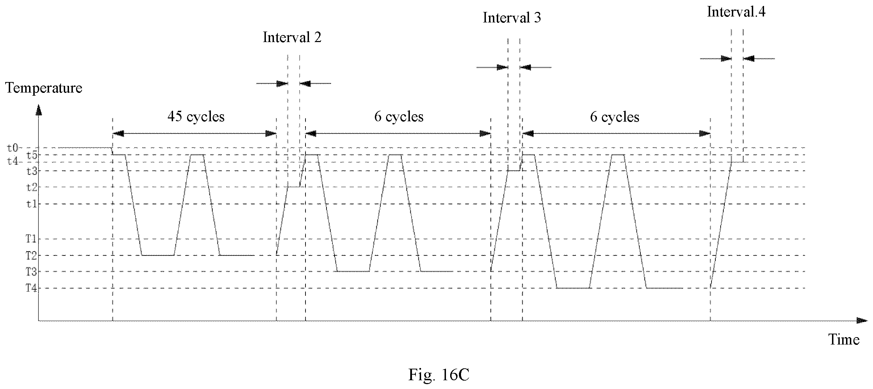

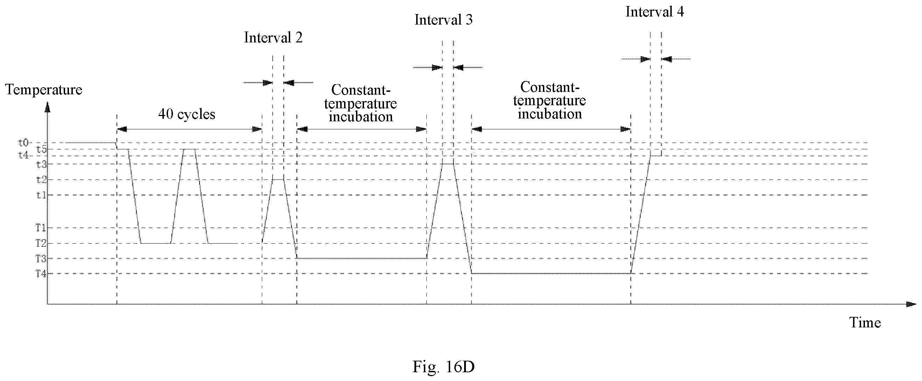

Fig. 16A is a temperature variation curve chart of the controller controlling the temperature adjustment part to adjust the temperature of the reaction liquid containing part disclosed by another embodiment of the present application; Fig. 16B is a temperature variation curve chart of the controller controlling the temperature adjustment part to adjust the temperature of the reaction liquid containing part disclosed by yet another embodiment of the present application; Fig. 16C is a temperature variation curve chart of the controller controlling the temperature adjustment part to adjust the temperature of the reaction liquid containing part disclosed by still another embodiment of the present application; and Fig. 16D is a temperature variation curve chart of the controller controlling the temperature adjustment part to adjust the temperature of the reaction liquid containing part disclosed by still yet another embodiment of the present application.

### Detailed Description of the Embodiments

In order to make the present invention easier to understand, the present invention will be described below in detail in conjunction with examples, which are illustrative only and are not limited to the scope of application of the present invention.

### First implementation

The present invention will be specifically described hereinafter in conjunction with specific embodiments and examples, and the advantages and various effects of the present invention will thus be more clearly presented. It should be understood by a person skilled in the art that these specific embodiments and examples are intended to illustrate the present invention and not to limit the present invention.

All of the following examples use a full-automatic digital PCR analysis system and reagent consumables for detection and data analysis (wherein, Examples 1-7 use a D600 full-automatic digital PCR analysis system and reagent consumables of Maccura Biotechnology Co., Ltd; Examples 8-12 use an OS-500 digital PCR all-in-one machine and reagent consumables of Beijing DAWEIBIO Co., Ltd). The digital PCR analysis system disperses a reaction system containing a sample, a primer-probe composition and an amplification reagent into four reaction wells for amplification and detection (each reaction well containing a plurality of small droplets, each of which being one reaction unit). To avoid repetition, the drawings of the following examples are each provided with a graphical result of the droplets in one of the reaction wells.

### Example 1

(1) A monoplex detection primer probe system for detecting non-small cell lung cancer EGFR Exon 21 L858R mutation is shown in Table 1:

**Table 1 base sequences of primers, probes in Example 1**

| Names of primers, probes | Serial numbers | Nucleotide sequences (5'-3') | Modification |
|---|---|---|---|
| P1 | SEQ ID NO: 1 | GTGCACACGGTACTGTCAGCTGAACT GGTCTCACTGCAGCGCTGTCCGAGCTC GACGTG | T14-FAM, T26-BHQ1 3'Spacer C3 |
| R1 | SEQ ID NO: 2 | TTTTTCTGTCAGCTGAACTGGTCTCAC AAACGCACCCAGCAGTTTGGCCC | / |
| F1 | SEQ ID NO: 3 | CCGTGTGCACTTTCGACCTGGCAGCCA GGAAC | / |

In the above primer probes,
a forward primer F1 (SEQ ID NO: 3), as a specific primer designed for a target sequence of non-small cell lung cancer EGFR Exon 21 L858R mutation, has an overall length of 32 bp, with first to 19th bases at a 3' end as a target sequence binding region, and first to tenth bases at a 5' end as a probe anchoring region (A1), which is reversely complementary with first to tenth base sequences at a 5' end of a probe P1 (SEQ ID NO: 1), i.e., a primer anchoring region (A1');
a reverse primer R1 (SEQ ID NO: 2), as a specific primer designed for a target sequence of non-small cell lung cancer EGFR Exon 21 L858R mutation, has an overall length of 50 bp, with first to 20th bases at a 3' end as a target sequence binding region, first to fifth bases at a 5' end as an extension block region (M), and sixth to 27th bases at the 5' end as a primer signal detection region (h3), which is the same as 13th to 34th base sequences at the 5' end of the probe P1 (SEQ ID NO: 1).

(2) The above primer probe system is used for PCR detection, and a reaction system used in detection is shown in Table 2.

**Table 2 detection reaction system in Example 1**

| Reagent composition | Concentration |
|---|---|
| 2 × PCR Reaction Buffer | 1 × |
| DNA Polymerase | 2U |
| Forward primer (F1) | 500 nM |
| Reverse primer (R1) | 100 nM |
| Probe (P1) | 400 nM |
| Nucleic acid template | 150 copies |
| Ultrapure water | Added to 20 µL |

| | |
|---|---|
| Note: in Table 2, 2X PCR Reaction Buffer includes 3 mM MgCl₂, 30 mM Tris-HCl with pH of 8.3, 0.5 mM dNTP, and 70 mM (NH₄)₂SO₄; and mM refers to mmol/L (the same below). | |

Sample preparation: non-small cell lung cancer EGFR Exon 21 L858R mutation DNA was used as a positive sample, and a non-small cell lung cancer EGFR Exon 21 L858R mutation target sequence was detected using the reverse primer (R1) with a signal detection region sequence to verify the detectability of a single mutation. Pure water was used as a no template control (NTC).

Reaction preparation:
after sample preparation was completed, the reaction system was configured according to the ratios in Table 2.

Digital PCR amplification and signal acquisition (the D600 full-automatic digital PCR analysis system and reagent consumables of Maccura Biotechnology Co., Ltd were used for detection):
after a digital PCR tube cap was sealed, the samples were gently mixed, and then briefly centrifuged and left for standing for 5 min at room temperature. A digital PCR tube was placed in a handheld centrifuge again, briefly centrifuged and transferred to a sample stand of a digital PCR instrument. Amplification and lighting procedures used were: initial denaturation at 95°C for 2 min; denaturation at 94°C for 10 s, and annealing and extending at 56°C for 30 s, for a total of 45 cycles; a first signal acquisition at 60°C; denaturation at 94°C for 15 s, and annealing and extending at 40°C for 20 s, for a total of 10 cycles; and a second signal acquisition at 78°C.

Data analysis (the D600 full-automatic digital PCR analysis system of Maccura Biotechnology Co., Ltd was used for performing data analysis):
a result was interpreted through positive droplets at different signal acquisition temperatures.

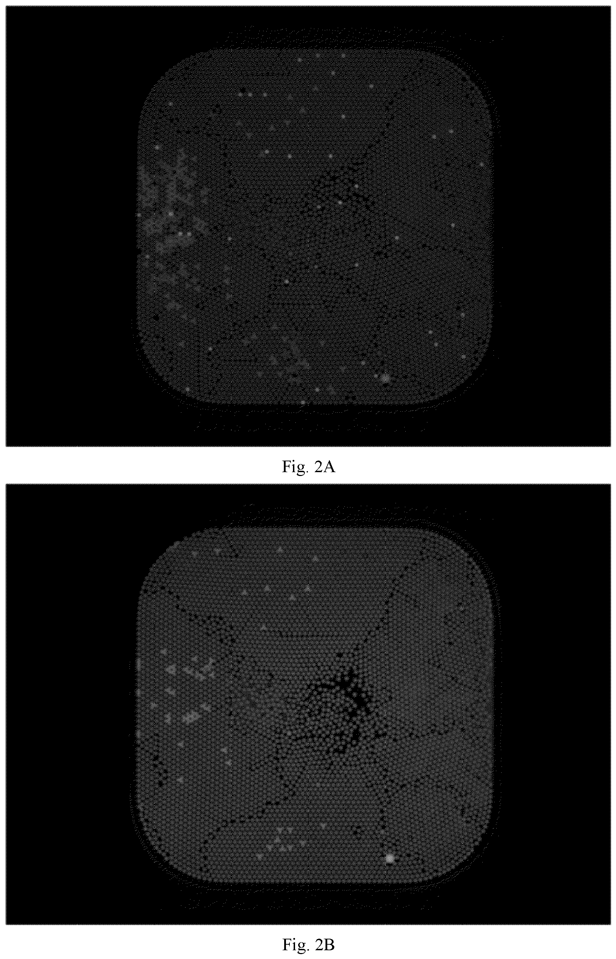

Fig. 2A is a droplet diagram of a monoplex-PCR non-small cell lung cancer EGFR Exon 21 L858R mutation positive sample amplified by the reverse primer (R1) at a first signal acquisition temperature (60°C), and positive droplets different from the background can be seen.

Fig. 2B is a droplet diagram of the monoplex-PCR non-small cell lung cancer EGFR Exon 21 L858R mutation positive sample amplified by the reverse primer (R1) at a second signal acquisition temperature (78°C), and it can be seen that all droplets are negative.

It can be seen from Figs. 2A and 2B that by adopting the detection method of the present invention, different detection signal results can be obtained at different signal acquisition temperatures, and a target signal is turned "on" and "off' to achieve more accurately detection of a target.

### Example 2

(1) A monoplex detection primer probe system for detecting non-small cell lung cancer EGFR Exon 21 L858R mutation is shown in Table 3:

**Table 3 sequences of primers, probes in Example 2**

| Names of primers, probes | Serial numbers | Nucleotide sequences (5'-3') | Modification |
|---|---|---|---|
| P1 | SEQ ID NO: 1 | GTGCACACGGTACTGTCAGCTGAACT GGTCTCACTGCAGCGCTGTCCGAGCTC GACGTG | T14-FAM, T26-BHQ1 3'Spacer C3 |
| R2 | SEQ ID NO: 4 | GCTCGACGTGAAACGCACCCAGCAGT TTGGCCC | / |
| F1 | SEQ ID NO: 3 | CCGTGTGCACTTTCGACCTGGCAGCCA GGAAC | / |

In the above primer probes,
a forward primer F1 (SEQ ID NO: 3), as a specific primer designed for a target sequence of non-small cell lung cancer EGFR Exon 21 L858R mutation, has an overall length of 32 bp, with first to 19th bases at a 3' end as a target sequence binding region, and first to tenth bases at a 5' end as a probe anchoring region (A1), which is reversely complementary with first to tenth base sequences at a 5' end of a probe P1 (SEQ ID NO: 1), i.e., a primer anchoring region (A1');
a reverse primer R2 (SEQ ID NO: 4), as a specific primer designed for a target sequence of non-small cell lung cancer EGFR Exon 21 L858R mutation, has an overall length of 33 bp, with first to 20th bases at a 3' end as a target sequence binding region, and first to tenth bases at a 5' end as a primer signal detection region (h2), which is the same as first to tenth base sequences at a 3' end of the probe P1 (SEQ ID NO: 1).

(2) The above primer probe system is used for PCR detection, and a reaction system used in detection is shown in Table 4.

**Table 4 detection reaction system in Example 2**

| Reagent composition | Concentration |
|---|---|
| 2 × PCR Reaction Buffer | 1 × |
| DNA Polymerase | 2U |
| Forward primer (F1) | 500 nM |
| Reverse primer (R2) | 100 nM |
| Probe (P1) | 400 nM |
| Nucleic acid template | 130 copies |
| Ultrapure water | Added to 20 µL |

| | |
|---|---|
| Note: in Table 4, 2X PCR Reaction Buffer includes 3 mM MgCl₂, 30 mM Tris-HCl with pH of 8.3, 0.5 mM dNTP, and 70 mM (NH₄)₂SO₄. | |

Sample preparation: non-small cell lung cancer EGFR Exon 21 L858R mutation DNA was used as a positive sample, and a non-small cell lung cancer EGFR Exon 21 L858R mutation target sequence was detected using the reverse primer (R2) with a signal detection region sequence to verify the detectability of a single mutation. Pure water was used as a no template control (NTC).

Reaction preparation:
after sample preparation was completed, the reaction system was configured according to the ratios in Table 4.

Digital PCR amplification and signal acquisition (the D600 full-automatic digital PCR analysis system and reagent consumables of Maccura Biotechnology Co., Ltd was used for detection):
after a digital PCR tube cap was sealed, the samples were gently mixed, and then briefly centrifuged and left for standing for 5 min at room temperature. A digital PCR tube was placed in a handheld centrifuge again, briefly centrifuged and transferred to a sample stand of a digital PCR instrument. Amplification and lighting procedures used were: initial denaturation at 95°C for 2 min; denaturation at 94°C for 10 s, and annealing and extending at 56°C for 30 s, for a total of 45 cycles; a first signal acquisition at 60°C; denaturation at 94°C for 15 s, and annealing and extending at 40°C for 20 s, for a total of 10 cycles; and a second signal acquisition at 78°C.

Data analysis (the D600 full-automatic digital PCR analysis system of Maccura Biotechnology Co., Ltd was used for performing data analysis):
a result was interpreted through positive droplets at different signal acquisition temperatures.

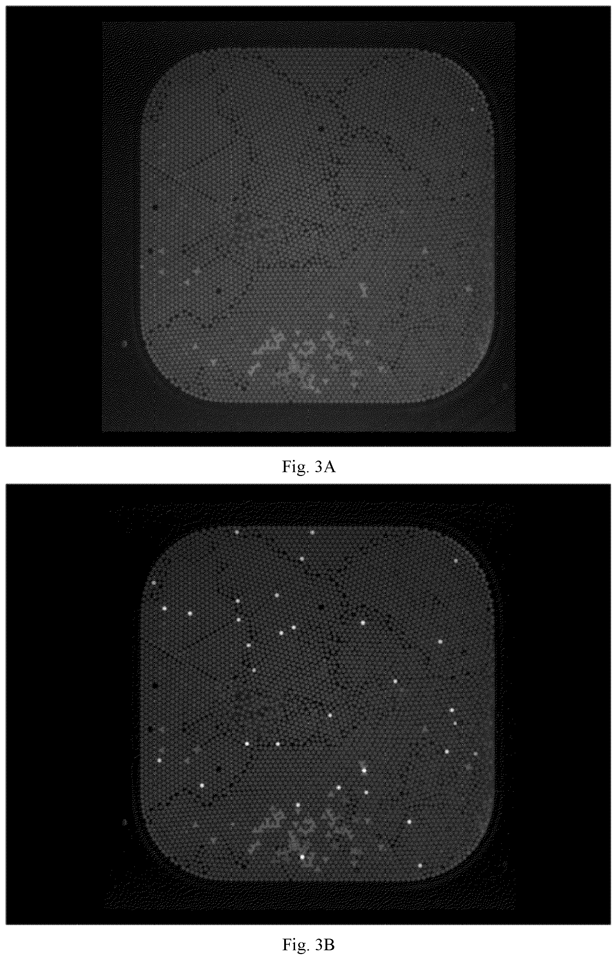

Fig. 3A is a droplet diagram of a monoplex-PCR non-small cell lung cancer EGFR Exon 21 L858R mutation positive sample amplified by the reverse primer (R2) at a first signal acquisition temperature (60°C), and it can be seen that all droplets are negative.

Fig. 3B is a droplet diagram of the monoplex-PCR non-small cell lung cancer EGFR Exon 21 L858R mutation positive sample amplified by the reverse primer (R2) at a second signal acquisition temperature (78°C), and positive droplets different from the background can be seen.

It can be seen from Figs. 3A and 3B that by adopting the primer-probe composition and digital PCR detection method of the present invention, different detection signal results can be obtained at different signal acquisition temperatures, and a target signal is turned "on" and "off' to achieve more accurately detection of a target.

### Example 3

(1) A monoplex detection primer probe system for detecting non-small cell lung cancer EGFR Exon 21 L858R mutation is shown in Table 5:

**Table 5 base sequences of primers, probes in Example 3**

| Names of primers, probes | Serial numbers | Nucleotide sequences (5'-3') | Modification |
|---|---|---|---|
| P1 | SEQ ID NO: 1 | GTGCACACGGTACTGTCAGCTGAAC TGGTCTCACTGCAGCGCTGTCCGAG CTCGACGTG | T14-FAM, T26-BHQ1 3'Spacer C3 |
| R2 | SEQ ID NO: 4 | GCTCGACGTGAAACGCACCCAGCAG TTTGGCCC | / |
| F1 | SEQ ID NO: 3 | CCGTGTGCACTTTCGACCTGGCAGC CAGGAAC | / |

In the above primer probes,
a forward primer F1 (SEQ ID NO: 3), as a specific primer designed for a target sequence of non-small cell lung cancer EGFR Exon 21 L858R mutation, has an overall length of 32 bp, with first to 19th bases at a 3' end as a target sequence binding region, and first to tenth bases at a 5' end as a probe anchoring region (A1), which is reversely complementary with first to tenth base sequences at a 5' end of a probe P1 (SEQ ID NO: 1), i.e., a primer anchoring region (A1');
a reverse primer R2 (SEQ ID NO: 4), as a specific primer designed for a target sequence of non-small cell lung cancer EGFR Exon 21 L858R mutation, has an overall length of 33 bp, with first to 20th bases at a 3' end as a target sequence binding region, and first to tenth bases at a 5' end as a primer signal detection region (h2), which is the same as first to tenth base sequences at a 3' end of the probe P1 (SEQ ID NO: 1).

(2) The above primer probe system is used for PCR detection, and a reaction system used in detection is shown in Table 6.

**Table 6 detection reaction system in Example 3**

| Reagent composition | Concentration |
|---|---|
| 2 × PCR Reaction Buffer | 1 × |
| DNA Polymerase | 2U |
| Forward primer (F1) | 500 nM |
| Reverse primer (R2) | 100 nM |
| Probe (P1) | 400 nM |
| Nucleic acid template | 160 copies |
| Ultrapure water | Added to 20 µL |

| | |
|---|---|
| Note: in Table 6, 2X PCR Reaction Buffer includes 3 mM MgCl₂, 30 mM Tris-HCl with pH of 8.3, 0.5 mM dNTP, and 70 mM (NH₄)₂SO₄. | |

Sample preparation: non-small cell lung cancer EGFR Exon 21 L858R mutation DNA was used as a positive sample, and a non-small cell lung cancer EGFR Exon 21 L858R mutation target sequence was detected using the reverse primer (R2) with a signal detection region sequence to verify the detectability of a single mutation. Pure water was used as a no template control (NTC).

Reaction preparation:
after sample preparation was completed, the reaction system was configured according to the ratios in Table 6.

Digital PCR amplification and signal acquisition (the D600 full-automatic digital PCR analysis system and reagent consumables of Maccura Biotechnology Co., Ltd were used for detection):
after a digital PCR tube cap was sealed, the samples were gently mixed, and then briefly centrifuged and left for standing for 5 min at room temperature. A digital PCR tube was placed in a handheld centrifuge again, briefly centrifuged and transferred to a sample stand of a digital PCR instrument. Amplification and lighting procedures used were: initial denaturation at 95°C for 2 min; denaturation at 94°C for 10 s, and annealing and extending at 56°C for 30 s, for a total of 45 cycles; a first signal acquisition at 60°C; constant-temperature incubation at 40°C for 5 min; and a second signal acquisition at 78°C.

Data analysis (the D600 full-automatic digital PCR analysis system of Maccura Biotechnology Co., Ltd was used for performing data analysis):
a result was interpreted through positive droplets at different signal acquisition temperatures.

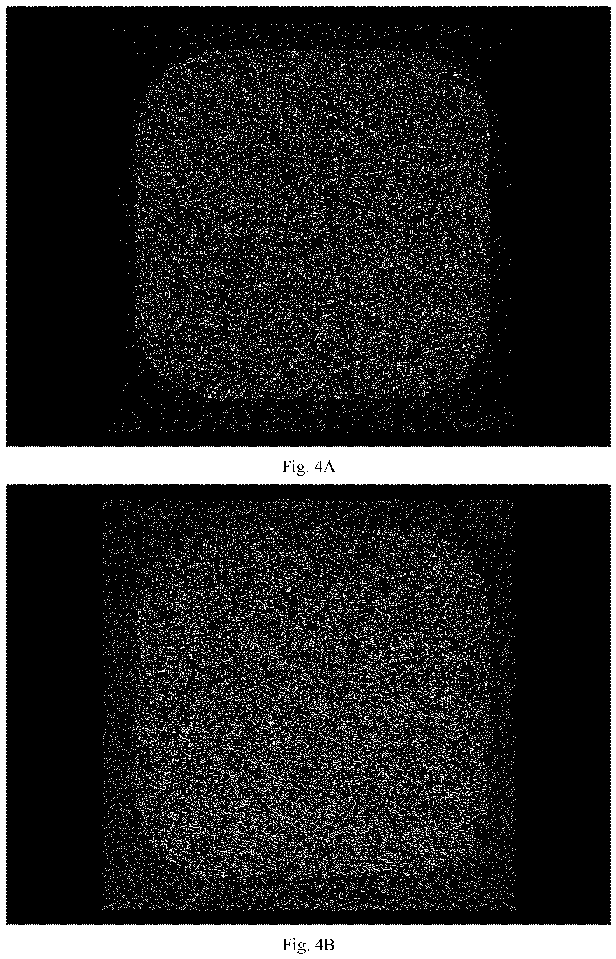

Fig. 4A is a droplet diagram of a monoplex-PCR non-small cell lung cancer EGFR Exon 21 L858R mutation positive sample amplified by the reverse primer (R2) at a first signal acquisition temperature (60°C), and it can be seen that all droplets are negative (droplets that are at edges and/or appear large are caused by an experimental operation problem and are not positive droplets).

Fig. 4B is a droplet diagram of the monoplex-PCR non-small cell lung cancer EGFR Exon 21 L858R mutation positive sample amplified by the reverse primer (R2) at a second signal acquisition temperature (78°C), and positive droplets different from the background can be seen.

It can be seen from Figs. 4A and 4B that by adopting the primer-probe composition and digital PCR detection method of the present invention, different detection signal results can be obtained at different signal acquisition temperatures (60°C and 78°C), and a target signal is turned "on" and "off" to achieve more accurately detection of a target.

### Example 4

(1) A duplex detection primer probe system for detecting carbapenem antibiotics resistant VIM gene mutation and carbapenem antibiotics resistant OXA-48 gene mutation is shown in Table 7:

**Table 7 base sequences of primers, probes in Example 4**

| Names of primers, probes | Serial numbers | Nucleotide sequences (5'-3') | Modification |
|---|---|---|---|
| P2-4 | SEQ ID NO: 5 | GGCTTCGAGCACTTGCACTGATCCGAT CGACGTGCGACCATGACGGAGAGCAG GACGAG | T14-ROX, T27-BHQ2 3'Spacer C3 |
| F1-4 | SEQ ID NO: 6 | TGCTCGAAGCCAAAAGCAAATTGGACT TCCCGTAACG | / |
| R1-4 | SEQ ID NO: 7 | TTTTTTGCACTGATCCGATCGACGTGCT TTCCTTCTAGAGAATGCGTGGGAATCT | / |
| F2-4 | SEQ ID NO: 8 | TGCTCGAAGCCAAAATTGGCGAGGCAC GTATGA | / |
| R2-4 | SEQ ID NO: 9 | GCAGGACGAGTTTCACCGTCGAGCCA GAAACTGTCTA | / |

In the above primer probes,
a forward primer F1-4 (SEQ ID NO: 6), as a specific primer designed for a target sequence of carbapenem antibiotics resistant VIM gene mutation, has an overall length of 37 bp, with first to 23rd bases at a 3' end as a target sequence binding region, and first to 11th bases at a 5' end as a probe anchoring region (A), which is reversely complementary with first to 11th base sequences at a 5' end of a probe P2-4 (SEQ ID NO: 5), i.e., a primer anchoring region (A');
a reverse primer R1-4 (SEQ ID NO: 7), as a specific primer designed for a target sequence of carbapenem antibiotics resistant VIM gene mutation, has an overall length of 55 bp, with first to 25th bases at a 3' end as a target sequence binding region, first to fifth bases at a 5' end as a signal block region (M), and sixth to 27th bases at the 5' end as a primer signal detection region (h1), which is the same as 14th to 35th base sequences at the 5' end of the probe P2-4 (SEQ ID NO: 5);
a forward primer F2-4 (SEQ ID NO: 8), as a specific primer designed for a target sequence of carbapenem antibiotics resistant OXA-48 gene mutation, has an overall length of 33 bp, with first to 19th bases at a 3' end as a target sequence binding region, and first to 11th bases at a 5' end as a probe anchoring region (A), which is reversely complementary with the first to 11th base sequences at the 5' end of the probe P2-4 (SEQ ID NO: 5), i.e., the primer anchoring region (A');
a reverse primer R2-4 (SEQ ID NO: 9), as a specific primer designed for a target sequence of carbapenem antibiotics resistant OXA-48 gene mutation, has an overall length of 37 bp, with first to 24th bases at a 3' end as a target sequence binding region, and first to 10th bases at a 5' end being the same as first to 10th base sequences a 3' end of the probe P2-4 (SEQ ID NO: 5).

(2) The above primer probe system is used for PCR detection, and a reaction system used in detection is shown in Table 8.

**Table 8 detection reaction system in Example 4**

| Reagent composition | Concentration |
|---|---|
| 2 × PCR Reaction Buffer | 1 × |
| DNA Polymerase | 2U |
| Forward primer (F1-4) | 500 nM |
| Reverse primer (R1-4) | 100 nM |
| Forward primer (F2-4) | 500 nM |
| Reverse primer (R2-4) | 100 nM |
| Probe (P2-4) | 400 nM |
| Carbapenem antibiotics resistant VIM gene mutation nucleic acid template | 130 copies |
| Carbapenem antibiotics resistant OXA-48 gene mutation nucleic acid template | 10 copies |
| Ultrapure water | Added to 20 µL |

| | |
|---|---|
| Note: in Table 8, 2X PCR Reaction Buffer includes 3 mM MgCl₂, 30 mM Tris-HCl with pH of 8.3, 0.5 mM dNTP, and 70 mM (NH₄)₂SO₄. | |

Sample preparation: a nucleic acid for carbapenem antibiotics resistant VIM gene mutation and a nucleic acid for carbapenem antibiotics resistant OXA-48 gene mutation were used as positive samples, and target sequences of carbapenem antibiotics resistant VIM gene mutation and carbapenem antibiotics resistant OXA-48 gene mutation were detected using the reverse primers (R1-4 and R2-4) with different signal detection region sequences to verify the detectability of dual mutations. Pure water was used as a no template control (NTC).

Reaction preparation:
after sample preparation was completed, the reaction system was configured according to the ratios in Table 8.

Digital PCR amplification and signal acquisition (the D600 full-automatic digital PCR analysis system and reagent consumables of Maccura Biotechnology Co., Ltd were used for detection):
after a digital PCR tube cap was sealed, the samples were gently mixed, and then briefly centrifuged and left for standing for 5 min at room temperature. A digital PCR tube was placed in a handheld centrifuge again, briefly centrifuged and transferred to a sample stand of a digital PCR instrument. Amplification and lighting procedures used were: initial denaturation at 95°C for 2 min; denaturation at 94°C for 10 s, and annealing and extending at 56°C for 30 s, for a total of 45 cycles; a first signal acquisition at 56°C; denaturation at 94°C for 15 s, and annealing and extending at 40°C for 20 s, for a total of 10 cycles; and a second signal acquisition at 72°C.

Data analysis (the D600 full-automatic digital PCR analysis system of Maccura Biotechnology Co., Ltd was used for performing data analysis):
a result was interpreted through positive droplets at different signal acquisition temperatures.

Fig. 5i is a droplet diagram of the Example at a first signal acquisition temperature (56°C), and positive droplets different from the background can be seen and are carbapenem antibiotics resistant VIM gene mutation positive droplets (droplets that are at edges and/or appear larger are caused by an experimental operation problem and are not positive droplets).

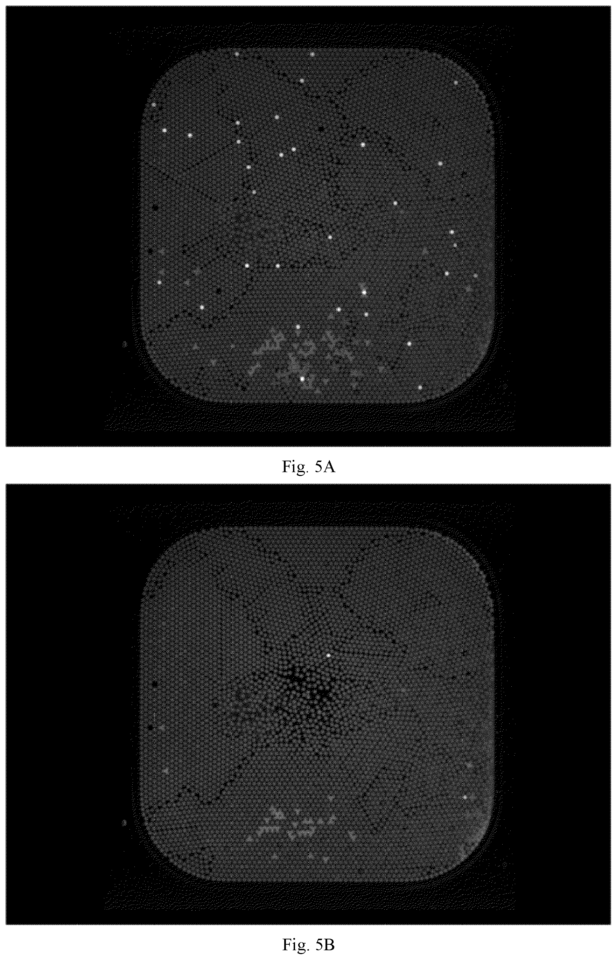

Fig. 5B is a droplet diagram of the Example at a second signal acquisition temperature (72°C), positive droplets different from the background can be seen and are carbapenem antibiotics resistant OXA-48 gene mutation positive droplets, and positions of the positive droplets presented at that temperature are different from those of the positive droplets presented at the first signal acquisition temperature (56°C).

It can be seen from Figs. 5A and Fig. 5B that by adopting the primer-probe composition and digital PCR detection method of the present invention, detection signals of different target sequences are realized to be obtained at different signal detection temperatures (56°C and 72°C) respectively, without crossing each other.

### Example 5

(1) A triple detection primer probe system for detecting carbapenem antibiotics resistant IMP gene mutation, carbapenem antibiotics resistant OXA-48 gene mutation and carbapenem antibiotics resistant KPC gene mutation is shown in Table 9:

**Table 9 base sequences of primers, probes in Example 5**

| Names of primers, probes | Serial numbers | Nucleotide sequences (5'-3') | Modification |
|---|---|---|---|
| P1-5 | SEQ ID NO: 10 | CCTCACGCTCATGCCATATATCACCGTA CGTCATCATGCCGAACCCGATCTGCTC ACCA | T21-CY5, T34-BHQ2 3' Spacer C3 |
| F1-5 | SEQ ID NO: 11 | GGCATGAGCGTGAGGTTTTAGTAGTTT GGCTGCCTGAAAGGA | / |
| R1-5 | SEQ ID NO: 12 | TTTTTTATATCACCGTACGTCATCATGCC GAAAGTCACCCAAATTACCTAGACCGT | / |
| F2-5 | SEQ ID NO: 13 | GGCATGAGCGTGAGGTTTATTGGCGAG GCACGTATGA | / |
| R2-5 | SEQ ID NO: 14 | CATGCCGAACCAAACACCGTCGAGCCA GAAACTGTCTA | / |
| F3-5 | SEQ ID NO: 15 | GGCATGAGCGTGAGGTTTAAACTGACA CTGGGCTCTGCA | / |
| R3-5 | SEQ ID NO: 16 | GCTCACCAAAAGTCATTTGCCGTGCCA TACACT | / |

In the above primer probes,
a forward primer F1-5 (SEQ ID NO: 11), as a specific primer designed for a target sequence of carbapenem antibiotics resistant IMP gene mutation, has an overall length of 42 bp, with first to 24th bases at a 3' end as a target sequence binding region, and first to 15th bases at a 5' end as a probe anchoring region (A), which is reversely complementary with first to 15th base sequences at a 5' end of a probe P1-5 (SEQ ID NO: 10), i.e., a primer anchoring region (A');
a reverse primer R1-5 (SEQ ID NO: 12), as a specific primer designed for a target sequence of carbapenem antibiotics resistant IMP gene mutation, has an overall length of 56 bp, with first to 23rd bases at a 3' end as a target sequence binding region, first to fifth bases at a 5' end as an signal block region (M), and sixth to 30th bases at the 5' end as a primer signal detection region (h1), which is the same as 17th to 41st base sequences at the 5' end of the probe P1-5 (SEQ ID NO: 10);
a forward primer F2-5 (SEQ ID NO: 13), as a specific primer designed for a target sequence of carbapenem antibiotics resistant OXA-48 gene mutation, has an overall length of 37 bp, with first to 19th bases at a 3' end as a target sequence binding region, and first to 15th bases at a 5' end as a probe anchoring region (A), which is reversely complementary with first to 15th base sequences at the 5' end of the probe P1-5 (SEQ ID NO: 10), i.e., the primer anchoring region (A');
a reverse primer R2-5 (SEQ ID NO: 14), as a specific primer designed for a target sequence of carbapenem antibiotics resistant OXA-48 gene mutation, has an overall length of 38 bp, with first to 24th bases at a 3' end as a target sequence binding region, and first to 11th bases at a 5' end being the same as 15th to 25th base sequences a 3' end of the probe P1-5 (SEQ ID NO: 10).

A forward primer F3-5 (SEQ ID NO: 15), as a specific primer designed for a target sequence of carbapenem antibiotics resistant KPC gene mutation, has an overall length of 39 bp, with first to 21st bases at a 3' end as a target sequence binding region, and first to 15th bases at a 5' end as a probe anchoring region (A), which is reversely complementary with first to 15th base sequences at the 5' end of the probe P1-5 (SEQ ID NO: 10), i.e., the primer anchoring region (A');
a reverse primer R3-5 (SEQ ID NO: 16), as a specific primer designed for a target sequence of carbapenem antibiotics resistant KPC gene mutation, has an overall length of 33 bp, with first to 22th bases at a 3' end as a target sequence binding region, and first to eighth bases at a 5' end being the same as first to eighth base sequences a 3' end of the probe P1-5 (SEQ ID NO: 10).

(2) The above primer probe system is used for PCR detection, and a reaction system used in detection is shown in Table 10.

**Table 10 detection reaction system in Example 5**

| Reagent composition | Concentration |
|---|---|
| 2 × PCR Reaction Buffer | 1 × |
| DNA Polymerase | 2U |
| Forward primer (F1-5) | 250 nM |
| Reverse primer (R1-5) | 100 nM |
| Forward primer (F2-5) | 250 nM |
| Reverse primer (R2-5) | 100 nM |
| Forward primer (F3-5) | 250 nM |
| Reverse primer (R3-5) | 100 nM |
| Probe (P1-5) | 400 nM |
| Carbapenem antibiotics resistant IMP gene mutation nucleic acid template | 30 copies |
| Carbapenem antibiotics resistant OXA-48 gene mutation nucleic acid template | 120 copies |
| Carbapenem antibiotics resistant KPC gene mutation nucleic acid template | 6 copies |
| Ultrapure water | Added to 20 µL |

| | |
|---|---|
| Note: in Table 10, 2X PCR Reaction Buffer includes 3 mM MgCl₂, 30 mM Tris-HCl with pH of 8.3, 0.5 mM dNTP, and 70 mM (NH₄)₂SO₄. | |

Sample preparation: a nucleic acid sample for carbapenem antibiotics resistant IMP gene mutation, a nucleic acid sample for carbapenem antibiotics resistant OXA-48 gene mutation, and a nucleic acid sample for carbapenem antibiotics resistant KPC gene mutation were used as positive samples, and target sequences of carbapenem antibiotics resistant IMP gene mutation, carbapenem antibiotics resistant OXA-48 gene mutation and carbapenem antibiotics resistant KPC gene mutation were detected using the reverse primers (R1-5/R2-5/R3-5) with different signal detection region sequences to verify the detectability of triple mutations. Pure water was used as a no template control (NTC).

Reaction preparation:
after sample preparation was completed, the reaction system was configured according to the ratios in Table 10.

Digital PCR amplification and signal acquisition (the D600 full-automatic digital PCR analysis system and reagent consumables of Maccura Biotechnology Co., Ltd were used for detection):
after a digital PCR tube cap was sealed, the samples were gently mixed, and then briefly centrifuged and left for standing for 5 min at room temperature. A digital PCR tube was placed in a handheld centrifuge again, briefly centrifuged and transferred to a sample stand of a digital PCR instrument. Amplification and lighting procedures used were: initial denaturation at 95°C for 2 min; denaturation at 94°C for 10 s, and annealing and extending at 56°C for 30 s, for a total of 45 cycles; a first signal acquisition at 55°C; denaturation at 94°C for 15 s, and annealing and extending at 52°C for 20 s, for a total of 5 cycles; and a second signal acquisition at 65°C. Denaturation at 94°C for 15 s, and annealing and extending at 40°C for 20 s, for a total of 5 cycles; and a third signal acquisition at 73°C.

Data analysis (the D600 full-automatic digital PCR analysis system of Maccura Biotechnology Co., Ltd was used for performing data analysis):
a result was interpreted through positive droplets at different signal acquisition temperatures.

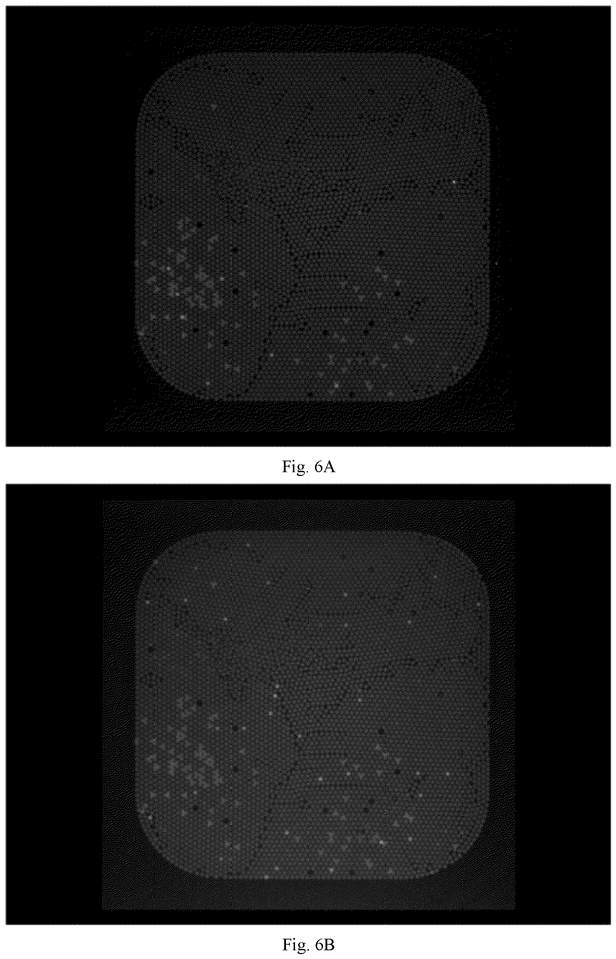

Fig. 6A is a droplet diagram of positive samples of carbapenem antibiotics resistant IMP gene mutation, carbapenem antibiotics resistant OXA-48 gene mutation and carbapenem antibiotics resistant KPC gene mutation in the Example at a first signal acquisition temperature (55°C), and positive droplets different from the background can be seen and are carbapenem antibiotics resistant IMP gene mutation positive droplets.

Fig. 6B is a droplet diagram of positive samples of carbapenem antibiotics resistant IMP gene mutation, carbapenem antibiotics resistant OXA-48 gene mutation and carbapenem antibiotics resistant KPC gene mutation in the Example at a second signal acquisition temperature (65°C), positive droplets different from the background can be seen and are carbapenem antibiotics resistant OXA-48 gene mutation positive droplets, and positions of the positive droplets presented at that temperature are different from those of the positive droplets presented at the first signal acquisition temperature (55°C).

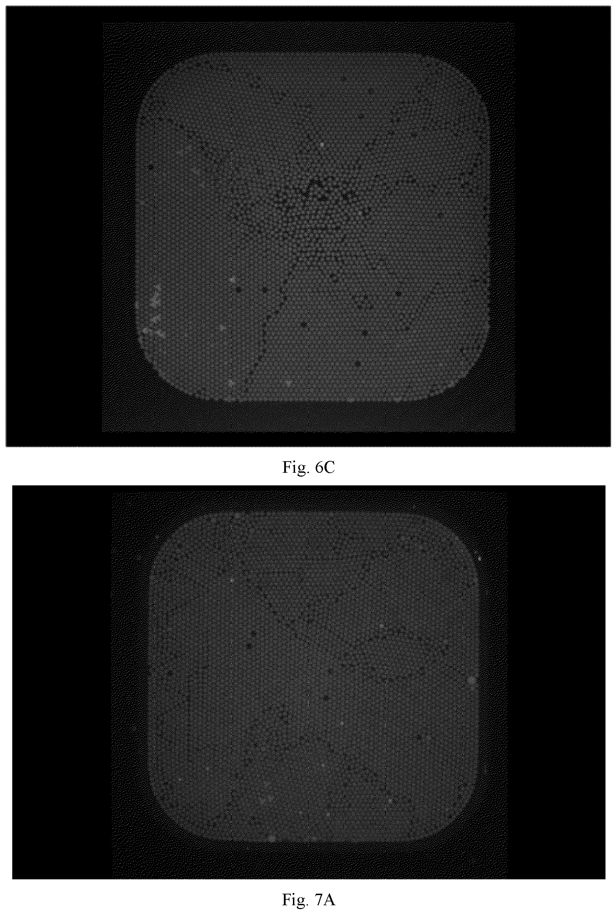

Fig. 6C is a droplet diagram of positive samples of carbapenem antibiotics resistant IMP gene mutation, carbapenem antibiotics resistant OXA-48 gene mutation and carbapenem antibiotics resistant KPC gene mutation in the Example at a third signal acquisition temperature (73°C), positive droplets are carbapenem antibiotics resistant KPC gene mutation positive droplets, and the positive droplets presented at that temperature are different from those of the positive droplets presented at the first signal acquisition temperature (55°C) and the positive droplets presented at the second signal acquisition temperature (65°C).

It can be seen from Figs. 6A-6C that by adopting the primer-probe composition and digital PCR detection method of the present invention, detection signals of different target sequences are realized to be obtained at different signal detection temperatures (55°C, 65°C, and 73°C) respectively, without crossing each other.

### Example 6

(1) A monoplex detection primer probe system for detecting non-small cell lung cancer EGFR Exon 21 L858R mutation and non-small cell lung cancer EGFR Exon 21 L858R mutation is shown in Table 11:

**Table 11 base sequences of primers, probes in Example 6**

| Names of primers, probes | Serial numbers | Nucleotide sequences (5'-3') | Modification |
|---|---|---|---|
| P1-6 | SEQ ID NO: 5 | GGCTTCGAGCACTTGCACTGATCCGAT CGACGTGCGACCATGACGGAGAGCAG GACGAG | T14-VIC, T26-BHQ1 3' Spacer C3 |
| R2-6 | SEQ ID NO: 17 | GCAGGACGAGAAACGCACCCAGCAGT TTGGCCC | / |
| F2-6 | SEQ ID NO: 18 | GCTCGAAGCCTTTCGACCTGGCAGCCA GGAAC | / |

In the above primer probes,
a forward primer F2-6 (SEQ ID NO: 18), as a specific primer designed for a target sequence of non-small cell lung cancer EGFR Exon 21 L858R mutation, has an overall length of 32 bp, with first to 19th bases at a 3' end as a target sequence binding region, and first to tenth bases at a 5' end as a probe anchoring region (A2), which is reversely complementary with first to tenth base sequences at a 5' end of a probe P1-6 (SEQ ID NO: 5), i.e., a primer anchoring region (A2');
a reverse primer R2-6 (SEQ ID NO: 17), as a specific primer designed for a target sequence of non-small cell lung cancer EGFR Exon 21 L858R mutation, has an overall length of 33 bp, with first to 20th bases at a 3' end as a target sequence binding region, and first to tenth bases at a 5' end as a primer signal detection region (h2), which is the same as first to tenth base sequences at a 3' end of the probe P1-6 (SEQ ID NO: 5).

(2) The above primer probe system is used for PCR detection, and a reaction system used in detection is shown in Table 12.

**Table 12 detection reaction system in Example 6**

| Reagent composition | Concentration |
|---|---|
| 2 × PCR Reaction Buffer | 1 × |
| DNA Polymerase | 2U |
| Forward primer (F2-6) | 500 nM |
| Reverse primer (R2-6) | 100 nM |
| Probe (P1-6) | 400 nM |
| Nucleic acid template | 30 copies |
| Ultrapure water | Added to 20 µL |

| | |
|---|---|
| Note: in Table 12, 2X PCR Reaction Buffer includes 3 mM MgCl₂, 30 mM Tris-HCl with pH of 8.3, 0.5 mM dNTP, and 70 mM (NH₄)₂SO₄. | |

Sample preparation: non-small cell lung cancer EGFR Exon 21 L858R mutation was used as a positive sample, and pure water was used as a no template control (NTC).

Reaction preparation:
after sample preparation was completed, the reaction system was configured according to the ratios in Table 12.

Digital PCR amplification and signal acquisition (the D600 full-automatic digital PCR analysis system and reagent consumables of Maccura Biotechnology Co., Ltd were used for detection):
after a digital PCR tube cap was sealed, the samples were gently mixed, and then briefly centrifuged and left for standing for 5 min at room temperature. A digital PCR tube was placed in a handheld centrifuge again, briefly centrifuged and transferred to a sample stand of a digital PCR instrument. Amplification and lighting procedures used were: initial denaturation at 95°C for 2 min; denaturation at 94°C for 10 s, and annealing and extending at 56°C for 30 s, for a total of 45 cycles; denaturation at 94°C for 15 s, and annealing and extending at 40°C for 20 s, for a total of 10 cycles; a first signal acquisition at 72°C; and a second signal acquisition at 82°C.

Data analysis (the D600 full-automatic digital PCR analysis system of Maccura Biotechnology Co., Ltd was used for performing data analysis):
a result was interpreted through signals acquired at different signal acquisition temperatures.

Fig. 7A is a droplet diagram at a first signal acquisition temperature (72°C), and positive droplets different from the background can be seen.

Fig. 7B is a droplet diagram at a second signal acquisition temperature (82°C), and it can be seen that all droplets are negative.

It can be seen from Figs. 7A and 7B that by adopting the detection method of the present invention, different detection signal results may be obtained at different signal acquisition temperatures (72°C and 82°C), and a target signal is turned "on" and "off' to achieve more accurately detection of a target.

### Example 7

(1) A triple detection primer probe system for detecting carbapenem antibiotics resistant KPC gene mutation, carbapenem antibiotics resistant IMP gene mutation and carbapenem antibiotics resistant VIM gene mutation is shown in Table 13:

**Table 13 base sequences of primers, probes in Example 7**

| Names of primers, probes | Serial numbers | Nucleotide sequences (5'-3') | Modification |
|---|---|---|---|
| P1-7 | SEQ ID NO: 19 | AGACGCACGGTGACATGTGTTTGACTC AGTTCTGCTGCTGCCCACCGTCTGGAG TGCGT | T16-VIC, T31-BHQ1 3' Spacer C3 |
| P2-7 | SEQ ID NO: 1 | GTGCACACGGTACTGTCAGCTGAACTG GTCTCACTGCAGCGCTGTCCGAGCTCG ACGTG | T14-FAM, T26-BHQ1 3' Spacer C3 |
| F1-7 | SEQ ID NO: 20 | CACCGTGCGTCTTTTAAACTGACACTG GGCTCTGCA | / |
| R1-7 | SEQ ID NO: 21 | TTTTTCATGTGTTTGACTCAGTTCTGCA AAGTCATTTGCCGTGCCATACACT | / |
| F2-7 | SEQ ID NO: 22 | CACCGTGCGTCTTTTTAGTAGTTTGGCT GCCTGAAAGGA | / |
| R2-7 | SEQ ID NO: 23 | CTGGAGTGCGTAAAGTCACCCAAATTA CCTAGACCGT | / |
| F3-7 | SEQ ID NO: 24 | CCGTGTGCACTTTAGCAAATTGGACTT CCCGTAACG | |
| R3-7 | SEQ ID NO: 19 | GCTCGACGTGAAACCTTCTAGAGAATG CGTGGGAATCT | |

In the above primer probes,
a forward primer F1-7 (SEQ ID NO: 20), as a specific primer designed for a target sequence of carbapenem antibiotics resistant KPC gene mutation, has an overall length of 36 bp, with first to 21st bases at a 3' end as a target sequence binding region, and first to 12th bases at a 5' end as a probe anchoring region (A), which is reversely complementary with first to 12th base sequences at a 5' end of a probe P1-7 (SEQ ID NO: 19), i.e., a primer anchoring region (A');
a reverse primer R1-7 (SEQ ID: 21), as a specific primer designed for a target sequence of carbapenem antibiotics resistant KPC gene mutation, has an overall length of 52 bp, with first to 22nd bases at a 3' end as a target sequence binding region, first to fifth bases at a 5' end as an signal block region (M), and sixth to 27th bases at the 5' end as a primer signal detection region (h1), which is the same as 14th to 35th base sequences at the 5' end of the probe P1-7 (SEQ ID NO: 19);
a forward primer F2-7 (SEQ ID NO: 22), as a specific primer designed for a target sequence of carbapenem antibiotics resistant IMP gene mutation, has an overall length of 39 bp, with first to 24th bases at a 3' end as a target sequence binding region, and first to 12th bases at a 5' end as a probe anchoring region (A), which is reversely complementary with first to 12th base sequences at the 5' end of the probe P1-7 (SEQ ID NO: 19), i.e., the primer anchoring region (A');
a reverse primer R2-7 (SEQ ID NO: 23), as a specific primer designed for a target sequence of carbapenem antibiotics resistant IMP gene mutation, has an overall length of 37 bp, with first to 23rd bases at a 3' end as a target sequence binding region, and first to 11th bases at a 5' end being the same as first to 11th base sequences a 3' end of the probe P1-7 (SEQ ID NO: 19).

A forward primer F3-7 (SEQ ID NO: 24), as a specific primer designed for a target sequence of carbapenem antibiotics resistant VIM gene mutation, has an overall length of 36 bp, with first to 23rd bases at a 3' end as a target sequence binding region, and first to tenth bases at a 5' end as a probe anchoring region (A), which is reversely complementary with first to tenth base sequences at a 5' end of a probe P2-7 (SEQ ID NO: 1), i.e., a primer anchoring region (A');
a reverse primer R3-7 (SEQ ID NO: 19), as a specific primer designed for a target sequence of carbapenem antibiotics resistant VIM gene mutation, had an overall length of 38 bp, with first to 25th bases at a 3' end as a target sequence binding region, and first to tenth bases at a 5' end being the same as first to tenth base sequences a 3' end of the probe P2-7 (SEQ ID NO: 1).

The principle of detection using the above primer probes is as follows.

When a pathogen target is specifically amplified, annealing is performed at a relatively high first temperature (T1), the forward primer (F1-7) and the reverse primer (R1-7) specifically bind to a target sequence 1 (carbapenem antibiotics resistant KPC gene mutation) respectively and extend to generate a double-stranded pre-amplification product, with one single-stranded pre-amplification product (S1) sequentially having a probe anchoring region (A1), a target sequence, a reverse complementary sequence (h1') of a primer signal detection region, and a reverse complementary sequence (M') of an extension block region from a 5' end to a 3' end; the forward primer (F2-7) and the reverse primer (R2-7) specifically bind to a target sequence 2 (carbapenem antibiotics resistant IMP gene mutation) respectively and extend to generate a double-stranded pre-amplification product, with one single-stranded pre-amplification product (S2) sequentially having a probe anchoring region (A2), a target sequence, and a reverse complementary sequence (h2') of a primer signal detection region from a 5' end to a 3' end; and the forward primer (F3-7) and the reverse primer (R3-7) specifically bind to a target sequence 3 (carbapenem antibiotics resistant VIM gene mutation) respectively and extend to generate a double-stranded pre-amplification product, with one single-stranded pre-amplification product (S3) sequentially having a probe anchoring region (A3), a target sequence, and a reverse complementary sequence (h3') of a primer signal detection region from a 5' end to a 3' end.

After PCR amplification is completed, signal acquisition is performed at a relatively low first signal acquisition temperature (11). At this time, a probe signal detection region (H) of the probe (P1-7) is reversely and complementarily paired with the reverse complementary sequence (h1') of the primer signal detection region of the single-stranded pre-amplification product (S1), the primer anchoring region (A1') of the probe (P1-7) is reversely and complementarily paired with the probe anchoring region (A1) at the 5' end of the single-stranded pre-amplification product (S1), to obtain a hybrid double-stranded product (D1) formed with the probe (P1-7), while the reverse complementary sequence (M') of the extension block region at the 3' end of the single-stranded pre-amplification product (S1) is not complementary with any parts of the probe (P1-7) and the target sequence; and at this time, part of sequences of the probe (P1-7) changes from a single-stranded state to a double-stranded state, and the distance between a first detection group and a second detection group changes, thus generating a signal that may be detected by an instrument. While at this time, both the reverse complementary sequence (h2') of the primer signal detection region at the 3' end of the single-stranded pre-amplification product (S2) and the reverse complementary sequence (h3') of the primer signal detection region at the 3' end of the single-stranded pre-amplification product (S3) have annealing temperatures that are lower than the first annealing temperature (T1) and the first signal acquisition temperature (t1), and thus are not reversely complementary with the probe signal detection region (H) of the probe (P) and do not generate extending; after the completion of the first signal acquisition at the first signal acquisition temperature (t1), a secondary amplification cycle of the single-stranded pre-amplification products (S2 and S3) is performed with a cycle condition of high-temperature melting, and annealing is performed at a relatively low second annealing temperature (T2) (T2 < T1, and T2 < 11), at which time, the probe signal detection region (H) of the probe (P1-7) is complementarily paired with the reverse complementary sequence (h2') of the primer signal detection region at the 3' end of the single-stranded pre-amplification product (S2), the primer anchoring region (A2') of the probe is reversely and complementarily paired with the probe anchoring region (A2) at the 5' end of the single-stranded pre-amplification product (S2), the 3' end of the single-stranded pre-amplification product (S2) may continue extending to the 5' end of the probe (P), to obtain all reverse complementary sequences of the probe (P1-7), i.e., completing secondary amplification with the single-stranded pre-amplification product (S2) as a primer and the probe (P1-7) as a template, and an amplified double-stranded product (D2) formed with the probe (P1-7) is obtained, at which time, part of sequences of the probe (P1-7) changes from a single-stranded state to a double-stranded state, and the distance between a first detection group and a second detection group changes, thus generating a signal; at this time, the primer signal detection region (H) of the probe (P2-7) is complementarily paired with the reverse complementary sequence (h3') of the primer signal detection region at the 3' end of the single-stranded pre-amplification product (S3), the primer anchoring region (A3') of the probe is reversely and complementarily paired with the probe anchoring region (A3) at the 5' end of the single-stranded pre-amplification product (S3), the 3' end of the single-stranded pre-amplification product (S3) may continue extending to the 5' end of the probe (P2-7), to obtain all reverse complementary sequences of the probe (P2-7), i.e., completing secondary amplification with the single-stranded pre-amplification product (S3) as a primer and the probe (P2-7) as a template, and an amplified double-stranded product (D3) formed with the probe (P2-7) is obtained, at which time, part of sequences of the probe (P2-7) changes from a single-stranded state to a double-stranded state, and the distance between the first detection group and the second detection group changes, thus generating a signal.

(2) The above primer probe system is used for PCR detection, and a reaction system used in detection is shown in Table 14.

**Table 14 detection reaction system in Example 7**

| Reagent composition | Concentration |
|---|---|
| 2 × PCR Reaction Buffer | 1 × |
| DNA Polymerase | 2U |
| Forward primer (F1-7) | 250 nM |
| Reverse primer (R1-7) | 100 nM |
| Forward primer (F2-7) | 250 nM |
| Reverse primer (R2-7) | 100 nM |
| Forward primer (F3-7) | 250 nM |
| Reverse primer (R3-7) | 100 nM |
| Probe (P1-7) | 400 nM |
| Probe (P2-7) | 400 nM |
| Nucleic acid template for carbapenem antibiotics resistant KPC gene mutation nucleic acid template | 150 copies |
| Nucleic acid template for carbapenem antibiotics resistant IMP gene mutation nucleic acid template | 80 copies |
| Nucleic acid template for carbapenem antibiotics resistant VIM gene mutation nucleic acid template | 120 copies |
| Ultrapure water | Added to 20 µL |

| | |
|---|---|
| Note: in Table 14, 2X PCR Reaction Buffer includes 3 mM MgCl₂, 30 mM Tris-HCl with pH of 8.3, 0.5 mM dNTP, and 70 mM (NH₄)₂SO₄. | |

Sample preparation: carbapenem antibiotics resistant KPC gene mutation, IMP gene mutation and VIM gene mutation were used as positive samples, and the three samples were mixed for detection to verify the detectability of triple targets. Pure water was used as a no template control (NTC).

Reaction preparation:
after sample preparation was completed, the reaction system was configured according to the ratios in Table 14.

Digital PCR amplification and signal acquisition (the D600 full-automatic digital PCR analysis system and reagent consumables of Maccura Biotechnology Co., Ltd were used for detection):
after a digital PCR tube cap was sealed, the samples were gently mixed, and then briefly centrifuged and left for standing for 5 min at room temperature. A digital PCR tube was placed in a handheld centrifuge again, briefly centrifuged and transferred to a sample stand of a digital PCR instrument. Amplification and lighting procedures used were: initial denaturation at 95°C for 2 min; denaturation at 94°C for 10 s, and annealing and extending at 56°C for 30 s, for a total of 45 cycles; a first signal acquisition (VIC channel) at 60°C; denaturation at 94°C for 15 s, and annealing and extending at 52°C for 20 s, for a total of 5 cycles; and a second signal acquisition at 68°C (including two signal channel acquisitions, i.e., a VIC channel and a FAM channel, respectively).

Data analysis:
a result was interpreted through positive droplets under different signal channels and at different signal acquisition temperatures.

Fig. 8A is a diagram of droplets collected at a first signal acquisition temperature (60°C) under a VIC fluorescence channel in the example, and positive droplets different from the background can be seen and are carbapenem antibiotics resistant KPC gene mutation positive droplets.

Fig. 8B is a diagram of droplets collected at a second signal acquisition temperature (68°C) under the VIC fluorescence channel in the Example, positive droplets different from the background can be seen and are carbapenem antibiotics resistant IMP gene mutation positive droplets, and positions of the positive droplets presented at that temperature and under that channel are different from those of the positive droplets presented at the first signal acquisition temperature (60°C) and under the VIC channel.

Fig. 8C is a diagram of droplets collected at a second signal acquisition temperature (68°C) under a FAM fluorescence channel in the Example, positive droplets different from the background can be seen and are carbapenem antibiotics resistant VIM gene mutation positive droplets, and positions of the positive droplets presented at that temperature and under that channel are different from those of the positive droplets presented at the first signal acquisition temperature (60°C) and under the VIC channel and the second signal acquisition temperature (68°C) and under the VIC channel.

It can be seen from Figs. 8A-8C that by adopting the primer-probe composition and digital PCR detection method of the present invention, detection signals of different target sequences are obtained at different signal detection temperatures and under different signal acquisition channels, without crossing each other, and the detection result is more intuitive and clearer.

### Example 8

(1) A monoplex detection primer probe system for detecting non-small cell lung cancer EGFR Exon 20 H773_V774insH mutation is shown in Table 15:

**Table 15 base sequences of primers, probes in Example 8**

| Names of primer probes | Serial numbers | Nucleotide sequences (5'-3') | Modification |
|---|---|---|---|
| P1-8 | SEQ ID NO: 25 | CCCTGGGAGCTCATGGAATCGCTGGTCC TGTCGACGCTGCGCACACTGGGACGGCA CCG | T14-VIC, T26-BHQ1 3' Spacer C3 |
| R1-8 | SEQ ID NO: 26 | TTTTTATGGAATCGCTGGTCCTGTCGAAA AAGTCTGAACGACCTACACTCC | / |
| F1-8 | SEQ ID NO: 27 | GCTCCCAGGGTTTCCCAGCAGCTGACAC A | / |

In the above primer probes,
a forward primer F1-8 (SEQ ID NO: 27), as a specific primer designed for a target sequence of non-small cell lung cancer EGFR Exon 20 H773_V774insH mutation, has an overall length of 29 bp, with first to 16th bases at a 3' end as a target sequence binding region, and first to tenth bases at a 5' end as a probe anchoring region (A1), which is reversely complementary with first to tenth base sequences at a 5' end of a probe P1-8 (SEQ ID NO: 25), i.e., a primer anchoring region (A1');
a reverse primer R1-8 (SEQ ID NO: 26), as a specific primer designed for a target sequence of non-small cell lung cancer EGFR Exon 20 H773_V774insH mutation, has an overall length of 51 bp, with first to 21st bases at a 3' end as a target sequence binding region, first to fifth bases at a 5' end as an extension block region (M), and sixth to 27th bases at the 5' end as a primer signal detection region (h3), which is the same as 26th to 47th base sequences at a 3' end of the probe P1-8 (SEQ ID NO: 25).

The principle of detection using the above primer probes is as follows. When a target is specifically amplified, annealing is performed at a relatively high first temperature (T1), the forward primer (F1-8) and the reverse primer (R1-8) specifically bind to a target sequence 1 respectively and extend to generate a double-stranded pre-amplification product, with one single-stranded pre-amplification product (S1) sequentially having a probe anchoring region (A1), a target sequence, a reverse complementary sequence (h1') of a primer signal detection region, and a reverse complementary sequence (M') of an extension block region from a 5' end to a 3' end; and after PCR amplification is completed, signal acquisition is performed at a relatively low first signal acquisition temperature (11). At this time, a probe signal detection region (H) of the probe (P1-8) is reversely and complementarily paired with the reverse complementary sequence (h1') of the primer signal detection region of the single-stranded pre-amplification product (S1), the primer anchoring region (A1') of the probe (P1-8) is reversely and complementarily paired with the probe anchoring region (A1) at the 5' end of the single-stranded pre-amplification product (S1), to obtain a hybrid double-stranded product (D1) formed with the probe (P1-8), while the reverse complementary sequence (M') of the extension block region at the 3' end of the single-stranded pre-amplification product (S1) is not complementary with any parts of the probe (P1-8) and the target sequence; and at this time, all or part of sequences of the probe (P) changes from a single-stranded state to a double-stranded state, and the distance between a first detection group and a second detection group changes, thus generating a signal that may be detected by an instrument. After the completion of the signal acquisition at the first signal acquisition temperature (t1), it proceeds to a procedure of a cycle with a condition of high-temperature melting as well as annealing at a relatively low second annealing temperature (T2) (T2 < T1, and T2 < 11), and acquires signals at the second signal acquisition temperature (t2) (t2 > t1, and t2 > T1), at which time, since the second signal acquisition temperature (t2) is higher than the melting temperature of the hybrid double-stranded product (D1), the hybrid double-stranded product (D1) formed by the single-stranded pre-amplification product (S1) and the probe (P1-8) cannot form a double-stranded structure, and the distance between a first detection group and a second detection group changes, so that a fluorescence signal changes and may be detected by an instrument.

(2) The above primer probe system is used for PCR detection, and a reaction system used in detection is shown in Table 16.

**Table 16 detection reaction system in Example 8**

| Reagent composition | Concentration |
|---|---|
| 2 × PCR Reaction Buffer | 1 × |
| DNA Polymerase | 2U |
| Forward primer (F1-8) | 500 nM |
| Reverse primer (R1-8) | 100 nM |
| Probe (P1-8) | 400 nM |
| Nucleic acid template | 30 copies |
| Ultrapure water | Added to 20 µL |

| | |
|---|---|
| Note: in Table 16, 2X PCR Reaction Buffer includes 3 mM MgCl₂, 30 mM Tris-HCl with pH of 8.3, 0.5 mM dNTP, and 70 mM (NH₄)₂SO₄. | |

Sample preparation: non-small cell lung cancer EGFR Exon 20 H773_V774insH mutation DNA was used as a positive sample, and a non-small cell lung cancer EGFR Exon 20 H773_V774insH mutation target sequence was detected using the reverse primer (R1) with a signal detection region sequence to verify the detectability of a single mutation. Pure water was used as a no template control (NTC).

Reaction preparation:
after sample preparation was completed, the reaction system was configured according to the ratios in Table 16.

Digital PCR amplification and signal acquisition (the OS-500 digital PCR all-in-one machine and reagent consumables of Beijing DAWEIBIO Co., Ltd were used for detection):
after a digital PCR tube cap was sealed, the samples were gently mixed, and then briefly centrifuged and left for standing for 5 min at room temperature. A digital PCR tube was placed in a handheld centrifuge again, briefly centrifuged and transferred to a sample stand of a digital PCR instrument. Amplification and lighting procedures used were: initial denaturation at 95°C for 2 min; denaturation at 94°C for 10 s, and annealing and extending at 56°C for 30 s, for a total of 45 cycles; a first signal acquisition at 60°C; denaturation at 94°C for 15 s, and annealing and extending at 40°C for 20 s, for a total of 10 cycles; and a second signal acquisition at 78°C.

Data analysis (OS-500 digital PCR all-in-one machine analysis software of Beijing DAWEIBIO Co., Ltd was used for performing data analysis):
a result was interpreted through positive droplets at different signal acquisition temperatures.

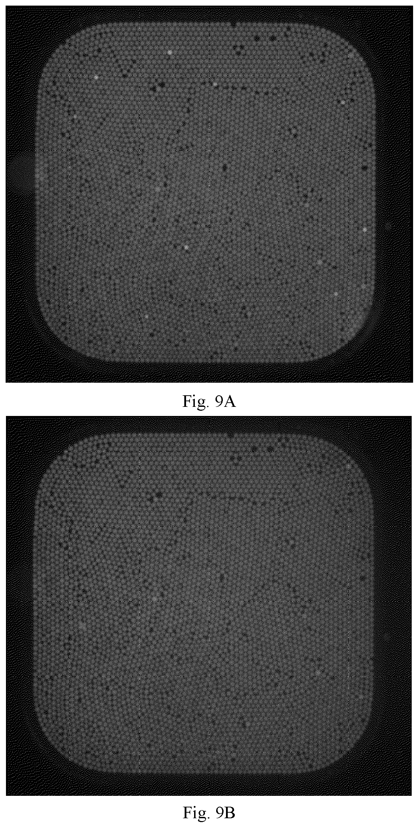

Fig. 9A is a droplet diagram of a monoplex-PCR non-small cell lung cancer EGFR Exon 20 H773_V774insH mutation positive sample amplified by the reverse primer (R1-8) at a first signal acquisition temperature (60°C), and positive droplets different from the background can be seen.

Fig. 9B is a droplet diagram of the monoplex-PCR non-small cell lung cancer EGFR Exon 20 H773_V774insH mutation positive sample amplified by the reverse primer (R1-8) at a second signal acquisition temperature (78°C), and it can be seen that all droplets are negative.

It can be seen from Figs. 9A and 9B that by adopting the primer-probe composition and digital PCR detection method of the present invention, different detection signal results can be obtained at different signal acquisition temperatures, and a target signal is turned "on" and "off' to achieve more accurately detection of a target.

### Example 9

(1) A monoplex detection primer probe system for detecting non-small cell lung cancer EGFR Exon 20 H773_V774insH mutation is shown in Table 17:

**Table 17 base sequences of primers, probes in Example 9**

| Names of primers, probes | Serial numbers | Nucleotide sequences (5'-3') | Modification |
|---|---|---|---|
| P1-9 | SEQ ID NO: 25 | CCCTGGGAGCTCATGGAATCGCTGGT CCTGTCGACGCTGCGCACACTGGGAC GGCACCG | T14-VIC, T26-BHQ1 3' Spacer C3 |
| R2-9 | SEQ ID NO: 28 | CGGCACCGAAAAGTCTGAACGACCTA CACTCC | / |
| F 1-9 | SEQ ID NO: 27 | GCTCCCAGGGTTTCCCAGCAGCTGAC ACA | / |

In the above primer probes,
a forward primer F1-9 (SEQ ID NO: 27), as a specific primer designed for a target sequence of non-small cell lung cancer EGFR Exon 20 H773_V774insH mutation, has an overall length of 29 bp, with first to 16th bases at a 3' end as a target sequence binding region, and first to tenth bases at a 5' end as a probe anchoring region (A1), which is reversely complementary with first to tenth base sequences at a 5' end of a probe P1-9 (SEQ ID NO: 25), i.e., a primer anchoring region (A1');
a reverse primer R2-9 (SEQ ID NO: 28), as a specific primer designed for a target sequence of non-small cell lung cancer EGFR Exon 20 H773_V774insH mutation, has an overall length of 32 bp, with first to 21st bases at a 3' end as a target sequence binding region, and first to eighth bases at a 5' end as a primer signal detection region (h2), which is the same as first to eighth base sequences at a 3' end of the probe P1-9 (SEQ ID NO: 25).

The principle of detection using the above primer probes is as follows. When a target is specifically amplified, annealing is performed at a relatively high first temperature (T1), the forward primer (F1-9) and the reverse primer (R2-9) specifically bind to a target sequence respectively and extend to generate a double-stranded pre-amplification product, with one single-stranded pre-amplification product (S2) sequentially having a probe anchoring region (A1), a target sequence, and a reverse complementary sequence (h2') of a primer signal detection region from a 5' end to a 3' end; a first signal acquisition is performed at a relatively low first signal acquisition temperature (t1) (t1 > T1), and the reverse complementary sequence (h2') of the primer signal detection region at the 3' end of the single-stranded pre-amplification product (S2) has an annealing temperature that is lower than the first annealing temperature (T1) and the first signal acquisition temperature (t1), and thus is not reversely complementary with a probe signal detection region (H) of the probe (P1-9) and do not generate extending; after the completion of the first signal acquisition at the first signal acquisition temperature (t1), it proceeds to a procedure of a cycle with a condition of high-temperature melting as well as annealing at a relatively low second annealing temperature (T2) (T2 < T1, and T2 < 11), at which time, the probe signal detection region (H) of the probe (P1-9) is complementarily paired with the reverse complementary sequence (h2') of the primer signal detection region at the 3' end of the single-stranded pre-amplification product (S2), the primer anchoring region (A1') of the probe is reversely and complementarily paired with the probe anchoring region (A1) at the 5' end of the single-stranded pre-amplification product (S2), the 3' end of the single-stranded pre-amplification product (S2) may continue extending to the 5' end of the probe (P1-9), to obtain a reverse complementary sequence of the probe (P1-9), i.e., completing secondary amplification with the single-stranded pre-amplification product (S2) as a primer and the probe (P1-9) as a template, to obtain an amplified double-stranded product (D2) formed with the probe (P1-9), at which time, the temperature is increased to a relatively high second signal acquisition temperature (t2) for signal acquisition (t2 > t1, and t2 > T1), the amplified double-stranded product (D2) formed by the single-stranded pre-amplification product (S2) and the probe (P1-9) forms a double-stranded state, the distance between a first detection group and a second detection group becomes longer, and the efficiency of fluorescence resonance energy transfer is relatively low, so that a fluorescence signal changes and may be detected by an instrument.

(2) The above primer probe system is used for PCR detection, and a reaction system used in detection is shown in Table 18.

**Table 18 detection reaction system in Example 9**

| Reagent composition | Concentration |
|---|---|
| 2 × PCR Reaction Buffer | 1 × |
| DNA Polymerase | 2U |
| Reverse transcriptase | 5U |
| Forward primer (F1-9) | 500 nM |
| Reverse primer (R2-9) | 100 nM |
| Probe (P1-9) | 400 nM |
| Nucleic acid template | 30 copies |
| Ultrapure water | Added to 20 µL |

| | |
|---|---|
| Note: in Table 18, 2X PCR Reaction Buffer includes 3 mM MgCl₂, 30 mM Tris-HCl with pH of 8.3, 0.5 mM dNTP, and 70 mM (NH₄)₂SO₄. | |

Sample preparation: non-small cell lung cancer EGFR Exon 20 H773_V774insH mutation DNA was used as a positive sample, and a non-small cell lung cancer EGFR Exon 20 H773_V774insH mutation target sequence was detected using the reverse primer (R2-9) with a signal detection region sequence to verify the detectability of a single mutation. Pure water was used as a no template control (NTC).

Reaction preparation:
after sample preparation was completed, the reaction system was configured according to the ratios in Table 18.

Digital PCR amplification and signal acquisition (the OS-500 digital PCR all-in-one machine and reagent consumables of Beijing DAWEIBIO Co., Ltd were used for detection):
after a digital PCR tube cap was sealed, the samples were gently mixed, and then briefly centrifuged and left for standing for 5 min at room temperature. A digital PCR tube was placed in a handheld centrifuge again, briefly centrifuged and transferred to a sample stand of a digital PCR instrument. Amplification and lighting procedures used were: initial denaturation at 95°C for 2 min; denaturation at 94°C for 10 s, and annealing and extending at 56°C for 30 s, for a total of 45 cycles; a first signal acquisition at 60°C; denaturation at 94°C for 15 s, and annealing and extending at 40°C for 20 s, for a total of 10 cycles; and a second signal acquisition at 78°C.

Data analysis (OS-500 digital PCR all-in-one machine analysis software of Beijing DAWEIBIO Co., Ltd was used for performing data analysis):
a result was interpreted through positive droplets at different signal acquisition temperatures.

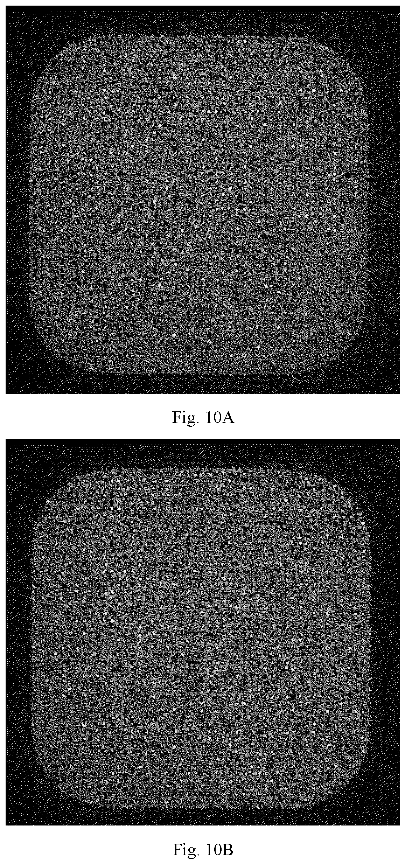

Fig. 10A is a droplet diagram of a monoplex-PCR non-small cell lung cancer EGFR Exon 20 H773_V774insH mutation positive sample amplified by the reverse primer (R2-9) at a first signal acquisition temperature (60°C), and it can be seen that all droplets are negative.

Fig. 10B is a droplet diagram of the monoplex-PCR non-small cell lung cancer EGFR Exon 20 H773_V774insH mutation positive sample amplified by the reverse primer (R2-9) at a second signal acquisition temperature (78°C), and positive droplets different from the background can be seen.

It can be seen from Figs. 10A and 10B that by adopting the primer-probe composition and digital PCR detection method of the present invention, different detection signal results may be obtained at different signal acquisition temperatures, and a target signal is turned "on" and "off" to achieve more accurately detection of a target.

### Example 10

(1) A monoplex detection primer probe system for detecting non-small cell lung cancer EGFR Exon 20 H773_V774insH mutation is shown in Table 19:

**Table 19 base sequences of primers, probes in Example 10**

| Names of primers, probes | Serial numbers | Nucleotide sequences (5'-3') | Modification |
|---|---|---|---|
| P1-10 | SEQ ID NO: 25 | CCCTGGGAGCTCATGGAATCGCTGGTC CTGTCGACGCTGCGCACACTGGGACGG | T14-VIC, T26-BHQ1 |
| | | CACCG | 3' Spacer C3 |
| R2-10 | SEQ ID NO: 28 | CGGCACCGAAAAGTCTGAACGACCTAC ACTCC | / |
| F1-10 | SEQ ID NO: 27 | GCTCCCAGGGTTTCCCAGCAGCTGACA CA | / |

In the above primer probes,
a forward primer F1-10 (SEQ ID NO: 27), as a specific primer designed for a target sequence of non-small cell lung cancer EGFR Exon 20 H773_V774insH mutation, has an overall length of 29 bp, with first to 16th bases at a 3' end as a target sequence binding region, and first to tenth bases at a 5' end as a probe anchoring region (A1), which is reversely complementary with first to tenth base sequences at a 5' end of a probe P1-10 (SEQ ID NO: 25), i.e., a primer anchoring region (A1');
a reverse primer R2-10 (SEQ ID NO: 28), as a specific primer designed for a target sequence of non-small cell lung cancer EGFR Exon 20 H773_V774insH mutation, has an overall length of 32 bp, with first to 21st bases at a 3' end as a target sequence binding region, and first to eighth bases at a 5' end as a primer signal detection region (h2), which is the same as first to eighth base sequences at a 3' end of the probe P1-10 (SEQ ID NO: 25).

The principle of detection using the above primer probes is as follows. When a target is specifically amplified, annealing is performed at a relatively high first temperature (T1), the forward primer (F1-10) and the reverse primer (R2-10) specifically bind to a target sequence 2 respectively and extend to generate a double-stranded pre-amplification product, with one single-stranded pre-amplification product (S3) sequentially having a probe anchoring region (A1), a target sequence, and a reverse complementary sequence (h2') of a primer signal detection region from a 5' end to a 3' end; a first signal acquisition is performed at a relatively low first signal acquisition temperature (t1) (t1 > T1), and the reverse complementary sequence (h2') of the primer signal detection region at the 3' end of the single-stranded pre-amplification product (S3) has an annealing temperature that is lower than the first annealing temperature (T1) and the first signal acquisition temperature (t1), and thus is not reversely complementary with a probe signal detection region (H) of the probe (P1-10) and do not generate extending; after the completion of the first signal acquisition at the first signal acquisition temperature (t1), it proceeds to a procedure of a cycle with a condition of constant-temperature amplifying as well as annealing at a relatively low constant second annealing temperature (T2) (T2 < T1, and T2 < 11), at which time, the probe signal detection region (H) of the probe (P1-10) is complementarily paired with the reverse complementary sequence (h2') of the primer signal detection region at the 3' end of the single-stranded pre-amplification product (S3), the primer anchoring region (A1') of the probe is reversely and complementarily paired with the probe anchoring region (A1) at the 5' end of the single-stranded pre-amplification product (S3), the 3' end of the single-stranded pre-amplification product (S3) may continue extending to the 5' end of the probe (P1), to obtain a reverse complementary sequence of the probe (P1-10), i.e., completing secondary amplification with the single-stranded pre-amplification product (S3) as a primer and the probe (P1-10) as a template, to obtain an amplified double-stranded product (D3) formed with the probe (P1-10), at which time, the temperature is increased to a relatively high second signal acquisition temperature (t2) for signal acquisition (t2 > t1, and t2 > T1), the amplified double-stranded product (D3) formed by the single-stranded pre-amplification product (S3) and the probe (P) formes a double-stranded state, the distance between a first detection group and a second detection group becomes longer, and the efficiency of fluorescence resonance energy transfer is relatively low, so that a fluorescence signal changes and can be detected by an instrument.

(2) The above primer probe system is used for PCR detection, and a reaction system used in detection is shown in Table 20.

**Table 20 detection reaction system in Example 10**

| Reagent composition | Concentration |
|---|---|
| 2 × PCR Reaction Buffer | 1 × |
| DNA Polymerase | 2U |
| Reverse transcriptase | 5U |
| Forward primer (F1-10) | 500 nM |
| Reverse primer (R2-10) | 100 nM |
| Probe (P1-10) | 400 nM |
| Nucleic acid template | 30 copies |
| Ultrapure water | Added to 20 µL |

| | |
|---|---|
| Note: in Table 20, 2X PCR Reaction Buffer includes 3 mM MgCl₂, 30 mM Tris-HCl with pH of 8.3, 0.5 mM dNTP, and 70 mM (NH₄)₂SO₄. | |

Sample preparation: non-small cell lung cancer EGFR Exon 20 H773_V774insH mutation DNA was used as a positive sample, and a non-small cell lung cancer EGFR Exon 20 H773_V774insH mutation target sequence was detected using the reverse primer (R2-10) with a signal detection region sequence to verify the detectability of a single mutation. Pure water was used as a no template control (NTC).

Reaction preparation:
after sample preparation was completed, the reaction system was configured according to the ratios in Table 20.

Digital PCR amplification and signal acquisition (the OS-500 digital PCR all-in-one machine and reagent consumables of Beijing DAWEIBIO Co., Ltd were used for detection):
after a digital PCR tube cap was sealed, the samples were gently mixed, and then briefly centrifuged and left for standing for 5 min at room temperature. A digital PCR tube was placed in a handheld centrifuge again, briefly centrifuged and transferred to a sample stand of a digital PCR instrument. Amplification and lighting procedures used were: initial denaturation at 95°C for 2 min; denaturation at 94°C for 10 s, and annealing and extending at 56°C for 30 s, for a total of 45 cycles; a first signal acquisition at 60°C; constant-temperature incubation at 40°C for 5 min; and a second signal acquisition at 78°C.

Data analysis (OS-500 digital PCR all-in-one machine analysis software of Beijing DAWEIBIO Co., Ltd was used for performing data analysis):
a result was interpreted through positive droplets at different signal acquisition temperatures.

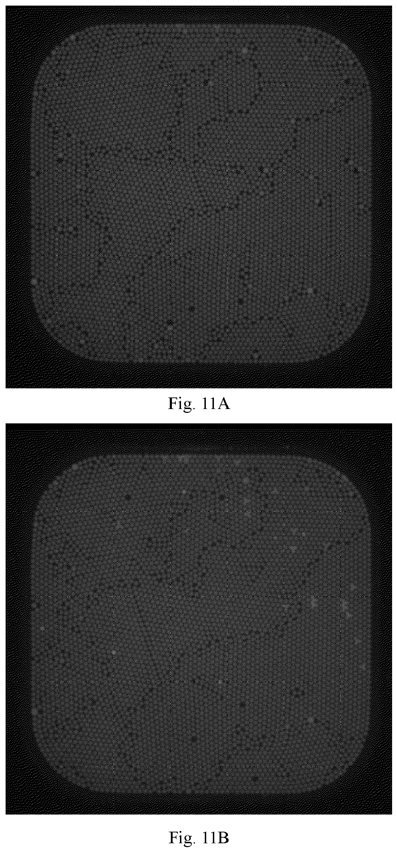

Fig. 11A is a droplet diagram of a monoplex-PCR non-small cell lung cancer EGFR Exon 20 H773_V774insH mutation positive sample amplified by the reverse primer (R2-10) at a first signal acquisition temperature (60°C), and it can be seen that all droplets are negative (droplets that are at edges and/or appear larger are by an experimental operation problem and are not positive droplets).

Fig. 11B is a droplet diagram of the monoplex-PCR non-small cell lung cancer EGFR Exon 20 H773_V774insH mutation positive sample amplified by the reverse primer (R2-10) at a second signal acquisition temperature (78°C), and positive droplets different from the background can be seen.

It can be seen from Figs. 11A and 11B that by adopting the primer-probe composition and digital PCR detection method of the present invention, different detection signal results may be obtained at different signal acquisition temperatures, and a target signal is turned "on" and "off" to achieve more accurately detection of a target.

### Example 11

(1) A duplex detection primer probe system for detecting Influenza A virus (IAV) and Respiratory Syncytial Virus (RSV) is shown in Table 21:

**Table 21 base sequences of primers, probes in Example 11**

| Names of primers, probes | Serial numbers | Nucleotide sequences (5'-3') | Modification |
|---|---|---|---|
| P1-11 | SEQ ID NO: 25 | CCCTGGGAGCTCATGGAATCGCTGG TCCTGTCGACGCTGCGCACACTGGG ACGGCACCG | T14-VIC, T26-BHQ1 3' Spacer C3 |
| R1-11 | SEQ ID NO: 29 | TTTTTATGGAATCGCTGGTCCTGTCG AAAATATTGCAGATGTGACACCTTAG TA | / |
| F1-11 | SEQ ID NO: 30 | GCTCCCAGGGTTTAACCTGGACTAA GAAGAGGAACGACAGAT | / |
| F2-11 | SEQ ID NO: 31 | GCTCCCAGGGTTTGACTAGCAACCT CCATGGC | / |
| R2-11 | SEQ ID NO: 32 | CGGCACCGAAAATAATCAGGCACGA GAACAG | / |

In the above primer probes,
a forward primer F1-11 (SEQ ID NO: 30), as a specific primer designed for a target sequence of Respiratory Syncytial Virus, has an overall length of 42 bp, with first to 29th bases at a 3' end as a target sequence binding region, and first to tenth bases at a 5' end as a probe anchoring region (A), which is reversely complementary with first to tenth base sequences at a 5' end of a probe P1-11 (SEQ ID NO: 25), i.e., a primer anchoring region (A');
a reverse primer R1-11 (SEQ ID NO: 29), as a specific primer designed for a target sequence of Respiratory Syncytial Virus, has an overall length of 54 bp, with first to 24th bases at a 3' end as a target sequence binding region, first to sixth bases at a 5' end as a signal block region (M), and seventh to 27th bases at the 5' end as a primer signal detection region (h1), which is the same as 26th to 46th base sequences at a 3' end of the probe P1-11 (SEQ ID NO: 25);
a forward primer F2-11 (SEQ ID NO: 31), as a specific primer designed for a target sequence of Influenza A virus, has an overall length of 32 bp, with first to 20th bases at a 3' end as a target sequence binding region, and first to tenth bases at a 5' end as a probe anchoring region (A), which is reversely complementary with first to tenth base sequences at the 5' end of the probe P1-11 (SEQ ID NO: 25), i.e., the primer anchoring region (A');
a reverse primer R2-11 (SEQ ID NO: 32), as a specific primer designed for a target sequence of Influenza A virus, has an overall length of 31 bp, with first to 19th bases at a 3' end as a target sequence binding region, and first to eighth bases at a 5' end being the same as first to eighth base sequences at the 3' end of the probe P1-11 (SEQ ID NO: 25).

The principle of detection using the above primer probes is as follows. When a target is specifically amplified, annealing is performed at a relatively high first temperature (T1), the forward primer (F1-11) and the reverse primer (R1-11) specifically bind to a target sequence 1 (Respiratory Syncytial Virus) respectively and extend to generate a double-stranded pre-amplification product, with one single-stranded pre-amplification product (S1) sequentially having a probe anchoring region (A1), a target sequence, a reverse complementary sequence (h1') of a primer signal detection region, and a reverse complementary sequence (M') of an extension block region from a 5' end to a 3' end; and the forward primer (F2-11) and the reverse primer (R2-11) specifically bind to a target sequence 2 (Influenza A virus) respectively and extend to generate a double-stranded pre-amplification product, with one single-stranded pre-amplification product (S2) sequentially having a probe anchoring region (A2), a target sequence, and a reverse complementary sequence (h2') of a primer signal detection region from a 5' end to a 3' end. After PCR amplification is completed, signal acquisition is performed at a relatively low first signal acquisition temperature (11). At this time, a probe signal detection region (H) of the probe (P1-11) is reversely and complementarily paired with the reverse complementary sequence (h1') of the primer signal detection region of the single-stranded pre-amplification product (S1), the primer anchoring region (A1') of the probe (P1-11) is reversely and complementarily paired with the probe anchoring region (A1) at the 5' end of the single-stranded pre-amplification product (S1), to obtain a hybrid double-stranded product (D1) formed with the probe (P1-11), while the reverse complementary sequence (M') of the extension block region at the 3' end of the single-stranded pre-amplification product (S1) is not complementary with any parts of the probe (P1-11) and the target sequence; and at this time, part of sequences of the probe (P1-11) changes from a single-stranded state to a double-stranded state, and the distance between a first detection group and a second detection group changes, thus generating a signal that may be detected by an instrument. While at this time, the reverse complementary sequence (h2') of the primer signal detection region at the 3' end of the single-stranded pre-amplification product (S2) has an annealing temperature that is lower than the first annealing temperature (T1) and the first signal acquisition temperature (t1), and thus is not reversely complementary with a probe signal detection region (H) of the probe (P1-11) and do not generate extending; after the completion of the first signal acquisition at the first signal acquisition temperature (t1), a secondary amplification cycle of the single-stranded pre-amplification product (S2) is performed with a cycle condition of high-temperature melting, and annealing is performed at a relatively low second annealing temperature (T2) (T2 < T1, and T2 < 11), at which time, the probe signal detection region (H) of the probe (P1-11) is complementarily paired with the reverse complementary sequence (h2') of the primer signal detection region at the 3' end of the single-stranded pre-amplification product (S2), the primer anchoring region (A2') of the probe is reversely and complementarily paired with the probe anchoring region (A2) at the 5' end of the single-stranded pre-amplification product (S2), the 3' end of the single-stranded pre-amplification product (S2) may continue extending to the 5' end of the probe (P1-11), to obtain all reverse complementary sequences of the probe (P1-11), i.e., completing secondary amplification with the single-stranded pre-amplification product (S2) as a primer and the probe (P1-11) as a template, to obtain an amplified double-stranded product (D2) formed with the probe (P1-11), at which time, the temperature is increased to a relatively high second signal acquisition temperature (t2) for signal acquisition (t2 > t1, and t2 > T1), the amplified double-stranded product (D2) formed by the single-stranded pre-amplification product (S2) and the probe (P1-11) forms a double-stranded state, the distance between the first detection group and the second detection group becomes longer, and the efficiency of fluorescence resonance energy transfer is relatively low, so that a fluorescence signal changes and may be detected by an instrument; and at this time, since the second signal acquisition temperature (t2) is higher than the melting temperature of the hybrid double-stranded product (D1), the hybrid double-stranded product (D1) formed by the single-stranded pre-amplification product (S1) and the probe (P1-11) cannot form a double-stranded structure.

(2) The above primer probe system is used for PCR detection, and a reaction system used in detection is shown in Table 22.

**Table 22 detection reaction system in Example 11**

| Reagent composition | Concentration |
|---|---|
| 2 × PCR Reaction Buffer | 1 × |
| DNA Polymerase | 2U |
| Reverse transcriptase | 5U |
| Forward primer (F1-11) | 500 nM |
| Reverse primer (R1-11) | 100 nM |
| Forward primer (F2-11) | 500 nM |
| Reverse primer (R2-11) | 100 nM |
| Probe (P1-11) | 400 nM |
| Nucleic acid template | 30 copies |
| Ultrapure water | Added to 20 µL |

| | |
|---|---|
| Note: in Table 22, 2X PCR Reaction Buffer includes 3 mM MgCl₂, 30 mM Tris-HCl with pH of 8.3, 0.5 mM dNTP, and 70 mM (NH₄)₂SO₄. | |

Sample preparation: Influenza A virus (IAV) and Respiratory Syncytial Virus (RSV) were used as positive samples, target sequences of Influenza A virus (IAV) and Respiratory Syncytial Virus (RSV) were detected using reverse primers (R1-11 and R2-11) with different signal detection region sequences to verify the detectability of duplex virus infections. Pure water was used as a no template control (NTC).

Reaction preparation:
after sample preparation was completed, the reaction system was configured according to the ratios in Table 22.

Digital PCR amplification and signal acquisition (the OS-500 digital PCR all-in-one machine and reagent consumables of Beijing DAWEIBIO Co., Ltd were used for detection):
after a digital PCR tube cap was sealed, the samples were gently mixed, and then briefly centrifuged and left for standing for 5 min at room temperature. A digital PCR tube was placed in a handheld centrifuge again, briefly centrifuged and transferred to a sample stand of a digital PCR instrument. Amplification and lighting procedures used were: reverse transcription at 55°C for 15 min; initial denaturation at 95°C for 2 min, denaturation at 94°C for 10 s, and annealing and extending at 56°C for 30 s, for a total of 45 cycles; a first signal acquisition at 60°C; denaturation at 94°C for 15 s, and annealing and extending at 40°C for 20 s, for a total of 10 cycles; and a second signal acquisition at 78°C.

Data analysis (OS-500 digital PCR all-in-one machine analysis software of Beijing DAWEIBIO Co., Ltd was used for performing data analysis):
a result was interpreted through positive droplets at different signal acquisition temperatures.

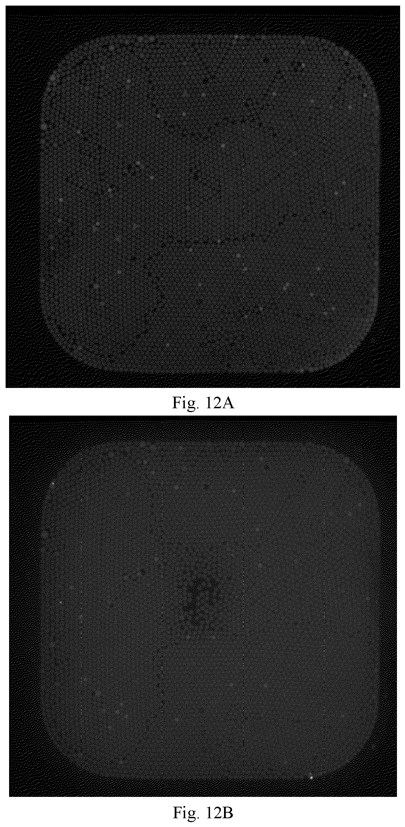

Fig. 12A is a droplet diagram of Influenza A virus (IAV) and Respiratory Syncytial Virus (RSV) positive samples in the Example at a first signal acquisition temperature (60°C), and positive droplets different from the background can be seen and are Influenza A virus positive droplets (droplets that are at edges and/or appear larger are caused by an experimental operation problem and are not positive droplets).

Fig. 12B is a droplet diagram of the Influenza A virus (IAV) and Respiratory Syncytial Virus (RSV) positive samples in the Example at a second signal acquisition temperature (78°C), positive droplets different from the background can be seen and are Respiratory Syncytial Virus positive droplets, and positions of the positive droplets presented at that temperature are different from those of the positive droplets presented at the first signal acquisition temperature (60°C).

It can be seen from Figs. 12A and 12B that by adopting the primer-probe composition and digital PCR detection method of the present invention, detection signals of different target sequences are achieved to be obtained at different signal detection temperatures (t1 and t2) respectively, without crossing each other.

### Example 12

(1) A triple detection primer probe system for detecting Influenza A virus (IAV), Influenza B virus (IBV) and Respiratory Syncytial Virus (RSV) is shown in Table 23:

**Table 23 base sequences of primers, probes in Example 12**

| Names of primers, probes | Serial numbers | Nucleotide sequences (5'-3') | Modification |
|---|---|---|---|
| P1-12 | SEQ ID NO: 33 | GTCACAGCTGTCATGAAGCCGACACT GTCCGTGGAGCGACGTGGACCTCTGG ACGTCCGG | T14-VIC, T26-BHQ1 3' Spacer C3 |
| R1-12 | SEQ ID NO: 34 | TTTTTATGAAGCCGACACTGTCCGAA AAGACTAGCAACCTCCATGGC | / |
| F1-12 | SEQ ID NO: 35 | CAGCTGTGACTTTATAATCAGGCACG AGAACAG | / |
| R2-12 | SEQ ID NO: 36 | GAGCGACGTGGAAAAATGAGAACTT GCCATAGGTT | / |
| F2-12 | SEQ ID NO: 37 | CAGCTGTGACTTTCTAAGCAGACTCA CAGAACT | / |
| R3-12 | SEQ ID NO: 38 | CGTCCGGTTTGCAATTCAGCATCACA GAC | |
| F3-12 | SEQ ID NO: 39 | CAGCTGTGACAAAAAGCAACCAACA ACATTCATTG | |

In the above primer probes,
a forward primer F1-12 (SEQ ID NO: 35), as a specific primer designed for a target sequence of Influenza A virus (IAV), has an overall length of 33 bp, with first to 20th bases at a 3' end as a target sequence binding region, and first to tenth bases at a 5' end as a probe anchoring region (A), which is reversely complementary with first to tenth base sequences at a 5' end of a probe P1-12 (SEQ ID NO: 33), i.e., a primer anchoring region (A');
a reverse primer R1-12 (SEQ ID NO: 34), as a specific primer designed for a target sequence of Influenza A virus (IAV), has an overall length of 47 bp, with first to 20th bases at a 3' end as a target sequence binding region, first to fifth bases at a 5' end as a signal block region (M), and sixth to 24th bases at the 5' end as a primer signal detection region (h1), which is the same as 30th to 48th base sequences at a 3' end of the probe P1-12 (SEQ ID NO: 33);
a forward primer F2-12 (SEQ ID NO: 37), as a specific primer designed for a target sequence of Influenza B virus (IBV), has an overall length of 33 bp, with first to 20th bases at a 3' end as a target sequence binding region, and first to tenth bases at a 5' end as a probe anchoring region (A), which is reversely complementary with first to tenth base sequences at the 5' end of the probe P1-12 (SEQ ID NO: 33), i.e., the primer anchoring region (A');
a reverse primer R2-12 (SEQ ID NO: 36), as a specific primer designed for a target sequence of Influenza B virus (IBV), has an overall length of 35 bp, with first to 21st bases at a 3' end as a target sequence binding region, and first to 11th bases at a 5' end being the same as 17th to 27th base sequences at the 3' end of the probe P1-12 (SEQ ID NO: 33).

A forward primer F3-12 (SEQ ID NO: 39), as a specific primer designed for a target sequence of Respiratory Syncytial Virus (RSV), has an overall length of 35 bp, with first to 22nd bases at a 3' end as a target sequence binding region, and first to tenth bases at a 5' end as a probe anchoring region (A), which is reversely complementary with first to tenth base sequences at the 5' end of the probe P1-12 (SEQ ID NO: 33), i.e., the primer anchoring region (A');
a reverse primer R3-12 (SEQ ID NO: 38), as a specific primer designed for a target sequence of Respiratory Syncytial Virus (RSV), has an overall length of 29 bp, with first to 19th bases at a 3' end as a target sequence binding region, and first to seventh bases at a 5' end being the same as first to seventh base sequences at the 3' end of the probe P1-12 (SEQ ID NO: 33).

The principle of detection using the above primer probes is as follows. When a target is specifically amplified, annealing is performed at a relatively high first temperature (T1), the forward primer (F1-12) and the reverse primer (R1-12) specifically bind to a target sequence 1 (Respiratory Syncytial Virus) respectively and extend to generate a double-stranded pre-amplification product, with one single-stranded pre-amplification product (S1) sequentially having a probe anchoring region (A1), a target sequence, a reverse complementary sequence (h1') of a primer signal detection region, and a reverse complementary sequence (M') of an extension block region from a 5' end to a 3' end; the forward primer (F2-12) and the reverse primer (R2-12) specifically bind to a target sequence 2 (Influenza B virus) respectively and extend to generate a double-stranded pre-amplification product, with one single-stranded pre-amplification product (S2) sequentially having a probe anchoring region (A2), a target sequence, and a reverse complementary sequence (h2') of a primer signal detection region from a 5' end to a 3' end; and the forward primer (F3-12) and the reverse primer (R3-12) specifically bind to a target sequence 3 and extend to generate a double-stranded pre-amplification product, with one single-stranded pre-amplification product (S3) sequentially having a probe anchoring region (A3), a target sequence, and a reverse complementary sequence (h3') of a primer signal detection region from a 5' end to a 3' end. After PCR amplification is completed, signal acquisition is performed at a relatively low first signal acquisition temperature (11). At this time, a probe signal detection region (H) of the probe (P1-12) is reversely and complementarily paired with the reverse complementary sequence (h1') of the primer signal detection region of the single-stranded pre-amplification product (S1), the primer anchoring region (A1') of the probe (P1-12) is reversely and complementarily paired with the probe anchoring region (A1) at the 5' end of the single-stranded pre-amplification product (S1), to obtain a hybrid double-stranded product (D1) formed with the probe (P1-12), while the reverse complementary sequence (M') of the extension block region at the 3' end of the single-stranded pre-amplification product (S1) is not complementary with any parts of the probe (P1-12) and the target sequence; and at this time, part of sequences of the probe (P1-12) changes from a single-stranded state to a double-stranded state, and the distance between a first detection group and a second detection group changes, thus generating a signal that can be detected by an instrument. While at this time, both the reverse complementary sequence (h2') of the primer signal detection region at the 3' end of the single-stranded pre-amplification product (S2) and the reverse complementary sequence (h3') of the primer signal detection region at the 3' end of the single-stranded pre-amplification product (S3) have annealing temperatures that are lower than the first annealing temperature (T1) and the first signal acquisition temperature (t1), and thus are not reversely complementary with the probe signal detection region (H) of the probe (P1-12) and do not generate extending; after the completion of the first signal acquisition at the first signal acquisition temperature (t1), a secondary amplification cycle of the single-stranded pre-amplification product (S2) is performed with a cycle condition of high-temperature melting, and annealing is performed at a relatively low second annealing temperature (T2) (T2 < T1, and T2 < 11), at which time, the probe signal detection region (H) of the probe (P1-12) is complementarily paired with the reverse complementary sequence (h2') of the primer signal detection region at the 3' end of the single-stranded pre-amplification product (S2), the primer anchoring region (A2') of the probe is reversely and complementarily paired with the probe anchoring region (A2) at the 5' end of the single-stranded pre-amplification product (S2), the 3' end of the single-stranded pre-amplification product (S2) may continue extending to the 5' end of the probe (P1-12), to obtain all reverse complementary sequences of the probe (P1-12), i.e., completing secondary amplification with the single-stranded pre-amplification product (S2) as a primer and the probe (P1-12) as a template, and an amplified double-stranded product (D2) formed with the probe (P1-12) is obtained, at which time, all sequences of the probe (P1-12) change from the single-stranded state to the double-stranded state, and the distance between the first detection group and the second detection group changes, thus generating a signal; while at this time, since the second signal acquisition temperature (t2) is higher the melting temperature of the hybrid double-stranded product (D1), the hybrid double-stranded product (D1) formed by the single-stranded pre-amplification product (S1) and the probe (P1-12) cannot form a double-stranded structure; and the reverse complementary sequence (h3') of the primer signal detection region at the 3' end of the single-stranded pre-amplification product (S3) has an annealing temperature that is lower than the second annealing temperature (T2), and thus is not reversely complementary with the primer signal detection region (H) of the probe (P1-12) and do not generate extending; after the completion of the second signal acquisition at the second signal acquisition temperature (t2), a secondary amplification cycle of the single-stranded pre-amplification product (S3) is performed with a cycle condition of high-temperature melting, and annealing is performed at a lower third annealing temperature (T3) (T3 < T2, and T3 < t2), at which time, the probe signal detection region (H) of the probe (P1-12) is complementarily paired with the reverse complementary sequence (h3') of the primer signal detection region at the 3' end of the single pre-amplification product (S3), the primer anchoring region (A3') of the probe is reversely and complementarily paired with the probe anchoring region (3A) at the 5' end of the single pre-amplification product (S3), the 3' end of the single pre-amplification product (S3) may continue extending to the 5' end of the probe (P1-12), to obtain part or all of reverse complementary sequences of the probe (P1-12), i.e., completing secondary amplification with the single-stranded pre-amplification product (S3) as a primer and the probe (P1-12) as a template, to obtain an amplified double-stranded product (D2) formed with the probe (P1-12), at which time, all or part of sequences of the probe (P1-12) changes from a single-stranded state to a double-stranded state, and the distance between the first detection group and the second detection group changes, thus generating a signal; after the completion of cyclic amplification at the third annealing temperature (T3), the temperature is increased to a higher third signal acquisition temperature (t3) for a second signal acquisition (t3 > t2, an t3 > T2), at which time, since the third signal acquisition temperature (t3) is higher than the melting temperature of the hybrid double-stranded product (D1), the hybrid double-stranded product (D1) formed by the single-stranded pre-amplification product (S1) and the probe (P1-12) cannot form a double-stranded structure; since the third signal acquisition temperature (t3) is higher than the melting temperature of the amplified double-stranded product (D2), the amplified double-stranded product (D2) formed by the single-stranded pre-amplification product (S2) and the probe (P1-12) cannot form a double-stranded structure; while the amplified double-stranded product (D3) formed by the single-stranded pre-amplification product (S3) and the probe (P1-12) form a double-stranded state, the distance between the first detection group and the second detection group becomes longer, and the efficiency of fluorescence resonance energy transfer is relatively low, so that a fluorescence signal changes and can be detected by an instrument.

(2) The above primer probe system is used for PCR detection, and a reaction system used in detection is shown in Table 24.

**Table 24 detection reaction system in Example 12**

| Reagent composition | Concentration |
|---|---|
| 2 × PCR Reaction Buffer | 1 × |
| DNA Polymerase | 2U |
| Reverse transcriptase | 5U |
| Forward primer (F1-12) | 250 nM |
| Reverse primer (R2-12) | 100 nM |
| Forward primer (F2-12) | 250 nM |
| Reverse primer (R2-12) | 100 nM |
| Forward primer (F3-12) | 250 nM |
| Reverse primer (R3-12) | 100 nM |
| Probe (P1-12) | 400 nM |
| Nucleic acid template | 30 copies |
| Ultrapure water | Added to 20 µL |

| | |
|---|---|
| Note: in Table 24, 2X PCR Reaction Buffer includes 3 mM MgCl₂, 30 mM Tris-HCl with pH of 8.3, 0.5 mM dNTP, and 70 mM (NH₄)₂SO₄. | |

Sample preparation: Influenza A virus (IAV), Influenza B virus (IBV) and Respiratory Syncytial Virus (RSV) were used as positive samples, target sequences of Influenza A virus (IAV), Influenza B virus (IBV) and Respiratory Syncytial Virus (RSV) were detected using reverse primers (R1-12/R2-12/R3-12) with different signal detection region sequences to verify the detectability of triple virus infections. Pure water was used as a no template control (NTC).

Reaction preparation:
after sample preparation was completed, the reaction system was configured according to the ratios in Table 24.

Digital PCR amplification and signal acquisition (the OS-500 digital PCR all-in-one machine and reagent consumables of Beijing DAWEIBIO Co., Ltd were used for detection):
after a digital PCR tube cap was sealed, the samples were gently mixed, and then briefly centrifuged and left for standing for 5 min at room temperature. A digital PCR tube was placed in a handheld centrifuge again, briefly centrifuged and transferred to a sample stand of a digital PCR instrument. Amplification and lighting procedures used were: reverse transcription at 55°C for 15 min; initial denaturation at 95°C for 2 min, denaturation at 94°C for 10 s, and annealing and extending at 56°C for 30 s, for a total of 45 cycles; a first signal acquisition at 61°C; denaturation at 94°C for 15 s, and annealing and extending at 52°C for 20 s, for a total of 5 cycles; and a second signal acquisition at 68°C. Denaturation at 94°C for 15 s, and annealing and extending at 40°C for 20 s, for a total of 5 cycles; and a third signal acquisition at 76°C.

Data analysis (OS-500 digital PCR all-in-one machine analysis software of Beijing DAWEIBIO Co., Ltd was used for performing data analysis):
a result was interpreted through positive droplets at different signal acquisition temperatures.

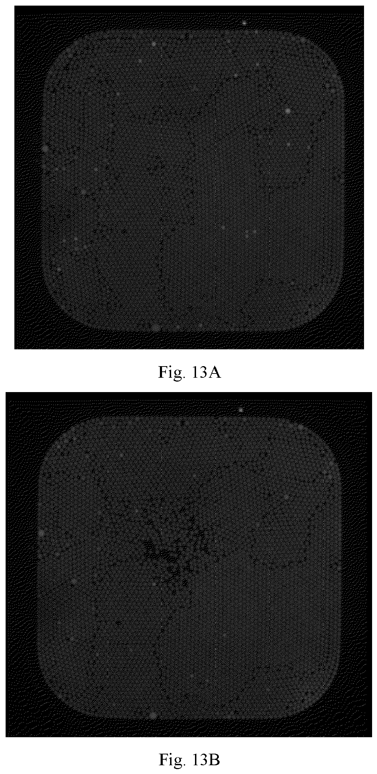

Fig. 13A is a droplet diagram of Influenza A virus (IAV), Influenza B virus (IBV) and Respiratory Syncytial Virus (RSV) positive samples in the Example at a first signal acquisition temperature (61°C), and positive droplets different from the background can be seen and are Influenza A virus positive droplets.

Fig. 13B is a droplet diagram of the Influenza A virus (IAV), Influenza B virus (IBV) and Respiratory Syncytial Virus (RSV) positive samples in the Example at a second signal acquisition temperature (68°C), positive droplets different from the background can be seen and are Influenza B virus positive droplets, and positions of the positive droplets presented at that temperature are different from those of the positive droplets presented at the first signal acquisition temperature (61°C).

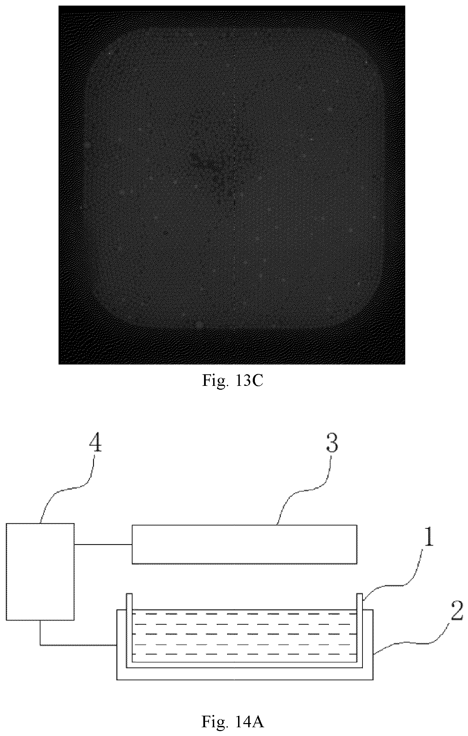

Fig. 13C is a droplet diagram of the Influenza A virus (IAV), Influenza B virus (IBV) and Respiratory Syncytial Virus (RSV) positive samples in the Example at a third signal acquisition temperature (76°C), positive droplets are Respiratory Syncytial Virus positive droplets, and positions of the positive droplets presented at that temperature are different from those of the positive droplets presented at the first signal acquisition temperature (61°C) and the positive droplets presented at the second signal acquisition temperature (68°C).

It can be seen from Figs. 13A to 13C that by adopting the primer-probe composition and digital PCR detection method of the present invention, detection signals of different target sequences are achieved to be obtained at different signal detection temperatures (11, t2, and t3) respectively, without crossing each other.

The above are only preferred examples of the present invention. It should be pointed out that for a person of ordinary skill in the art, under the technical inspiration provided by the present invention, as common general knowledge in the art, other equivalent variants and improvements may further be made and should also be regarded as falling within the scope of protection of the present invention.

### Second implementation

It should be noted that terms "including" and "having" and any variants thereof in the examples and drawings of the present application are intended to cover non-exclusive inclusion. For example, a process, method, system, product or apparatus including a series of steps or units is not limited to steps or units that are listed, but optionally further includes steps or units that are not listed, or optionally further includes other steps or units that are inherent to these processes, methods, products or apparatuses.

It may be understood that terms "first", "second", etc. used in the present application may be used herein for describing various elements, but these elements are not limited to these terms. These terms are only used for distinguishing the first element from another element. For example, without departing from the scope of the present application, a first mobile information may be referred to as a second mobile information, and similarly, the second mobile information may be referred to as the first mobile information. Both the first mobile information and the second mobile information are mobile informations, but they are not the same mobile information. In addition, it should be noted that the term "a plurality of' used in the examples of the present application refers to two or more.

The technical solutions of the present application will be further illustrated below by way of examples.

The multiple time-sharing nucleic acid detection device of the implementation is used for detecting a sample to be detected. The sample to be detected may include one kind of component to be detected, and may also include two, three, four, five or even more kinds of components to be detected. Multiple detection of the components to be detected may be realized through the multiple time-sharing nucleic acid detection device of the implementation.

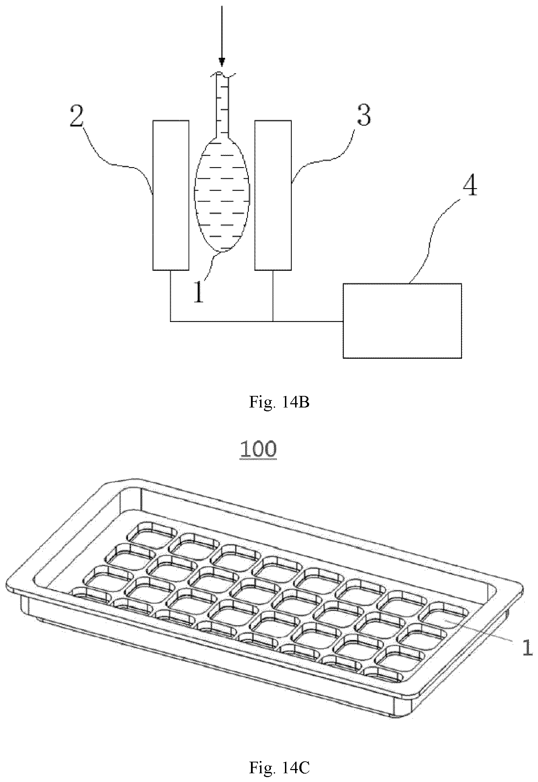

Referring to Figs. 14A and 14B, the multiple time-sharing nucleic acid detection device of the implementation includes a reaction liquid containing part 1, a temperature adjustment part 2, a signal detection part 3, and a control part 4. Wherein, the reaction liquid containing part 1 is configured to contain a sample containing a component to be detected and a reagent capable of reacting with the component to be detected, to provide a place for various reaction between the sample to be detected and the reagent. Wherein, the temperature adjustment part 2 is configured to adjust the temperature of a mixed liquid in the reaction liquid containing part 1 to make the component to be detected and the reagent react differently at different temperatures. Wherein, the signal detection part 3 is configured to detect a signal generated from a specific substance undergoing specific reaction in the mixed liquid, and the signal may be an optical signal and may also be an electric signal. Wherein, the control part 4 is configured to control the temperature adjustment part 2 to adjust the temperature of the mixed liquid in the reaction liquid containing part 1 in different temperature modes, and control the signal detection part 3 to detect the signal generated from the specific substance in the mixed liquid.

### I. Sample

The sample is a body fluid from a human body or an animal body, and includes different components to be detected, which may be nucleic acids or proteins. The implementation illustrates the technical solutions with the components to be detected being the nucleic acids. For the sample directly taken from the human body or the animal body, nucleic acid extraction purification operation may be performed to extract the nucleic acids, which may be performed manually or with a specialized nucleic acid extractor. For the sample directly taken from the human body or the animal body, the nucleic acid extraction purification operation may also not be performed. The sample includes different nucleic acid molecules to be detected, such as a first component to be detected (a first nucleic acid to be detected), a second component to be detected (a second nucleic acid to be detected), a third component to be detected (a third nucleic acid to be detected), a fourth component to be detected (a fourth nucleic acid to be detected), and a fifth component to be detected (a fifth nucleic acid to be detected).

### II. Reagent

The reagent includes an amplification substance for amplifying nucleic acids and a labeling substance for labeling the nucleic acids. The amplification substance may amplify different nucleic acid molecules, and the labeling substance can bind to the nucleic acids at a certain temperature to generate a detectable signal. The different nucleic acids bind to the labeling substance over different temperature ranges, and separate from the labeling substance over different temperature ranges after binding. Primers in the amplification substance for amplifying the different nucleic acids to be detected are different, and the labeling substance for labeling the different nucleic acids to be detected may be the same and may also be different. The present application uses the same labeling substance to label different nucleic acids to be detected.

### III. Micro liquids

The micro liquids may be tiny droplets formed in an emulsion, and may also be trace liquids contained in micro wells of a container. Each micro liquid contains at most one unit of nucleic acid molecule and a reagent sufficient to amplify and label many kinds of target nucleic acids. For example, if there are two kinds of nucleic acids to be detected, i.e., a first nucleic acid to be detected and a second nucleic acid to be detected, in the sample to be detected, after undergoing micro-partition treatment, each first nucleic acid to be detected and each second nucleic acid to be detected are distributed into different micro liquids, and there is a sufficient number of reagent in one micro liquid, which may not only meet the demand for amplifying and labeling the first nucleic acid to be detected in the micro liquid, but also meet the demand for amplifying and labeling the second nucleic acid to be detected in the micro liquid.

The process of making micro liquids may adopt a method in which the micro liquids are formed through high-frequency vibration: the sample and the sufficient number of reagent are sucked through a microchannel, the microchannel stretches under an oily liquid for high-frequency vibration, and micro liquids of the sample and the reagent in the microchannel are discharged at a certain velocity in the vibration process, so that micro droplets, i.e., the micro liquids are thrown out through vibration, and the micro liquids are incompatible and non-reactive with the oily liquid. By setting a reasonable vibration frequency, vibration amplitude and discharge velocity of the micro liquids, it may be ensured that there is at most only one unit of nucleic acid molecule in each formed micro liquid.

It is also possible to provide a plurality of micro-partition regions in the container, so that by adding the micro liquids of the sample and the reagent into the plurality of micro-partition regions, there is at most one unit of nucleic acid molecule in each micro-partition region.

### IV. Reaction liquid containing part

As shown in Fig. 14A, the reaction liquid containing part is a box-like container with an upward opening. Nucleic acids to be detected and a reagent are added into the box-like container through a liquid filling mechanism (i.e., the method in which micro liquids are formed through high-frequency vibration as described above), and the nucleic acids to be detected and the reagent may also be added into the box-like container by a laboratory worker.

As shown in Fig. 14B, the reaction liquid containing part is a cavity communicating with a microchannel, and the nucleic acids to be detected and the reagent are driven into the cavity by a microfluid driving structure.

In order to realize absolute quantitative detection of nucleic acids to be detected, micro liquids containing the nucleic acids to be detected and the reagent are encapsulated in about 20,000 micro liquids, each of which contains one or no nucleic acid molecules to be detected. Regardless of the number of classes of components to be detected in the nucleic acids to be detected, all the components are distributed into different micro liquids (it is an ideal state, and it is possible that a small number of micro liquids each contain one or more nucleic acid molecules to be detected).

In the examples, illustration is made with the reaction liquid containing part being the box-like container. An oily liquid is injected into the reaction liquid containing part, and the oily liquid is incompatible and non-reactive with the sample and the reagent. The micro liquids are formed in the oily liquid in a mode of high-frequency vibration, and flatly laid at the bottom of the reaction liquid containing part under the force of their own gravities.

As shown in Fig. 14C, a well plate 100 is composed of a plurality of reaction liquid containing parts 1, the bottoms of which are each in a flat plate shape, so that the plurality of micro liquids are flatly laid at the bottoms of the reaction liquid containing parts 1.

### V Temperature adjustment part

As shown in Figs. 14A and 14B, the temperature adjustment part 2 is provided below, above or to the side of the reaction liquid containing part 1, which may provide heat to the reaction liquid containing part 1 and may also absorb heat from the reaction liquid containing part 1, thereby realizing the functions of heating up and cooling down of the micro liquids in the reaction liquid containing part 1. The temperature adjustment part 2 may vary the temperature of the micro liquids in the reaction liquid containing part 1 within a range of 4°C to 105°C. A shown in Fig. 14A, when the reaction liquid containing part 1 is the box-like container with an upward opening, a placement location special for placing the reaction liquid containing part may be provided on the temperature adjustment part, and the reaction liquid containing part 1 is placed on the placement location when the temperature of the reaction liquid containing part 1 needs to be adjusted. As shown in Fig. 14B, when the reaction liquid containing part 1 is the cavity communicating with the microchannel, the temperature adjustment part 2 may be provided in a reasonable orientation of the reaction liquid containing part 1 in combination with factors such as heating efficiency.

In the examples, the temperature adjustment part 2 is provided below the well plate 100 composed of the plurality of reaction liquid containing parts 1, and contacts to heat and cool the flat well plate 100, so that the temperature of the micro liquid in each reaction liquid containing part 1 in the well plate 100 changes with the temperature rise and fall of the well plate 100.

### VI. Signal detection part

As shown in the figures, the signal detection part 3 is provided in the vicinity of of the reaction liquid containing part 1. Depending on signals generated from the micro liquids in the reaction liquid containing part 1, the signal detection part may be a device (a camera or a photoelectric device) for detecting optical signals (e.g., a fluorescence signal) or a device capable of detecting electric signals. For example, when the signal detection part is the camera, the signals flatly laid in the reaction liquid containing part may be photographed. The signal detection part 3 is movable relative to the reaction liquid containing part 1, and when it is required to detect signals, a controller controls a driving mechanism to move the signal detection part 3 to the position close to the reaction liquid containing part 1 (e.g. , above, as shown in Fig. 14A). As shown in Fig. 14B, the signal detection part 3 may also be fixedly provided relative to the reaction liquid containing part.

### VII. Control part

As shown in the figures, the control part 4 is communicably connected to the temperature adjustment part 2 and the signal detection part 3. The control part may include a first control part and a second control part (not shown in the figures). The first control part is communicably connected to the temperature adjustment part to control the temperature adjustment part to run in different modes, and the second control part is communicably connected to the signal detection part to control the signal detection part to acquire the signals from the micro liquids in the reaction liquid containing part. As shown in Figs. 14A and 14B, the control part 4 may also be a single part, which may simultaneously control the temperature adjustment part and the signal detection part.

### Example 13

A sample to be detected contains a first component to be detected (a first nucleic acid to be detected) and a second component to be detected (a second nucleic acid to be detected). It should be noted that a first associated product to be detected generated from the first nucleic acid to be detected (the nucleic acid to be detected undergoes operations such as polymerase chain reaction (PCR) and/or nucleic acid hybridization and/or nucleic acid enzyme digestion to generate the corresponding associated product to be detected, and under normal conditions, the associated product to be detected is a nucleic acid) may bind to a labeling substance in a reagent within a first binding temperature range; and a second associated product to be detected generated from the second nucleic acid to be detected may bind to the labeling substance in the reagent within a second binding temperature range. The first binding temperature range and the second binding temperature range have different maximum temperatures. In the example, the maximum temperature of the second binding temperature range is lower than that of the first binding temperature range. For example, the first associated product to be detected can bind to the labeling substance at a temperature lower than or equal to a temperature A1, and the second associated product to be detected can bind to the labeling substance at a temperature lower than or equal to a temperature A2, wherein the temperature A2 is lower than the temperature A1. The first associated product to be detected may be separated from the labeling substance in the reagent within a first separation temperature range, and the second associated product to be detected may be separated from the labeling substance in the reagent within a second separation temperature range. The first separation temperature range and the second separation temperature range have different minimum temperatures. In the example, the minimum temperature of the second separation temperature range is higher than that of the first separation temperature range. For example, the first associated product to be detected can be separated from the labeling substance at a temperature higher than or equal to a temperature B 1, and the second associated product to be detected can be separated from the labeling substance at a temperature higher than or equal to a temperature B2, wherein the temperature B2 is higher than the temperature B1.

In order to realize multiple detection of different nucleic acids to be detected in the sample, and prevent mutual interference of signals between the different nucleic acids to be detected, in the example, a mixed liquid of the sample and the reagent (containing an amplification substance and the labeling substance) is added to the reaction liquid containing part to form a plurality of micro liquids, and a controller performs the following controls.

As shown in Figs. 15A to 15D, a horizontal axis represents time, and a vertical axis represents a temperature value. The controller controls the temperature adjustment part to adjust the temperature of the micro liquids in the reaction liquid containing part, so that the nucleic acids to be detected in the sample generate associated products to be detected associated therewith (the nucleic acids to be detected contain the first nucleic acid to be detected and the second nucleic acid to be detected, but are presented in different micro liquids); and in this process, the micro liquids may undergo a plurality of temperature cycles, in each of which the micro liquids will change to have a plurality of different temperatures and maintain at the temperatures for a certain time, and there may be a plurality of cycles, e.g., 20-60, e.g., about 40. In each cycle, the micro liquids will reach a high-temperature state (temperature t5), double-stranded nucleic acids become single-stranded nucleic acids, then the micro liquids reach a low-temperature state (temperature T1), and the single-stranded nucleic acids undergo annealing and extending processes (annealing and extending may also be performed at different temperatures respectively, and the extending temperature is greater than the annealing temperature). After the plurality of temperature cycles, the numbers of the different nucleic acids to be detected and/or the different associated products to be detected in the sample are increased (i.e., the quantities of the nucleic acids to be detected and/or the associated products to be detected in each micro liquid are increased, but the types of the nucleic acids to be detected remain unchanged), which is easier to detect. By reasonably setting the annealing temperature T1 in the amplification process, the nucleic acids to be detected and/or the associated products to be detected are increased in number, but do not bind to the labeling substance in the reagent. The annealing temperature T1 (low temperature T1) may take a value within a first range, but cannot coincide with a subsequent desirable range (second range) of T2, and all values within the first range are greater than values within the second range.

As shown in Figs. 15A to 15D, before performing 40 temperature cycles, the micro liquids may be heated to a temperature t0, so that the nucleic acids to be detected in the sample are initially denaturated for a period of time (about 2 min). Initial denaturation for a period of time was to change the nucleic acids from a double-stranded state to a single-stranded state.

After the completion of 40 cycles, the controller may control the temperature adjustment part to adjust the temperature of the micro liquids in the reaction liquid containing part to a temperature t1 and maintain the temperature for a certain time. As shown in Figs. 15A to 15D, this interval period is interval 1, the controller may control a signal detection part to perform one detection action, and a signal detected at this time serves as a blank signal. The temperature t1 may be higher or lower than the annealing temperature T1; after the completion of cycles, the temperature adjustment part may also maintain the temperature of the micro liquids in a state of temperature T1, and the controller controls the signal detection part to perform one detection action. Since the signal detected obtained from this detection is the blank signal, this detection action may also be omitted in order to improve efficiency.

Then, the controller controls the temperature adjustment part to adjust the temperature of the micro liquids in the reaction liquid containing part in a first temperature adjustment mode. After the adjustment in the first temperature adjustment mode, the first associated product to be detected capable of binding to the labeling substance is generated in the micro liquids, and the labeling substance binding to the first associated product to be detected generates a signal. In this state, the controller controls the signal detection part to perform signal detection on the reaction liquid containing part to obtain a first signal. Since a single nucleic acid molecule to be detected is distributed into one micro liquid, that is, one micro liquid only contains one nucleic acid molecule to be detected, the nucleic acid molecule to be detected may be the first nucleic acid to be detected and may also be the second nucleic acid to be detected, after the adjustment in the first temperature adjustment mode, the micro liquids only containing the first nucleic acid to be detected can generate signals, and if a plurality of micro liquids overlap, signals emitted from such micro liquids are invalid signals; and signals emitted from single micro liquids that do not overlap are valid signals, and such micro liquids serve as a first part of micro liquids, that is, among the plurality of micro liquids, only the first part of micro liquids generates the valid signals. In the micro liquids, the amplification substance for amplifying the first nucleic acid to be detected and the labeling substance for labeling the first associated product to be detected serve as a first reagent.

Specifically, the first temperature adjustment mode includes an incubation stage and a signal acquisition stage. At the incubation stage, specific classes of associated products to be detected capable of binding to the labeling substance may be generated, at the signal acquisition stage, certain classes of associated products to be detected in the specific classes of associated products to be detected do not bind to the labeling substance, and one remaining associated product to be detected remains bound to the labeling substance to complete signal detection by the signal detection part to the reaction liquid containing part. If only one kind of associated product to be detected binds to the labeling substance after incubation at the incubation stage of the first temperature adjustment mode, the binding state of the associated product to be detected to the labeling substance is maintained at the signal acquisition stage.

At the incubation stage, in order to realize binding of the first associated product to be detected to the labeling substance, temperature adjustment part periodically changes the temperature of the micro liquids in the reaction liquid containing part, so that the temperature of the reaction liquid containing part is repeatedly reduced from a high temperature to a low temperature T2, or the temperature adjustment part maintains the reaction liquid containing part at the constant temperature T2, which is lower than the annealing temperature T1. When the temperature of the micro liquids in the reaction liquid containing part is T2, the first associated product to be detected in the first part of micro liquids binds to the first reagent to generate a signal. Temperature T2 may take a value within the second range, but cannot coincide with a subsequent desirable range (third range) of T3. All values within the second range are greater than values within the third range, and all the values within the second range are less than the values within the first range.

In order to increase the binding rate of the nucleic acids to be detected and the labeling substance, as shown in Figs. 15A and 15C, at the incubation stage of the first temperature adjustment mode, the control part controls the temperature adjustment part to periodically change the temperature of the micro liquids in the reaction liquid containing part. In each temperature cycle, the first associated product to be detected generated by the first nucleic acid to be detected binds to the labeling substance at T2, and is molten from the labeling substance at a high temperature t5. After experiencing a plurality of repeated cycles, the number of first associated products to be detected binding to the labeling substance reaches an ideal maximum, and the number of cycles is about 15, e.g., ten. In the last cycle, in the binding state of the first associated product to be detected to the labeling substance, the controller controls the signal detection part to perform signal detection on the micro liquids to obtain the first signal. Certainly, as shown in Figs. 15A and 15C, after the completion of the last cycle (i.e., at the signal acquisition stage), the controller may also control the temperature adjustment part to adjust the temperature of the micro liquids in the reaction liquid containing part to a temperature t2 (the temperature t2 does not reach the temperature at which the first associated product to be detected is separated/molten from the labeling substance), so that the first associated product to be detected and the labeling substance are maintained in the binding state for a certain time. This time period is interval 2, and in interval 2, the controller controls the signal detection part to perform signal detection on the reaction liquid containing part to obtain the first signal. The first signal contains the valid signals generated from the first part of micro liquids, and, if there are overlapping micro liquids, further includes the invalid signals generated from the overlapping micro liquids, which will be excluded in the subsequent analysis process.

Alternatively, as shown in Figs. 15B and 15D, at the incubation stage of the first temperature adjustment mode, the control part controls the temperature adjustment part to adjust the temperature of the micro liquids in the reaction liquid containing part to be maintained at the temperature T2 for a certain time, which may be 10-1,800 s, to incubate the micro liquids. At the end of the incubation time, the signal detection part is controlled to perform signal detection on the reaction liquid containing part to obtain the first signal. Certainly, as shown in Figs. 16B and 16D, after the completion of constant-temperature incubation (signal acquisition stage), the control part may also controls the temperature adjustment part to adjust the temperature of the micro liquids in the reaction liquid containing part to the temperature t2, so that the first nucleic acid to be detected and the labeling substance are maintained in the binding state for a certain time. This time period is interval 2, and in interval 2, the controller controls the signal detection part to perform signal detection on the reaction liquid containing part to obtain the first signal.

Then, the controller controls the temperature adjustment part to adjust the temperature of the micro liquids in the reaction liquid containing part in the second temperature adjustment mode, after the adjustment in the second temperature adjustment mode, only the second associated product to be detected in a pre-amplification product binds to the labeling substance in the reagent, and the labeling substance binding to the second associated product to be detected generates a signal. In this state, the controller controls the signal detection part to perform signal detection on the reaction liquid containing part to obtain a second signal. Similarly, since a single nucleic acid molecule is distributed into one micro liquid, that is, one micro liquid only contains one nucleic acid molecule, the nucleic acid molecule may be the first nucleic acid to be detected and may also be the second nucleic acid to be detected, after the adjustment in the second temperature adjustment mode, only the micro liquids in which the second nucleic acid to be detected is located can generate signals, and if a plurality of micro liquids overlap, signals emitted from such micro liquids are invalid signals; and signals emitted from single micro liquids that do not overlap are valid signals, and such micro liquids serve as a second part of micro liquids, that is, among the plurality of micro liquids, only the second part of micro liquids generates the valid signals. In the micro liquids, the amplification substance for amplifying the second nucleic acid to be detected and the labeling substance for labeling the nucleic acid to be detected serve as a second reagent.

Specifically, the second temperature adjustment mode includes an incubation stage and a signal acquisition stage. At the incubation stage, specific classes of associated products to be detected are achieved to bind to the labeling substance, at the signal acquisition stage, certain classes of associated products to be detected in the specific classes of associated products to be detected are achieved to be separated from the marking substance, and one remaining associated product to be detected remains a binding state to the labeling substance to complete signal detection by the signal detection part to the reaction liquid containing part.

At the incubation stage, in order to realize binding of the second associated product to be detected to the labeling substance, the temperature adjustment part periodically changes the temperature of the micro liquids in the reaction liquid containing part, so that the temperature of the reaction liquid containing part is repeatedly reduced from a high temperature to a low temperature T3, or the temperature adjustment part maintains the reaction liquid containing part at the constant temperature T3, which is lower than the temperature T2. When the temperature of the micro liquids in the reaction liquid containing part is T3, the second associated product to be detected in the second part of micro liquids binds to the second reagent to generate a signal, and in the meantime, the first associated product to be detected in the micro liquids containing the first associated product to be detected can also bind to the first reagent to generate a signal. The temperature T3 may take a value within the third range, but cannot coincide with a subsequent desirable range (fourth range) of T4. All the values within the third range are greater than values within the fourth range, and all the values within the third range are less than the values within the second range.

In order to increase the binding rate of the associated products to be detected and the labeling substance, as shown in Figs. 15A and 15C, at the incubation stage of the second temperature adjustment mode, the control part controls the temperature adjustment part to periodically change the temperature of the micro liquids in the reaction liquid containing part. In each temperature cycle, the second associated product to be detected generated by the second nucleic acid to be detected binds to the labeling substance at T3, and is molten from the labeling substance at the high temperature t5. After experiencing a plurality of repeated cycles, the number of second associated products to be detected binding to the labeling substance reaches an ideal maximum, and the number of cycles is about 15, e.g., 10. After the completion of the last cycle (i.e., at the signal acquisition stage), the control part controls the temperature adjustment part to adjust the temperature of the micro liquids in the reaction liquid containing part to a temperature t3 (the temperature t3 reaches a temperature at which the first associated product to be detected is separated/molten from the labeling substance, and does not reach a temperature at which the second associated product to be detected is separated/molten from the labeling substance), so that the first associated product to be detected is separated/molten from the labeling substance (the first associated product to be detected in the first part of micro liquids is separated/molten from the labeling substance, and a signal generated due to binding disappears), and the second associated product to be detected and the labeling substance are maintained in the binding state for a certain time. This time period is interval 3, and in interval 3, the controller controls the signal detection part to perform signal detection on the reaction liquid containing part to obtain the second signal. The second signal contains the valid signals generated from the second part of micro liquids, and, if there are overlapping micro liquids, further includes the invalid signals generated from the overlapping micro liquids, which will be excluded in the subsequent analysis process.

Since all the values within the third range are less than the values within the second range, when the temperature reaches the third temperature range, the first associated product to be detected will also bind to the labeling substance. Therefore, in each temperature cycle, both the first associated product to be detected and the second associated product to be detected will undergo the process of binding to and being separated from the labeling substance. After the last cycle, before the temperature reaches t3, or exceeds t3 but does not reaches t4 (as described below, the temperature t4 reaches a temperature at which the first associated product to be detected and the second associated product to be detected can be separated/molten from the labeling substance, but does not reach a temperature at which the third associated product to be detected can be separated/molten from the labeling substance), the first associated product to be detected is molten from the labeling substance, at which time, only the second associated product to be detected binds to the labeling substance, and in this state, the signal detection part is controlled to perform signal detection on the reaction liquid containing part to obtain the second signal.

Alternatively, as shown in Figs. 15B and 15D, at the incubation stage of the second temperature adjustment mode, the control part controls the temperature adjustment part to adjust the temperature of the micro liquids in the reaction liquid containing part to be maintained at the temperature T3 for a certain time, which may be 10-1,800 s, to incubate the micro liquids. At the end of the incubation time, the signal detection part is controlled to perform signal detection on the reaction liquid containing part to obtain the second signal. At the temperature T3, both the first associated product to be detected and the second associated product to be detected will bind to the labeling substance. After the completion of incubation (i.e., a signal detection stage), the control part controls the temperature adjustment part to adjust the temperature of the micro liquids in the reaction liquid containing part to the temperature t3 and maintain the temperature for a certain time, and at the temperature t3, the first associated product to be detected is molten/separated from the labeling substance; and as shown in Figs. 15B and 15D, the time period is interval 3, and in interval 3, the first associated product to be detected and the labeling substance are separated/molten and maintained in a separated/molten state, the second associated product to be detected still binds to the labeling substance, and in this state (interval 3), the controller controls the signal detection part to perform signal detection on the reaction liquid containing part to obtain the second signal.

Through the above controls of the controller, when the signal detection part achieves to detect signals generated from the micro liquids, only one kind of nucleic acid to be detected or an associated product thereof binds to the labeling substance, so that signals generated from micro liquids of two kinds of nucleic acids to be detected are completely separated, and high-precision duplex nucleic acid detection is realized.

### Example 14

The example differs from Example 13 in that the nucleic acids in the sample bind to the labeling substance in the reagent while being amplified, which may improve the detection efficiency and increase the detection flux.

Details are as follows.

As shown in Figs. 16A to 16, the controller controls the temperature adjustment part to adjust the temperature of the micro liquids in the reaction liquid containing part in the first temperature adjustment mode, so that the nucleic acids in the micro liquids are amplified by the amplification substance in the reagent to achieve an increase in number, and at the same time, products thereof may bind to the labeling substance in the reagent. In the process, the micro liquids will undergo a plurality of temperature cycles, and in each temperature cycle, the micro liquids will change into a plurality of different temperatures and maintain at the temperature for a certain time. The number of cycles is about 40. The annealing temperature in the amplification process is the temperature T2. Since the annealing temperature reaches a temperature at which the first associated product to be detected (e.g., an amplification product of the first nucleic acid to be detected) can bind to the labeling substance, amplification of the nucleic acid to be detected and binding of the amplification product thereof to the labeling substance may be simultaneously realized under the adjustment in the first temperature adjustment mode. After the completion of amplification, the first part of micro liquids containing the first nucleic acid to be detected generates a signal, and at this time, the controller controls the signal detection part to perform signal detection on the reaction liquid containing part to obtain the first signal. Specifically, after 40 cycles in the first temperature adjustment mode, the controller controls the temperature adjustment part to adjust the temperature of the micro liquids in the reaction liquid containing part to the temperature t2, so that the first associated product to be detected and the labeling substance are maintained in the binding state for a certain time. This time period is interval 2, and in interval 2, the controller controls the signal detection part to perform signal detection on the reaction liquid containing part to obtain the first signal. Certainly, after the completion of cycles, the temperature of the micro liquids in the reaction liquid containing part may also be maintained at T2, and the controller controls the signal detection part to perform signal detection on the reaction liquid containing part to obtain the first signal.

Then, the controller controls the temperature adjustment part to adjust the temperature of the micro liquids in the reaction liquid containing part in the second temperature adjustment mode, which is the same as in Example 13 and will no longer be described in detail.

### Example 15

The embodiment differs from Example 13 or Example 14 in that:
First, the sample further includes a third component to be detected (a third nucleic acid to be detected). It should be noted that the third nucleic acid to be detected or a third associated product to be detected (the third nucleic acid to be detected undergoes operations such as polymerase chain reaction (PCR) and/or nucleic acid hybridization and/or nucleic acid enzyme digestion to generate the corresponding third associated product to be detected, and under normal conditions, the third associated product to be detected is a nucleic acid) may bind to the labeling substance in the reagent within a certain temperature range. In the example, the maximum temperature at which the third nucleic acid to be detected or the third associated product to be detected binds to the labeling substance is lower than the maximum temperature at which the second nucleic acid to be detected or the second associated product to be detected binds to the labeling substance. For example, the third nucleic acid to be detected or the third associated product to be detected can bind to the labeling substance at a temperature lower than or equal to a temperature A3, which is lower than the temperature A2 (the second nucleic acid to be detected or the second associated product to be detected can bind to the labeling substance at a temperature lower than or equal to the temperature A2). The third nucleic acid to be detected or the third associated product to be detected may be separated from the labeling substance in the reagent within a certain temperature range. In the example, the minimum temperature at which the third nucleic acid to be detected or the third associated product to be detected is separated from the labeling substance is higher than the minimum temperature at which the second nucleic acid to be detected or the second associated product to be detected is separated from the labeling substance. For example, the third nucleic acid to be detected or the third associated product to be detected can be separated from the labeling substance at a temperature higher than or equal to a temperature B3, which is higher than the temperature B2 (the second nucleic acid to be detected or the second associated product to be detected can be separated from the labeling substance at a temperature higher than or equal to the temperature B2).

Second, after controlling the temperature adjustment part to adjust the temperature of the micro liquids in the reaction liquid containing part and controlling the signal detection part to obtain the second signal, the controller further performs the following controls.

The controller controls the temperature adjustment part to adjust the temperature of the micro liquids in the reaction liquid containing part in a third temperature adjustment mode. After the adjustment in the third temperature adjustment mode, only the third nucleic acid to be detected or the third associated product to be detected in the product binds to the labeling substance in the reagent, and the labeling substance generates a signal due to binding. In this state, the controller controls the signal detection part to perform signal detection on the reaction liquid containing part to obtain a third signal. Similarly, since a single nucleic acid molecule is distributed into one micro liquid, that is, one micro liquid only contains one nucleic acid molecule, the nucleic acid molecule may be the first nucleic acid to be detected, the second nucleic acid to be detected, or the third nucleic acid to be detected, after the adjustment in the third temperature adjustment mode, only the micro liquids in which the third nucleic acid to be detected is located can generate signals, and if a plurality of micro liquids overlap, signals emitted from such micro liquids are invalid signals; and signals emitted from single micro liquids that do not overlap are valid signals, and such micro liquids serve as a third part of micro liquids, that is, among the plurality of micro liquids, only the third part of micro liquids generates the valid signals. In the micro liquids, the amplification substance for amplifying the third nucleic acid to be detected and the labeling substance for labeling the third nucleic acid to be detected serve as a third reagent.

Specifically, the third temperature adjustment mode includes an incubation stage and a signal acquisition stage. At the incubation stage, specific classes of nucleic acids to be detected or corresponding associated products to be detected achieve to bind to the labeling substance, at the signal acquisition stage, certain classes of nucleic acids to be detected or corresponding associated products to be detected in the specific classes of nucleic acids to be detected or corresponding associated products to be detected are achieved to be separated from the labeling substance, and one remaining nucleic acid to be detected or a corresponding associated product to be detected remains bound to the labeling substance to complete signal detection by the signal detection part to the reaction liquid containing part.

At the incubation stage, in order to realize binding of the third nucleic acid to be detected or the corresponding third associated product to be detected to the labeling substance, the temperature adjustment part periodically changes the temperature of the micro liquids in the reaction liquid containing part, so that the temperature of the reaction liquid containing part is repeatedly reduced from a high temperature to a low temperature T4, or the temperature adjustment part maintains the reaction liquid containing part at the constant temperature T4, which is lower than the temperature T3. When the temperature of the micro liquids in the reaction liquid containing part is T4, the third nucleic acid to be detected or the third associated product to be detected in the micro liquids containing the third nucleic acid to be detected binds to the third reagent to generate a signal. The temperature T4 may take a value within the fourth range, but cannot coincide with a subsequent desirable range (fifth range) of T5 (if there is a fourth temperature adjustment mode). All the values within the fourth range are greater than values within the fifth range, and all the values within the fourth range are less than the values within the third range.

In order to increase the binding rate of the nucleic acids to be detected and the labeling substance, as shown in Figs. 15C and 16C, at the incubation stage of the third temperature adjustment mode, the control part controls the temperature adjustment part to periodically change the temperature of the micro liquids in the reaction liquid containing part. In each temperature cycle, the third nucleic acid to be detected or the third associated product to be detected binds to the labeling substance at the low temperature T4, and is molten/separated from the labeling substance at a high temperature t5. After experiencing a plurality of repeated cycles, the number of third nucleic acids to be detected or third associated products to be detected binding to the labeling substance reaches an ideal maximum, and the number of cycles is about 15 (e.g., nine cycles, ten cycles, etc.). After the completion of the last cycle (i.e., at the signal acquisition stage), the control part controls the temperature adjustment part to adjust the temperature of the micro liquids in the reaction liquid containing part to a temperature t4 (the temperature t4 reaches a temperature at which the first nucleic acid to be detected (or the first associated product to be detected) and the second nucleic acid to be detected (or the second associated product to be detected) are separated/molten from the labeling substance, and does not reach a temperature at which the third nucleic acid to be detected/the third associated product to be detected is separated/molten from the labeling substance), so that the first nucleic acid to be detected (or the first associated product to be detected) is separated/molten from the labeling substance (in the micro liquids containing the first nucleic acid to be detected, the first nucleic acid to be detected/the first associated product to be detected is separated/molten from the labeling substance, and a signal generated due to binding disappears), the second nucleic acid to be detected/the second associated product to be detected is separated/molten from the labeling substance (in the micro liquids containing the second nucleic acid to be detected, the second nucleic acid to be detected/the second associated product to be detected is separated/molten from the labeling substance, and a signal generated due to binding disappears), and the third nucleic acid to be detected/the third associated product to be detected and the labeling substance are maintained in a binding state for a certain time. This time period is interval 4, and in interval 4, the controller controls the signal detection part to perform signal detection on the reaction liquid containing part to obtain the third signal. The third signal contains the valid signals generated from the third part of micro liquids, and, if there are overlapping micro liquids, further includes the invalid signals generated from the overlapping micro liquids, which will be excluded in the subsequent analysis process.

Since all the values within the fourth range are less than the values within the third range, when the temperature reaches the fourth range, the first nucleic acid to be detected (or the first associated product to be detected) and the second nucleic acid to be detected (or the second associated product to be detected) will also bind to the labeling substance. Therefore, in each temperature cycle, all of the first nucleic acid to be detected (or the first associated product to be detected), the second nucleic acid to be detected (or the second associated product to be detected) and the third nucleic acid to be detected (or the third associated product to be detected) will undergo the process of binding to or being separated from the labeling substance. After the last cycle, before the temperature reaches t4, or exceeds t4 but does not reaches t5 (the temperature t4 reaches a temperature at which the first nucleic acid to be detected (or the first associated product to be detected) and the second nucleic acid to be detected (or the second associated product to be detected) can be separated/molten from the labeling substance, but does not reach a temperature at which the third nucleic acid to be detected (or the third associated product to be detected) can be separated/molten from the labeling substance), both the first nucleic acid to be detected (or the first associated product to be detected) and the second nucleic acid to be detected (or the associated product to be detected) are molten/separated from the labeling substance, at which time, only the third nucleic acid to be detected (or the third associated product to be detected) remains bound to the labeling substance, and in this state, the signal detection part is controlled to perform signal detection on the reaction liquid containing part to obtain the third signal.

Alternatively, as shown in Fig. 15D, at the incubation stage of the second temperature adjustment mode, the control part controls the temperature adjustment part to adjust the temperature of the micro liquids in the reaction liquid containing part to be maintained at the temperature T4 for a certain time, which may be 10-1,000 s, to incubate the micro liquids. At the end of the incubation time, the signal detection part is controlled to perform signal detection on the reaction liquid containing part to obtain the third signal. At the temperature T4, all the first nucleic acid to be detected (or the first associated product to be detected), the second nucleic acid to be detected (or the second associated product to be detected), and the third nucleic acid to be detected (or the third associated product to be detected) will bind to the labeling substance. After the completion of incubation (i.e., a signal detection stage), the control part controls the temperature adjustment part to adjust the temperature of the micro liquids in the reaction liquid containing part to the temperature t4 and maintain the temperature for a certain time. As shown in Figs. 15D and 16D, the time period is interval 4, and in interval 4, both the first nucleic acid to be detected (or the first associated product to be detected) and the second nucleic acid to be detected (or the second associated product to be detected) are separated/molten from the labeling substance and maintained in a separated/molten state, the third nucleic acid to be detected (or the associated product to be detected) still binds to the labeling substance, and in this state (interval 4), the controller controls the signal detection part to perform signal detection on the reaction liquid containing part to obtain the third signal.

Through the above controls of the controller, when the signal detection part achieves to detect signals generated from the micro liquids, only one kind of nucleic acid to be detected or an associated product thereof may bind to the labeling substance, so that signals generated from micro liquids of three kinds of nucleic acids to be detected are completely separated, and high-precision triple nucleic acid detection is realized.

### Example 16

The example differs from Example 13 in that due to the difference of components contained in the sample, the signal obtained in interval 1 shown in Figs. 15A to 15D is not a blank signal, but a signal generated from micro liquids containing a certain component to be detected. In the example, the component to be detected is a fourth component to be detected (a fourth nucleic acid to be detected).

Based on Example 13, the sample further includes the fourth component to be detected (the fourth nucleic acid to be detected). The fourth nucleic acid to be detected can be amplified by the amplification substance in the reagent, a resulting amplification product (a fourth associated product to be detected) can bind to the labeling substance in the reagent, and the labeling substance binding to the amplification product of the fourth nucleic acid to be detected may generate a detectable signal. It should be noted that amplification of the fourth nucleic acid and binding of the fourth nucleic acid to be detected to the labeling substance may be realized in a certain temperature range. Within the temperature range, other nucleic acids to be detected, e.g., the first nucleic acid to be detected and the second nucleic acid to be detected, can be amplified by the amplification substance in the reagent, but products thereof cannot bind to the labeling substance in the reagent. For example, the fourth associated product to be detected can bind to the labeling substance at a temperature lower than or equal to the temperature A4, which is higher than the temperature A1 (the first nucleic acid to be detected or the associated product thereof can bind to the labeling substance at a temperature lower than or equal to the temperature A1). The fourth nucleic acid to be detected or the associated product thereof can be separated/molten from the labeling substance within a certain temperature range, and in the example, the minimum temperature at which the fourth nucleic acid to be detected or the associated product thereof is separated from the labeling substance is lower than the minimum temperature at which the first nucleic acid to be detected or the associated product thereof is separated from the labeling substance. For example, the fourth nucleic acid to be detected or the associated product thereof can be separated from the labeling substance at a temperature higher than or equal to a temperature B4, which is lower than the temperature B1.

At the annealing temperature T1, other nucleic acids to be detected in the sample, e.g., the first nucleic acid to be detected and the second nucleic acid to be detected, are only amplified in the process and do not bind to the labeling substance in the reagent; and the fourth nucleic acid to be detected can be amplified and can bind to the labeling substance. At the temperature t1, the fourth nucleic acid to be detected or the associated product thereof are maintained in a state of binding to the labeling substance, and the signal detected by the signal detection part is no longer a blank signal, but a signal generated from micro liquids containing the fourth nucleic acid to be detected. The temperature t1 may be higher or lower than or equal to the annealing temperature T1.

The subsequent control process differs from Example 13 in that:
in the process of the controller controlling the temperature adjustment part to adjust the temperature of the reaction liquid containing part in the first temperature adjustment mode, in the case that the control part controls the temperature adjustment part to adjust the temperature of the micro liquids in the reaction liquid containing part to the temperature T2, the fourth nucleic acid to be detected or the associated product thereof binds to the labeling substance, and the first nucleic acid to be detected or the associated product thereof binds to the labeling substance; and in the case that the control part controls the temperature adjustment part to adjust the temperature of the micro liquids in the reaction liquid containing part to the temperature t2, the fourth nucleic acid to be detected or the associated product thereof is separated/molten from the labeling substance, and the first nucleic acid to be detected or the associated product thereof remains bound to the labeling substance.

In the process of the controller controlling the temperature adjustment part to adjust the temperature of the reaction liquid containing part in the second temperature adjustment mode, in the case that the control part controls the temperature adjustment part to adjust the temperature of the micro liquids in the reaction liquid containing part to the temperature T3, the fourth nucleic acid to be detected or the associated product thereof binds to the labeling substance, the first nucleic acid to be detected or the associated product thereof binds to the labeling substance, and the second nucleic acid to be detected or the associated product thereof binds to the labeling substance; and in the case that the control part controls the temperature adjustment part to adjust the temperature of the micro liquids in the reaction liquid containing part to the temperature t3, both the fourth nucleic acid to be detected or the associated product thereof and the first nucleic acid to be detected or the associated product thereof are separated/molten from the labeling substance, and the second nucleic acid to be detected or the associated product thereof is maintained in a state of binding to the labeling substance.

The rest of the control process is the same as in Example 13 and will not be described herein in detail.

The temperatures t1, t2, t3, t4 and t5 in the above Examples 13 to 16 may be understood as follows.

At the temperature t1, if the sample contains the fourth nucleic acid to be detected, the fourth nucleic acid or the associated product thereof can be maintained in a state of binding to the labeling substance in the reagent; and if the sample does not contain the fourth nucleic acid to be detected, there is no nucleic acid to be detected or associated product thereof binding to the labeling substance in the reagent.

At the temperature t2, only the first nucleic acid to be detected or the associated product thereof can be maintained in a state of binding to the labeling substance in the reagent.

At the temperature t3, only the second nucleic acid to be detected or the associated product thereof can be maintained in a state of binding to the labeling substance in the reagent.

At the temperature t4, only the third nucleic acid to be detected or the associated product thereof can be maintained in a state of binding to the labeling substance in the reagent.

At the temperature t5, all the nucleic acids to be detected or associated products thereof are molten.

The fourth reagent, the first reagent, the second reagent and the third reagent in the above Examples 13 to 16 may be understood as follows.

The fourth reagent includes an amplification substance capable of amplifying the fourth nucleic acid to be detected and a labeling substance capable of labeling the fourth nucleic acid to be detected.

The first reagent includes an amplification substance capable of amplifying the first nucleic acid to be detected and a labeling substance capable of labeling the first nucleic acid to be detected.

The second reagent includes an amplification substance capable of amplifying the second nucleic acid to be detected and a labeling substance capable of labeling the second nucleic acid to be detected.

The third reagent includes an amplification substance capable of amplifying the third nucleic acid to be detected and a labeling substance capable of labeling the third nucleic acid to be detected.

Wherein, primers in the amplification substances for amplifying the fourth nucleic acid to be detected, the first nucleic acid to be detected, the second nucleic acid to be detected and the third nucleic acid to be detected are different, and other components therein may be the same and may also be different; and the labeling substances for labeling the fourth nucleic acid to be detected, the first nucleic acid to be detected, the second nucleic acid to be detected and the third nucleic acid to be detected may be the same and may also be different.

In an initial state, i.e., before incubation of the micro liquids, one micro liquid contains at most one nucleic acid to be detected (the fourth nucleic acid to be detected, the first nucleic acid to be detected, the second nucleic acid to be detected, or the third nucleic acid to be detected) and a sufficient quantity of reagents capable of amplifying and labeling all the nucleic acids to be detected.

Certainly, a person skilled in the art may, according to the above examples, reasonably infer how to use the technical solutions of the present application to achieve high-precision quadruple nucleic acid detection, quintuple nucleic acid detection, sextuple nucleic acid detection, and the like.

A person of ordinary skill in the art may understand that all or part of the processes in the methods of the above examples may be implemented by instructing relevant hardware through a computer program. The program may be stored in a non-volatile computer-readable storage medium, and when the program is executed, the processes in the methods of the above examples may be included. Wherein, the storage medium may be a magnetic disk, an optical disk, an ROM, etc.

Any reference to a memory, a storage, a database, or other mediums as used herein may include a non-volatile and/or volatile memory. Suitable non-volatile memory may include an ROM, a Programmable ROM (PROM), an Erasable PROM (EPROM), an Electrically Erasable PROM (EEPROM), or a flash memory. The volatile memory may include a random access memory (RAM), which is used as an external cache memory. As an illustration rather than a limitation, the RAM may have multiple forms, such as a Static RAM (SRAM), a Dynamic Random Access Memory (DRAM), a synchronous DRAM (SDRAM), a Double Data Rate SDRAM (DDR SDRAM), an Enhanced Synchronous DRAM (ESDRAM), a Synchlink DRAM (SLDRAM), a Rambus DRAM (RDRAM) and a Direct Rambus DRAM (DRDRAM).

It should be understood that "one implementation (- 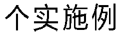 )" or "one embodiment/example (- 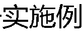 )" mentioned throughout the description means that specific characteristics, structures or features related to the example are included in at least one example of the present application. Therefore, "in one embodiment ( 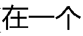 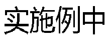 )" or "in one embodiment/example ( 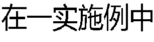 )" appearing throughout the description does not necessarily refer to the same example. In addition, these specific characteristics, structures or features may be combined in one or more examples in any suitable manners. A person skilled in the art should also be aware that all the examples described in the description are optional examples, and the actions and modules involved are not necessarily required by the present application.

In various examples of the present application, it should be understood that the magnitude of the serial number of each of the above processes does not mean an inevitable sequence of the order of execution, and the order of execution of each process should be determined by its function and inherent logic without constituting any limitation on the implementation processes of the examples of the present application.

The functional units in the examples of the present application may be integrated in one processing unit, or each unit may physically exist separately, or two or more units may be integrated in one unit. The above integrated unit may be implemented either in the form of hardware or in the form of a software function unit.

The above integrated unit may be stored in a computer-accessible memory if it is implemented in the form of the software function unit and sold or used as an independent product. Based on such an understanding, the technical solutions of the present application essentially, or the part contributing to the prior art, or all or part of the technical solutions may be presented in the form of a software product. The computer software product is stored in a memory including several requests to enable a computer device (which may be a personal computer, a server, a network device, or the like, specifically, a processor in the computer device) to perform part or all of the steps of the methods described in the examples of the present application.

The above provides a detailed introduction of a multiple time-sharing nucleic acid detection device and detection method according to the examples of the present application. The principles and implementations of the present application are set forth herein with specific examples. The description of the above examples is only for the purpose of assisting in understanding the method and core concept of the present application. In the meantime, for a person of general skill in the art, according to the idea of the present application, there will be changes in the specific implementations and the scope of application. To sum up, the contents of the description should not be understood as a limitation on the present application.

## Claims

1. A method for detecting a target nucleic acid, comprising the following steps:
S100, mixing a primer-probe composition, a sample to be detected and an amplification reagent to obtain a reaction system; the amplification reagent comprising a DNA polymerase and dNTPs;
S200, performing a first PCR amplification on the reaction system, with an annealing temperature for amplification of T1;
S300, performing any one of the following operations:
operation 1, comprising:
S310, performing a first signal acquisition at a first signal acquisition temperature t1;
S320, performing a second PCR amplification, with an annealing temperature for amplification of T2;
operation 2, comprising:
S310', performing a first signal acquisition at a first signal acquisition temperature t1;
operation 3, comprising:
S310", performing a second PCR amplification, with an annealing temperature for amplification of T2;
S320", performing a first signal acquisition at a first signal acquisition temperature;
the above operations satisfying: T2 < T1, and T2 < t1; preferably, 40°C ≤ T2 < T1 ≤ 75°C, and/or 0°C ≤ t1 < t2 ≤ 90°C;
S400, performing a second signal acquisition at a second signal acquisition temperature t2, with t2 > t1, and t2 > T1;
wherein there is one kind of target to be detected in the sample to be detected; and
at most one of the first signal acquisition and the second signal acquisition contains a signal of the target.

2. The method according to claim 1, **characterized in that** reaction conditions for the first PCR amplification in step S200 comprise: initial denaturation at about 85°C to about 105°C for about 0 to about 15 min; denaturation at about 85°C to about 105°C for about 1 to about 60 s, and annealing and extending at about 40°C to about 75°C for about 3 to about 90 s, for 20 to 60 cycles; preferably, when the target in the sample to be detected is RNA, the amplification reagent further comprises a reverse transcriptase, and the reaction conditions for performing the first PCR amplification on the reaction system comprise: reverse transcription at about 30 to about 65°C for about 2 to about 30 min; initial denaturation at about 85°C to about 105°C for about 0 to about 15 min; denaturation at about 85°C to about 105°C for about 1 to about 60 s, and annealing and extending at about 40°C to about 75°C for about 3 to about 90 s, for 20 to 60 cycles;
and/or, the second PCR amplification in step S300 comprises the following reaction conditions: denaturation at about 85°C to about 105°C for about 1 to about 60 s, and annealing and extending at about 20°C to about 75°C for about 3 to about 90 s, for 1 to 20 cycles; alternatively, constant-temperature incubation at about 20°C to about 75°C for about 10 to about 1800 s.

3. The method according to claim 1 or 2, **characterized in that** before performing the first PCR amplification on the reaction system, the method further comprises: distributing the reaction system into 500 or more reaction units, with each reaction unit containing one target nucleic acid of the sample to be detected or containing no target nucleic acids of the sample to be detected; preferably, the signal acquisition refers to an acquisition of a fluorescence signal by means of a camera; and the signal channel acquisition refers to an acquisition of the fluorescence signal by means of the camera under a fluorescence signal channel.

4. The method according to claim 1 or 2, **characterized in that** the primer-probe composition comprises: a probe (P) and a primer; the primer specifically binds to the target in the sample to be detected to generate a pre-product that contains one single-stranded pre-product capable of specifically binding to the probe, the single-stranded pre-product specifically binds to the probe and extends by ≥ 0 base to form a double-stranded product, and formation of the double-stranded product causes a probe signal change; preferably, the single-stranded pre-product specifically binds to the probe and extends by 0-100 bases, more preferably, by 1-100 bases; and/or, the primer has a concentration of about 30 nM to about 1,000 nM, and the probe (P) has a concentration of about 100 nM to about 1,200 nM.

5. The method according to claim 4, **characterized in that** the probe (P), as one freely-designed sequence that is not paired with or binding to any target sequences, comprises a probe signal detection region (H), the probe is modified with detection labels; the primer comprises a forward primer (F) and/or a reverse primer (R);
the forward primer (F) contains a target sequence binding region that is specifically paired with and binding to a target sequence;
the reverse primer (R) contains a primer signal detection region (h) and a target sequence binding region, and the primer signal detection region (h) is located at a 5' end of the target sequence binding region, or the reverse primer (R) sequentially contains an extension block region (M), the primer signal detection region (h) and the target sequence binding region from a 5' end to a 3' end;
wherein the target sequence binding region of the reverse primer (R) is a sequence that is specifically paired with and binding to the target sequence; the primer signal detection region (h) of the reverse primer (R) is one sequence that is not paired with or binding to any target sequences, and a complementary sequence (h') of the primer signal detection region (h) is specifically paired with and binding to the probe signal detection region (H) of the probe (P); the extension block region (M) of the reverse primer (R) is a freely-designed sequence that is not same as or complementary with a sequence of any part of the probe (P) or any target sequences;
preferably, there is a gap of 0-20 bases between the primer signal detection region (h) and the target sequence binding region in the reverse primer (R).

6. The method according to claim 5, **characterized in that** the forward primer (F) further comprises a probe anchoring region (A), and the probe anchoring region (A) in the forward primer (F) is located at a 5' end of the target sequence binding region; wherein the probe anchoring region (A) is one sequence that is not paired with or binding to any target sequences; preferably, there is a gap of 0-20 bases between the probe anchoring region (A) and the target sequence binding region in the forward primer (F);
the probe (P) further comprises a primer anchoring region (A'), the primer anchoring region (A') is a sequence that is complementary with the probe anchoring region (A) of the forward primer (F); preferably, there is a gap of 0-20 bases between the primer anchoring region (A') and the probe signal detection region (H) in the probe (P).

7. A method for detecting two or more kinds of target nucleic acids, comprising the following steps:
mixing a primer-probe composition, a sample to be detected and an amplification reagent to obtain a reaction system; the primer-probe composition comprising at least one kind of probe (P), and at least two kinds of primers for different targets; the amplification reagent comprising a DNA polymerase and dNTPs;
performing n PCR amplifications and n signal acquisitions on the reaction system; one signal acquisition being performed after each PCR amplification, and each signal acquisition comprising at least one signal channel acquisition; each signal channel acquisition contains at most one kind of target signal;
an annealing temperature for n<th> PCR amplification being Tn, an acquisition temperature for n<th> signal acquisition being tn, Tn < T(n-1), and Tn < t(n-1); tn > t(n-1), and tn > T(n-1); preferably, 40°C ≤ Tn < T(n-1) ≤ 75°C, and/or 0°C ≤ t(n-1) < tn ≤ 90°C;
n being an integer ≥ 2, and n ≤ a number of classes of targets to be detected in the sample to be detected;
preferably, the at least one signal acquisition comprising m signal channel acquisitions; m being a number of classes of detection labels of the probe in the primer-probe composition, and a product of n and m ≥ the number of the targets to be detected in the sample to be detected.

8. The method according to claim 7, **characterized in that** reaction conditions for performing a first PCR amplification on the reaction system comprise: initial denaturation at about 85°C to about 105°C for about 0 to about 15 min; denaturation at about 85°C to about 105°C for about 1 to about 60 s, and annealing and extending at about 40°C to about 75°C for about 3 to about 90 s, for 20 to 60 cycles; preferably, when a target in the sample to be detected is RNA, the amplification reagent further comprises a reverse transcriptase, and the reaction conditions for performing the first PCR amplification on the reaction system comprise: reverse transcription at about 30 to about 65°C for about 2 to about 30 min; initial denaturation at about 85°C to about 105°C for about 0 to about 15 min; denaturation at about 85°C to about 105°C for about 1 to about 60 s, and annealing and extending at about 40°C to about 75°C for about 3 to about 90 s, for 20 to 60 cycles.

9. The method according to claim 7, **characterized in that** reaction conditions for performing a first PCR amplification on the reaction system comprise: initial denaturation at about 85°C to about 105°C for about 0 to about 15 min; denaturation at about 85°C to about 105°C for about 1 to about 60 s, annealing and extending at about 40°C to about 75°C for about 3 to about 90 s, for 20 to 60 cycles; denaturation at about 85°C to about 105°C for about 1 to about 60 s, annealing and extending at about 20°C to about 75°C for about 3 to about 90 s, for 1 to 20 cycles; alternatively, the reaction conditions for performing the first PCR amplification on the reaction system comprise: initial denaturation at about 85°C to about 105°C for about 0 to about 15 min; denaturation at about 85°C to about 105°C for about 1 to about 60 s, and annealing and extending at about 40°C to about 75°C for about 3 to about 90 s, for 20 to 60 cycles; constant-temperature incubation at about 20°C to about 75°C for about 10 to about 1800 s;
preferably, when a target in the sample to be detected is RNA, the amplification reagent further comprises a reverse transcriptase, and the reaction conditions for performing the first PCR amplification on the reaction system comprise: reverse transcription at about 30 to about 65°C for about 2 to about 30 min; initial denaturation at about 85°C to about 105°C for about 0 to about 15 min; denaturation at about 85°C to about 105°C for about 1 to about 60 s, and annealing and extending at about 40°C to about 75°C for about 3 to about 90 s, for 20 to 60 cycles; denaturation at about 85°C to about 105°C for about 1 to about 60 s, and annealing and extending at about 20°C to about 75°C for about 3 to about 90 s, for 1 to 20 cycles; alternatively, the reaction conditions for performing the first PCR amplification on the reaction system comprise: reverse transcription at about 30 to about 65°C for about 2 to about 30 min; initial denaturation at about 85°C to about 105°C for about 0 to about 15 min; denaturation at about 85°C to about 105°C for about 1 to about 60 s, and annealing and extending at about 40°C to about 75°C for about 3 to about 90 s, for 20 to 60 cycles; and constant-temperature incubation at about 20°C to about 75°C for about 10 to about 1800 s.

10. The method according to any one of claims 7-9, **characterized in that** other PCR amplifications after the first PCR amplification comprise the following reaction conditions: denaturation at about 85°C to about 105°C for about 1 to about 60 s, and annealing and extending at about 20°C to about 75°C for about 3 to about 90 s, for 1 to 20 cycles; and constant-temperature incubation at about 20°C to about 75°C for about 10 to about 1800 s.

11. The method according to claim 7, **characterized in that** before performing the first PCR amplification on the reaction system, the method further comprises: distributing the reaction system into 500 or more reaction units, with each reaction unit containing one target nucleic acid of the sample to be detected or containing no target nucleic acids of the sample to be detected; preferably, the signal acquisition refers to an acquisition of a fluorescence signal by means of a camera; and the signal channel acquisition refers to an acquisition of the fluorescence signal by means of the camera under a fluorescence signal channel.

12. The method according to any one of claims 7-11, **characterized in that** at least one kind of the primers specifically binds to the target to generate a pre-product that contains one single-stranded pre-product capable of specifically binding to the probe (P), the single-stranded pre-product specifically binds to the probe (P) and extends to form a double-stranded product, and formation of the double-stranded product causes a probe signal change; preferably, the single-stranded pre-product specifically binds to the probe and extends by 0-100 bases; more preferably, the single-stranded pre-product specifically binds to the probe and extends by 1-100 bases; and/or, the primers have a concentration of about 30 nM to about 1,000 nM, and the probe (P) has a concentration of about 100 nM to about 1,200 nM.

13. The method according to any one of claims 7-11, **characterized in that** the at least two kinds of primers for the different targets comprise any one of the following:
(1) at least two kinds of forward primers (F) and at least one kind of reverse primer (R);
(2) at least one kind of forward primer (F) and at least two kinds of reverse primers (R);
(3) at least two kinds of forward primers (F) and at least two kinds of reverse primers (R);
the different forward primers (F) each independently contain a target sequence binding region that is specifically paired with and binding to different target sequences;
and/or, the probe (P), as one freely-designed sequence that is not specifically paired with or binding to any target sequences, comprises a probe signal detection region (H), and the probe is modified with detection labels; and/or, the reverse primer (R) contains a primer signal detection region (h) and a target sequence binding region, and the primer signal detection region (h) is located at a 5' end of the target sequence binding region; wherein target sequence binding regions of the different reverse primers (R) are sequences that are specifically paired with and binding to different target sequences; each of primer signal detection regions (h) of the different reverse primers (R) is one sequence that is not specifically paired with or binding to any target sequences, and a complementary sequence of the primer signal detection region (h) is specifically paired with and binding to the probe signal detection region (H) of the probe (P); preferably, sequences of the primer signal detection regions (h) of the different reverse primers (R) are different from each other.

14. The method according to any one of claims 7-13, **characterized in that** at least one kind of the reverse primers (R) in the primers sequentially contains an extension block region (M), a primer signal detection region (h) and a target sequence binding region from a 5' end to a 3' end; the extension block region (M) is a freely-designed sequence that is not same as or complementary with a sequence of any part of the probe (P) or any target sequences; if there are other kinds of reverse primers, 5' ends of the other kinds of reverse primers (R) contain no extension block regions (M).

15. The method according to any one of claims 7-14, **characterized in that** at least one kind of the forward primers (F) in the primers further comprises a probe anchoring region (A), and the probe anchoring region (A) in the forward primer (F) is located at a 5' end of the target sequence binding region; wherein the probe anchoring region (A) is one sequence that is not specifically paired with or binding to any target sequences; sequences of target sequence binding regions of different forward primers (F) are different; preferably, there is a gap of 0-20 bases between the probe anchoring region (A) and the target sequence binding region in the forward primer (F);
the probe (P), as one sequence that is not paired with or binding to any target sequences, comprises a primer anchoring region (A') and a probe signal detection region (H); the primer anchoring region (A') is a sequence that is complementary with the probe anchoring region (A) of the forward primer (F); preferably, there is a gap of 0-20 bases between the primer anchoring region (A') and the probe signal detection region (H) in the probe (P).
